(19) Europäisches Patentamt
European Patent Office
Office européen des brevets

(11) EP 1 973 539 B1

(12) **EUROPEAN PATENT SPECIFICATION**

(45) Date of publication and mention of the grant of the patent:
**21.04.2010 Bulletin 2010/16**

(21) Application number: **06847799.1**

(22) Date of filing: **19.12.2006**

(51) Int Cl.:
***A61K 31/352*** *(2006.01)* ***A61K 31/353*** *(2006.01)*
***A61K 31/357*** *(2006.01)* ***A61P 25/24*** *(2006.01)*

(86) International application number:
**PCT/US2006/048538**

(87) International publication number:
**WO 2007/075751 (05.07.2007 Gazette 2007/27)**

(54) **USE OF BENZO-FUSED HETEROCYCLE SULFAMIDE DERIVATIVES FOR THE TREATMENT OF DEPRESSION**

VERWENDUNG VON BENZOKONDENSIERTEN HETEROCYCLISCHEN SULFAMID-DERIVATEN ZUR BEHANDLUNG VON DEPRESSIONEN

UTILISATION DE DERIVES DE SULFAMIDE HETEROCYCLIQUE BENZO-FUSIONNE POUR LE TRAITEMENT DE LA DEPRESSION

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **19.12.2005 US 751730 P**
**18.12.2006 US 612249**

(43) Date of publication of application:
**01.10.2008 Bulletin 2008/40**

(73) Proprietor: **Janssen Pharmaceutica N.V.**
**2340 Beerse (BE)**

(72) Inventors:
- **SMITH-SWINTOSKY, Virginia, L.**
**Hatfield, Pennsylvania 19440 (US)**
- **REITZ, Allen, B.**
**Lansdale, Pennsylvania 19446 (US)**

(74) Representative: **Warner, James Alexander et al**
**Carpmaels & Ransford**
**43-45 Bloomsbury Square**
**London WC1A 2RA (GB)**

(56) References cited:
**WO-A-02/09694** **WO-A-2006/007435**

- **MARYANOFF B E ET AL: "Comparison of Sulfamate and Sulfamide Groups for the Inhibition of Carbonic Anhydrase-II by Using Topiramate as a Structural Platform" JOURNAL OF MEDICINAL CHEMISTRY, AMERICAN CHEMICAL SOCIETY. WASHINGTON, US, vol. 48, no. 6, 13 December 2004 (2004-12-13), pages 1941-1947, XP002345002 ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### CROSS REFERENCE TO RELATED APPLICATIONS

This application claims the benefit of U. S. Provisional Application 60/751,730, filed on December 19, 2005.

### FIELD OF THE INVENTION

The present invention is directed to the use of benzo-fused heterocycle sulfamide derivatives for the treatment of depression, including both monotherapy and co-therapy with at least one anti-depressant.

### BACKGROUND OF THE INVENTION

Unipolar depression is defined as depressed mood on a daily basis for a minimum duration of two weeks. An episode may be characterized by sadness, indifference or apathy, or irritability and is usually associated with a change in a number of neurovegetative functions, including sleep patterns, appetite and body weight, motor agitation or retardation, fatigue, impairment in concentration and decision making, feelings of shame or guilt, and thoughts of death or dying (Harrison's Principles of Internal Medicine, 2000). The criteria for a major depressive episode includes five or more symptoms present during the same 2-week period, where this represent a change from previous functioning; and where at least one of the symptoms is either depressed mood or loss of interest or pleasure. Symptoms of a depressive episode include depressed mood; markedly diminished interest or pleasure in all, or almost all, activities most of the day; weight loss when not dieting or weight gain, or decrease or increase in appetite nearly every day; insomnia or hypersomnia nearly every day; psychomotor agitation or retardation nearly every day; fatigue or loss of energy nearly every day; feelings of worthlessness or excessive or inappropriate guilt nearly every day; diminished ability to think or concentrate, or indecisiveness, nearly every day; recurrent thoughts of death, recurrent suicidal ideation without a specific plan, or a suicide attempt or a specific plan for committing suicide. Further, the symptoms cause clinically significant distress or impairment in social, occupational, or other important areas of functioning. *(*Diagnostic and Statistical Manual of Mental Disorders, 4th Edition, Text Revision, American Psychiatric Association, 2000)

Current treatment options for unipolar depression include monotherapy or combination therapy with various classes of drugs including mono-amine oxidase inhibitors, tricyclics, serotonin reuptake inhibitors, serotonin noradrenergic reuptake inhibitors, noradrenergic and specific serotonergic agents, noradrenaline reuptake inhibitor, "natural products" (such as Kava-Kava, St. John's Wort), dietary supplement (such as s-adenosylmethionine) and others. More specifically, drugs used in the treatment of depression include, but are not limited to imipramine, amitriptyline, desipramine, nortriptyline, doxepin, protriptyline, trimipramine, maprotiline, amoxapine, trazodone, bupropion, chlomipramine, fluoxetine, citalopram, sertraline, paroxetine, fluvoxamine, nefazadone, venlafaxine, reboxetine, mirtazapine, phenelzine, tranylcypromine, and/or moclobemide (eg, J.M. KENT, Lancet 2000, 355, 911-918;.J.W. WILLIAMS JR, C.D. MULROW, E. CHIQUETTE, P.H. NOEL, C. AGUILAR, and J. CORNELL, Ann. Intern. Med. 2000, 132, 743-756; P.J. AMBROSINI, Psychiatr. Serv. 2000, 51, 627-633). Several of these agents including, but not limited to, serotonin reuptake inhibitors are also used when depression and anxiety co-exist, such as in anxious depression (R.B. LYDIARD and O. BRAWMAN-MINTZER, J: Clin. Psychiatry 1998, 59, Suppl. 18, 10-17; F. ROUILLON, Eur. Neuropsychopharmacol. 1999, 9 Suppl. 3, S87-S92).

In the clinic, 40-50% of depressed patients who are initially prescribed antidepressant therapy do not experience a timely remission of depression symptoms. This group typifies treatment-refractory depression, that is, a failure to demonstrate an "adequate" response to an "adequate" treatment trial (that is, sufficient intensity of treatment for sufficient duration) (R.M. BERMAN, M. NARASIMHAN, and D.S. CHARNEY, Depress. Anxiety 1997, 5, 154-1.64). Moreover, about 20-30% of depressed patients remain partially or totally resistant to pharmacological treatment including combination treatments (J. ANANTH, Psychother. Psychosom. 1998, 67, 61-70; R.J. CADIEUX, Am. Fam. Physician 1998, 58, 2059-2062). Increasingly, treatment of resistant depression includes augmentation strategies including treatment with pharmacological agents such as, lithium, carbamazepine, and triiodothyronine, and the like (M. HATZINGER and E. HOLSBOER-TRACHSLER, Wien. Med. Wochenschr. 1999, 149, 511-514; C.B. NEMEROFF, Depress: Anxiety 1996-1997, 4, 169-181; T.A. KETTER, R.M. POST, P.I. PAREKH and K. WORTHINGTON, J. Clin. Psychiatry 1995, 56, 471-475; R.T. JOFFE, W. SINGER, A.J. LEVITT, C. MACDONALD, Arch. Gen. Psychiatry 1993, 50, 397-393).

Dysthymia is defined as a mood disorder characterized by chronic depressed mood for a period of at least 2 years. Dysthymia can have a persistent or intermittent course and the depressed mood occurs for most of the day, for more days than not, and for at least 2 years. (Diagnostic and Statistical Manual of Mental Disorders, 4th Edition, American Psychiatric Association, 1994).

Bipolar disorder, on the other hand, is characterized by unpredictable swings in mood between mania and depression (bipolar I disorder) or between hypomania and depression (bipolar II disorder) (Diagnostic and Statistical

Manual of Mental Disorders, 4th Edition, American Psychiatric Association, 1994**).** Antidepressant use in bipolar disorder is generally, intentionally restricted to avoid the risk of mania and the risk of rapid cycling induced by antidepressants in bipolar disorder (H.J. MOLLER and H. GRUNZE, Eur. Arch. Psychiatry Clin. Neurosci. 2000, 250, 57-68; J.R. CALABRESE, D.J. RAPPORT, S.E. KIMMEL, and M.D. SHELTON, Eur. Neuropsychopharmacol. 1999, 9, S109-S112). Moreover, none of the mood stabilizers used in bipolar disorder have proven antidepressive efficacy (H.J. MOLLER and H. GRUNZE, Eur. Arch. Psychiatry Clin. Neurosci. 2000, 250, 57-68).

There remains a need to provide an effective treatment for depression. B.E. Maryanoff *et al*. (B.E. Maryanoff, D.F. McComsey, M.J. Costanzo, C. Hochman, V. Smith-Swintosky, and R.P. Shank, J. Med Chem. 2004, 48(6), 1941-1947) examines the relative effectiveness of sulfamate and sulfamide groups for the inhibition of carbonic anhydrase-II (CA-II), in particular inhibition of human CA-II, by using topiramate as a structural platform.

Anticonvulsant derivatives useful for the treatment of depression as monotherapy or combination therapy are disclosed in WO 02/09694 A. These compounds may be administered in combination with one or more compounds selected from mono-amine oxidase inhibitors, tricyclics, serotonin reuptake inhibitors, serotonin noradrenergic reuptake inhibitors, noradrenergic and specific serotonergic agents, noradrenaline reuptake inhibitors, natural products, dietary supplements, neuropeptides, compounds targeting neuropeptide receptors or hormones.

SUMMARY OF THE INVENTION

The present invention is directed to a medicament for the treatment of depression comprising a therapeutically effective amount of a compound of formula (I)

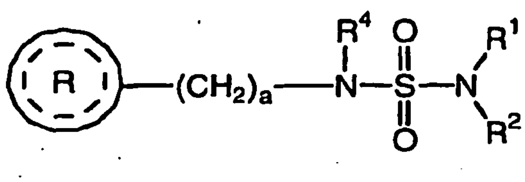

(I)

wherein

$R^1$ and $R^2$ are each independently selected from the group consisting of hydrogen and lower alkyl;
$R^4$ is selected from the group consisting of hydrogen and lower alkyl;
a is an integer from 1 to 2;

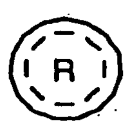

is selected from the group consisting of

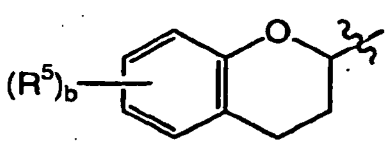

,

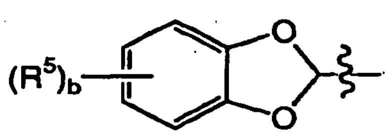

,

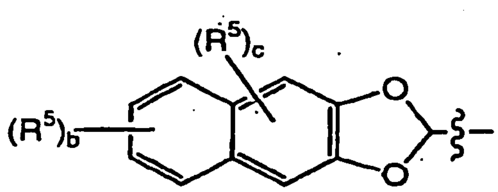

,

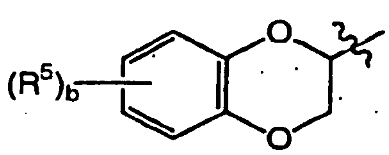

,

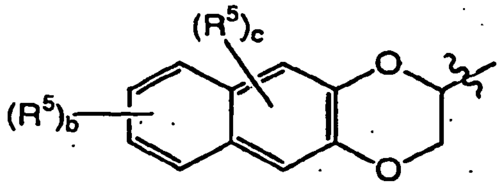,
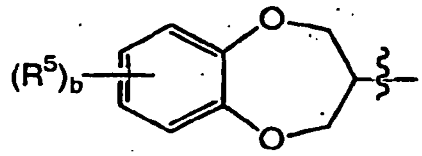

and

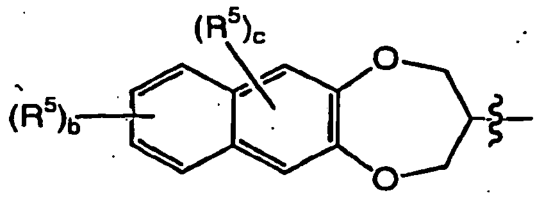;

wherein b is an integer from 0 to 4; and wherein c is an integer from 0 to 2;
each $R^5$ is independently selected from the group consisting of halogen, lower alkyl and nitro;
provided that when

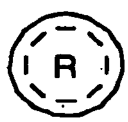

is

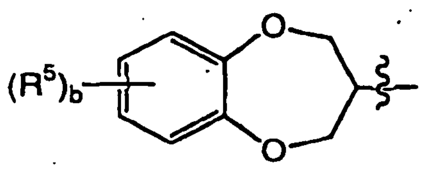

or

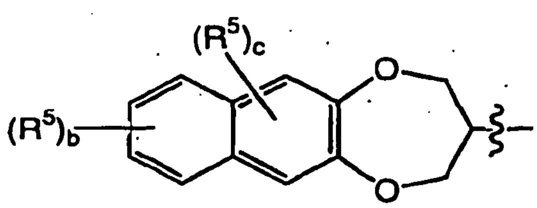,

then a is 1;

or a pharmaceutically acceptable salt thereof.

The present invention is further directed to a medicament for the treatment of depression comprising a therapeutically effective amount of compound of formula (II)

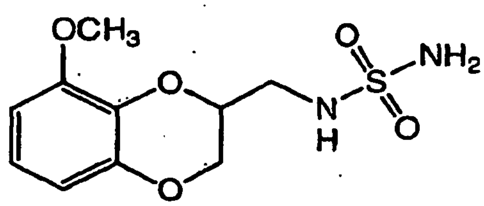

**(II)**

or a pharmaceutically acceptable salt thereof.

The present invention is further directed to a medicament for the treatment of depression comprising a therapeutically effective amount of at least one antidepressant and a compound of formula (I) or formula (II) as herein defined for use in co-therapy.

Exemplifying the invention is a medicament for treating major depressive disorder, unipolar depression, treatment refractory depression, resistant depression, anxious depression or dysthymia comprising a therapeutically effective amount of any of the compounds or pharmaceutical compositions described above.

In another example, the present invention is directed to a medicament fan treating major depressive disorder, unipolar depression, treatment refractory depression, resistant depression, anxious depression or dysthymia comprising at least one antidepressant in combination with any of the compounds or pharmaceutical compositions described above.

DETAILED DESCRIPTION OF THE INVENTION

The present invention is directed to a medicament for the treatment of depression comprising a therapeutically effective amount of a compound of formula (I)

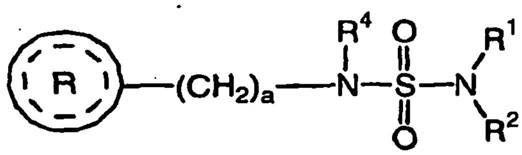

**(I)**

or a pharmaceutically acceptable salt thereof, wherein

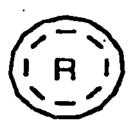

,

a, $R^1$, $R^2$ and $R^4$ are as herein defined. The present invention is further directed to the treatment of depression comprising administering to a subject in need thereof a therapeutically effective amount of a compound of formula (I) or formula (II) in combination with at least one antidepressant.

As used herein, the term **"depression"** shall be defined to include major depressive disorder (including single episode and recurrent), unipolar depression, treatment-refractory depression, resistant depression, anxious depression and dysthymia (also referred to as dysthymic disorder). Further, the term "depression" shall encompass any major depressive disorder, dysthymic disorder, mood disorders due to medical conditions with depressive features, mood disorders due to medical conditions with major depressive like episodes, substance-induced mood disorders with depressive features and depressive disorder not otherwise specific as defined by their diagnostic criteria, as listed in the Diagnostic and Statistical Manual of Mental Disorders, 4th Edition, Text Revision, American Psychiatric Association, 2000. Preferably, the depression is major depressive disorder, unipolar depression, treatment-refractory depression, resistant depression or anxious depression. More preferably, the depression is major depressive disorder.

As used herein, unless otherwise noted, the term **"antidepressant"** shall mean any pharmaceutical agent which treats depression. Suitable examples include, mono-amine oxidase inhibitors such as phenelzine, tranylcypromine, moclobemide; tricyclics such as imipramine, amitriptyline, desipramine, nortriptyline, doxepin, protriptyline, trimipramine, chlomipramine, amoxapine; tetracyclics such as maprotiline; non-cyclics such as nomifensine; triazolopyridines such as trazodone; serotonin reuptake inhibitors such as fluoxetine, sertraline, paroxetine, citalopram, fluvoxamine; serotonin

receptor antagonists such as nefazadone; serotonin noradrenergic reuptake inhibitors such as venlafaxine, milnacipran; noradrenergic and specific serotonergic agents such as mirtazapine; noradrenaline reuptake inhibitors such as reboxetine; atypical antidepressants such as bupropion; natural products such as Kava-Kava, St. John's Wort; dietary supplements such as s-adenosylmethionine; and neuropeptides such as thyrotropin-releasing hormone; compounds targeting neuropeptide receptors such as neurokinin receptor antagonists; and hormones such as triiodothyronine. Preferably, the antidepressant is selected from the group consisting of fluoxetine, imipramine, bupropion, venlafaxine and sertaline.

One skilled in the art would be able to readily determined recommended dosage levels for known and / or marketed antidepressant and antipsychotic drugs by consulting appropriate references such as drug package inserts, FDA guidelines, the Physician's Desk Reference.

The term **"subject"** as used herein, refers to an animal, preferably a mammal, most preferably a human, who has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Wherein the present invention is directed to co-therapy or combination therapy, comprising administration of one or more compound(s) of formula (I) or formula (II) and one or more antidepressants, "therapeutically effective amount" shall mean that amount of the combination of agents taken together so that the combined effect elicits the desired biological or medicinal response. For example, the therapeutically effective amount of co-therapy comprising administration of a compound of formula (I) or formule (II) and at least on antidepressant would be the amount of the compound of formula (I) or formula (II) and the amount of the antidepressant that when taken together or sequentially have a combined effect that is therapeutically effective. Further, it will be recognized by one skilled in the art that in the case of co-therapy with a therapeutically effective amount, as in the example above, the amount of the compound of formula (I) or formula (II) and/or the amount of the antidepressant individually may or may not be therapeutically effective.

As used herein, the terms **"co-therapy"** and **"combination therapy"** shall mean treatment of a subject in need thereof by administering one or more compounds of formula (I) or formula (II) in combination with one or more antidepressant(s), wherein the compound(s) of formula (I) or formula (II) and the antidepressant(s) are administered by any suitable means, simultaneously, sequentially, separately or in a single pharmaceutical formulation. Where the compound(s) of formula (I) or formula (II) and the antidepressant(s) are administered in separate dosage forms, the number of dosages administered per day for each compound may be the same or different. The compound(s) of formula (I) or formula (II) and the antidepressant(s) may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of formula (I) or formula (II) and the antidepressant(s) may be administered according to simultaneous or alternating regimens, at the same or different times during the course of the therapy, concurrently in divided or single forms.

In an embodiment of the present invention is a medicament for the treatment of depression comprising a combination of one or more compounds of formula (I) or formula (II) with one or more compounds selected from the group consisting of mono-amine oxidase inhibitors such as phenelzine, tranylcypromine, moclobemide; tricyclics such as imipramine, amitriptyline, desipramine, nortriptyline, doxepin, protriptyline, trimipramine, chlomipramine, amoxapine; tetracyclics such as maprotiline; non-cyclics such as nomifensine; triazolopyridines such as trazodone; serotonin reuptake inhibitors such as fluoxetine, sertraline, paroxetine, citalopram, fluvoxamine, escitalopram oxalate; serotonin receptor antagonists such as nefazadone; serotonin noradrenergic reuptake inhibitors such as venlafaxine, milnacipran, duloxetine; noradrenergic and specific serotonergic agents such as mirtazapine; noradrenaline reuptake inhibitors such as reboxetine; atypical antidepressants such as bupropion, ; natural products such as Kava-Kava, St. John's Wort; dietary supplements such as s-adenosylmethionine; and neuropeptides such as thyrotropin-releasing hormone; compounds targeting neuropeptide receptors such as neurokinin receptor antagonists; and hormones such as triiodothyronine.

In an embodiment of the present invention is a medicament for the treatment of depression comprising a combination of.one or more compounds of formula (I) or formula (II) with one or more compounds selected from the group consisting of mono-amine oxidase inhibitors; tricyclics; tetracyclics; non-cyclics; triazolopyridines; serotonin reuptake inhibitors; serotonin receptor antagonists; serotonin noradrenergic reuptake inhibitors; serotonin noradrenergic reuptake inhibitors; noradrenergic and specific serotonergic agents; noradrenaline reuptake inhibitors; atypical antidepressants; natural products; dietary supplements; neuropeptides; compounds targeting neuropeptide receptors; and hormones.

Preferably, one or more compounds of formula (I) or formula (II) are administered in combination with one or more compounds selected from the group consisting of mono-amine oxidase inhibitors, tricyclics, serotonin reuptake inhibitors, serotonin noradrenergic reuptake inhibitors; noradrenergic and specific serotonergic agents and atypical antidepressants.

More preferably, one or more compounds of formula (I) or formula (II) are administered in combination with one or more compound selected from the group consisting of mono-amino oxidase inhibitors, tricyclics and serotonin reuptake inhibitors.

Most preferably, one or more compounds of formula (I) or formula (II) are administered in combination with one or more compounds selected from the group consisting of serotonin reuptake inhibitors.

In an embodiment of the present invention is a medicament for the treatment of depression comprising a combination of one or more compounds of formula (I) or formula (II) with one or more compounds selected from the group consisting of phenelzine, tranylcypromine, moclobemide, imipramine, amitriptyline, desipramine, nortriptyline, doxepin, protriptyline, trimipramine, chlomipromine, amoxapine, fluoxetine, sertraline, paroxetine, citalopram, fluvoxamine, venlafaxine, milnacipran, duloxetine, mirtazapine, bupropion, thyrotropin-releasing hormone and triiodothyronine.

Preferably, one or more compounds of formula (I) or formula (II) are administered in combination with one or more compounds selected from the group consisting of phenelzine, tranylcypromine, moclobemide, imipramine, amitriptyline, desipramine, nortriptyline, doxepin, protriptyline, trimipramine, chlomipramine, amoxapine, fluoxetine, sertraline, paroxetine, citalopram, fluvoxamine, venlafaxine, milnacipran, mirtazapine and bupropion.

More preferably, one or more compounds of formula (I) or formula (II) are administered in combination with one or more compounds selected from the group consisting of phenelzine, tranylcypromine, moclobemide, imipramine, amitriptyline, desipramine, nortiptyline, doxepin, protriptyline, trimipramine, chlomipramine, amoxapine, fluoxetine, sertraline, paroxetine, citalopram, escitalopram and fluvoxamine.

Most preferably, one or more compounds of formula (I) or formula (II) are administered in combination with one or more compounds selected from the group consisting of fluoxetine, sertraline, paroxetine, citalopram and fluvoxamine.

In an embodiment of the present invention, is a medicament for the treatment of depression comprising a combination of one or more compounds of formula (I) or formula (II) with one or more compounds selected from the group consisting of neuropeptides such as thyrotropin-releasing hormone; compounds targeting neuropeptide receptors such as neurokinin receptors antagonists; and hormones such as triiodothyronine.

In an embodiment of the present invention $R^1$ is selected from the group consisting of hydrogen and methyl. In another embodiment of the present invention $R^2$ is selected from the group consisting of hydrogen and methyl. In yet another embodiment of the present invention $R^1$ and $R^2$ are each hydrogen or $R^1$ and $R^2$ are each methyl.

In an embodiment of the present invention $-(CH_2)_a-$ is selected from the group consisting of $-CH_2-$ and $-CH_2-CH_2-$. In another embodiment of the present invention $-(CH_2)_a-$ is $-CH_2-$.

In an embodiment of the present $R^4$ is selected from the group consisting of hydrogen and methyl, preferably, $R^4$ is hydrogen.

In an embodiment of the present invention a is 1.

In an embodiment of the present invention b is an integer from 0 to 2. in another embodiment of the present invention c is an integer from 0 to 2. In another embodiment of the present invention b is an integer from 0 to 1. In another embodiment of the present invention c is an integer from 0 to 1. In yet another embodiment of the present invention the sum of b and c is an integer form 0 to 2, preferably an integer form 0 to 1. In yet another embodiment of the present invention b is an integer from 0 to 2 and c is 0.

In an embodiment of the present invention,

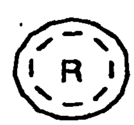

is selected from the group consisting of

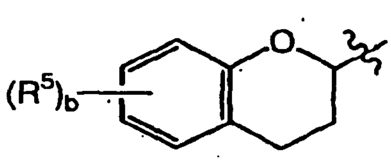 , 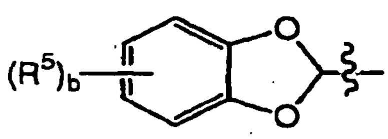 ,

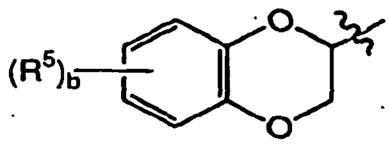,
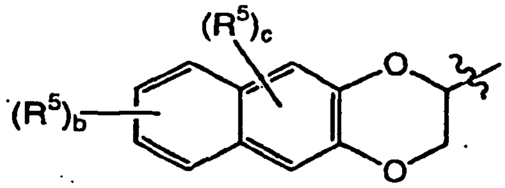

and

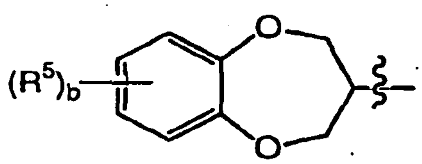

In another embodiment of the present invention,

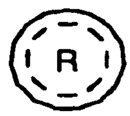

is selected from the group consisting of

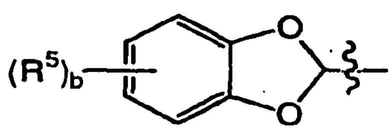,

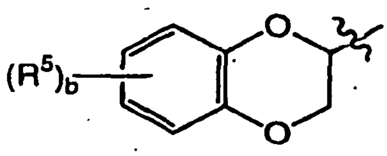

and

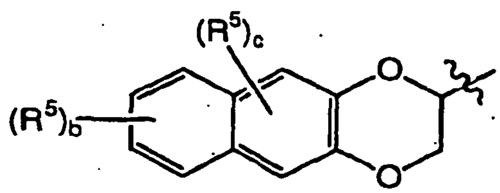

In an embodiment of the present invention,

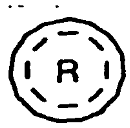

is selected from the group consisting of 2-(2,3-dihydro-benzo[1,4]dioxinyl), 2-(benzo[1,3]dioxolyl), 3-(3,4-dihydro-benzo [1,4]dioxepinyl), 2-(6-chloro-2,3-dihydro-benzo[1,4]dioxinyl), 2-(6-fluoro-2,3-dihydro-benzo[1,4]dloxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl); 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl),2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl).

In another embodiment of the present invention,

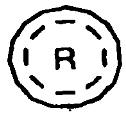

is selected from the group consisting 2-(benzo[1,3]dioxolyl), 4-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]diokinyl) and 2-(6,7-dichloro-2,3-dihydro-benzo[1,4]dioxinyl). In another embodiment of the present invention,

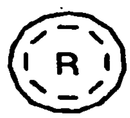

is selected from the group consisting of 2-(2,3-dihydro-benzo[1,4]dioxinyl), 2-(7-methyl-2,3-dihydro-benzo[1,4]dioxinyl) and 2-(6-bromo-2,3-dihydro-benzo[1,4]dioxinyl).

In an embodiment of the present invention $R^5$ is selected from the group consisting of halogen and lower alkyl. In another embodiment of the present invention $R^5$ is selected from chloro, fluoro, bromo and methyl.

In an embodiment of the present invention, the stereo-center on the compound of formula (I) is in the S-configuration. In another embodiment of the present invention, the stereo-center on the compound of formula (I) is in the R-configuration.

In an embodiment of the present invention the compound of formula (I) is present as an enantiomerically enriched mixture, wherein the % enantiomeric enrichment (% ee) is greater than about 75%, preferably greater than about 90%, more preferably greater than about 95%, most preferably greater than about 98%.

Additional embodiments of the present invention, include those wherein the substituents selected for one or more of the variables defined herein (i.e. $R^1$, $R^2$, $R^3$, $R^4$, X-Y and A) are independently selected to be any individual substituent or any subset of substituents selected from the complete list as defined herein.

Representative compounds of the present invention, are as listed in Tables 1 below. Additional compounds of the present invention are as listed in Table 3. In Tables 1 and 2 below, the column headed "stereo" defines the stereo-configuration at the carbon atom of the heterocycle attached at the starred bond. Where no designation is listed, the compound was prepared as a mixture of stereo-configurations. Where an "R" or "S" designation is listed, the stereo-configuration was based on the enantiomerically enriched starting material.

**Table 1: Representative Compounds of Formula (I)**

| 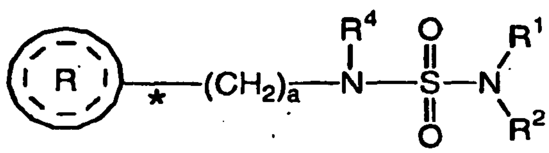 | | | | | | |
|---|---|---|---|---|---|---|
| ID No. | 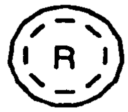 | Stereo | $(CH_2)_a$ | $NR^4$ | $R^1$ | $R^2$ |
| 1 | 2-(2,3-dihydro-benzo[1,4]dioxinyl) | | $CH_2$ | NH | H | H |
| 2 | 2-(benzo[1,3]dioxolyl) | | $CH_2$ | NH | H | H |
| 3 | 3-(3,4-dihydro-2H-benzo[1,4]dioxepinyl) | | $CH_2$ | NH | H | H |
| 4 | 2-(2,3-dihydro-benzo[1,4]dioxinyl) | S | $CH_2$ | NH | H | H |
| 5 | 2-(2,3-dihydro-benzo[1,4]dioxinyl) | R | $CH_2$ | NH | H | H |

(continued)

| ID No. | 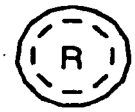 | Stereo | $(CH_2)_a$ | $NR^4$ | $R^1$ | $R^2$ |
|---|---|---|---|---|---|---|
| 6 | 2-(2,3-dihydro-benzo[1,4]dioxinyl) | | $CH_2$ | NH | methyl | methyl |
| 7 | 2-(2,3-dihydro-benzo[1,4]dioxinyl) | | $CH_2$ | $N(CH_3)$ | H | H |
| 8 | 2-(6-chloro-2,3-dihydro-benzo[1,4]dioxinyl) | S | $CH_2$ | NH | H | H |
| 9 | 2-(6-fluoro-2,3-dihydro-benzo[1,4]dioxinyl) | S | $CH_2$ | NH | H | H |
| 10 | 2-(chromanyl) | | $CH_2$ | NH | H | H |
| 13 | 2-(5-fluoro-2,3-dihydro-benzo[1,4]dioxinyl) | S | $CH_2$ | NH | H | H |
| 14 | 2-(7-chloro-2,3-dihydro-benzo[1,4]dioxinyl) | S | $CH_2$ | NH | H | H |
| 15 | 2-(6-chloro-benzo[1,3]dioxolyl) | | $CH_2$ | NH | H | H |
| 16 | 2-(2,3-dihydro-benzo[1,4]dioxinyl) | | $CH_2CH_2$ | NH | H | H |
| 18 | 2-(7-nitro-2,3-dihydro-benzo[1,4]dioxinyl) | S | $CH_2$ | NH | H | H |
| 19 | 2-(7-methyl-2,3-dihydro-benzo[1,4]dioxinyl) | S | $CH_2$ | NH | H | H |
| 20 | 2-(5-chloro-2,3-dihydro-benzo[1,4]dioxinyl) | S | $CH_2$ | NH | H | H |
| 22 | 2-(8-melhoxy-2,3-dihydro-benzo[1,4] dioxinyl) | S | $CH_2$ | NH | H | H |
| 24 | 2-(6-bromo-2,3-dihydro-benzo[1,4]dioxinyl) | S | $CH_2$ | NH | H | H |
| 29 | 2-(6,7-dichloro-2,3-dihydro-benzo[1,4] dioxinyl) | S | $CH_2$ | NH | H | H |
| 30 | 2-(8-chloro-2,3-dihydro-benzo[1,4]dioxinyl) | S | $CH_2$ | NH | H | H |
| 33 | 2-(2,3-dihydro-naphtho[2,3-b][1,4]dioxinyl) | S | $CH_2$ | NH | H | H |
| 35 | 2-(4-methyl-benzo[1,3]dioxolyl) | | $CH_2$ | NH | H | H |

**Table 2: Additional Compounds of the Present Invention**

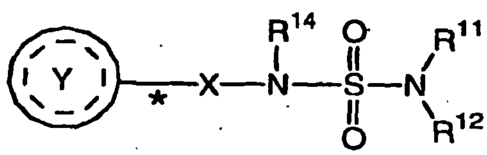

| ID No. | 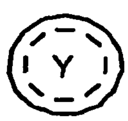 | Stereo | X | $NR^{14}$ | $R^{11}$ | $R^{12}$ |
|---|---|---|---|---|---|---|
| 23 | 2-(5-methoxy-2,3-dihydro-benzo[1,4]dioxinyl) | S | $CH_2$ | NH | H | H |
| 26 | 2-(6-methylcarbonyl-2,3-dihydro-benzo[1,4] dioxinyl) | S | $CH_2$ | NH | H | H |
| 32 | 2-(6-methoxycarbonyl-2,3-dihydro-benzo[1,4] dioxinyl) | S | $CH_2$ | NH | H | H |
| 34 | 2-(6-hydroxymethyl-2,3-dihydro-benzo[1,4] dioxinyl) | S | $CH_2$ | NH | H | H |

(continued)

| ID No. | 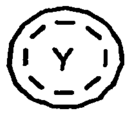 | Stereo | X | $NR^{14}$ | $R^{11}$ | $R^{12}$ |
|---|---|---|---|---|---|---|
| 36 | 2-(7-amino-2,3-dihydro-benzo[1,4]dioxinyl) | S | $CH_2$ | NH | H | H |

As used herein, unless otherwise noted, **"halogen"** shall mean chlorine, bromine, fluorine and iodine.

As used herein, unless otherwise noted, the term **"alkyl"** whether used alone or as part of a substituent group, includes straight and branched chains. For example, alkyl radicals include methyl, ethyl, propyl, isopropyl, butyl, isobutyl, sec-butyl, t-butyl, pentyl and the like. Unless otherwise noted, **"lower"** when used with alkyl means a carbon chain composition of 1-4 carbon atoms.

As used herein, unless otherwise noted, **"alkoxy"** shall denote an oxygen ether radical of the above described straight or branched chain alkyl groups. For example, methoxy, ethoxy, n-propoxy, sec-butoxy, t-butoxy, n-hexyloxy and the like.

As used herein, the notation "*" shall denote the presence of a stereogenic center.

When a particular group is **"substituted"** (e.g., alkyl, aryl, etc.), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a **"phenyl-alkyl-amino-carbonyl-alkyl"** substituent refers to a group of the formula

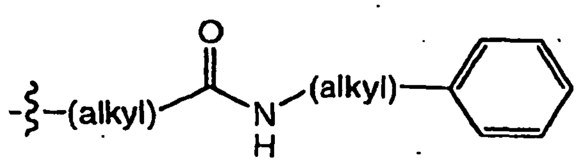

Abbreviations used in the specification, particularly the Schemes and Examples, are as follows:

| | |
|---|---|
| DCC = | Dicyclohexyl Carbodiimide |
| DCE = | Dichloroethane |
| DCM = | Dichloromethane |
| DIPEA or DIEA = | Diisopropylethylamine |
| DMF = | N,N-Dimethylformamide |
| DMSO = | Dimethylsulfoxide |
| EDC = | Ethylcarbodiimide |
| $Et_3N$ or TEA = | Triethylamine |
| $Et_2O$ = | Diethyl ether |
| EA or EtOAc = | Ethyl acetate |
| EtOH = | Ethanol |

| | |
|---|---|
| IPA = | 2-propanol |
| Hept = | Heptane |
| HOBT = | 1 -Hydroxybenzotriazole |
| HPLC = | High Pressure Liquid Chromatography |
| LAH = | Lithium Aluminum Hydride |
| M or MeOH = | Methanol |
| NMR = | Nuclear Magnetic Resonance |
| Pd-C = | Palladium on Carbon Catalyst |
| RP HPLC = | Reverse Phase High Pressure Liquid Chromatography |
| RT or rt | = Room temperature |
| TEA = | Triethylamine |
| TFA = | Trifluoroacetic Acid |
| THF = | Tetrahydrofuran |
| TLC | = Thin Layer Chromatography |

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:

acetate, benzenesulfonate; benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:

acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) may be prepared according to the process outlined in Scheme 1.

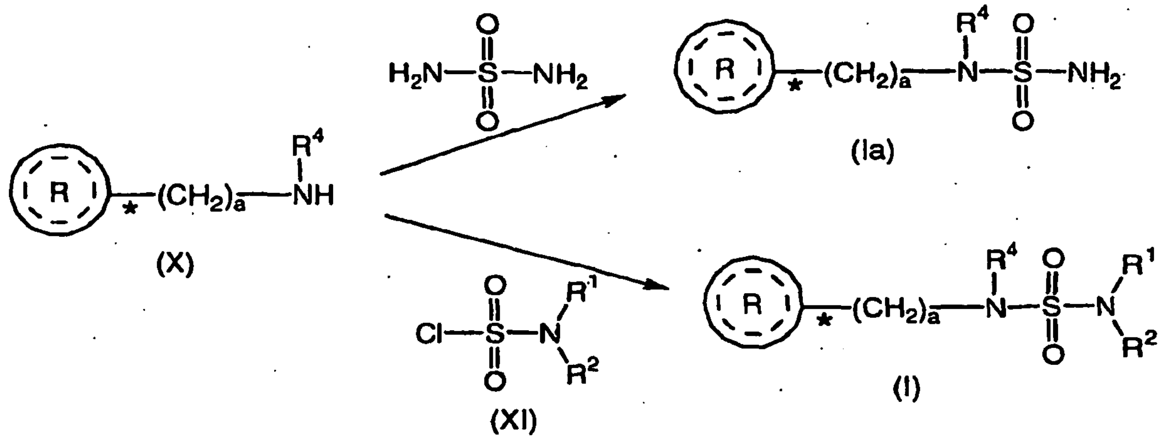

Scheme 1

Accordingly, a suitably substituted compound of formula (X), a known compound or compound prepared by known methods, is reacted with sulfamide, a known compound, preferably wherein the sulfamide is present in an amount in the range of about 2 to about 5 equivalents, in an organic solvent such as THF, dioxane, and the like, preferably at an elevated temperature in the range of about 50˚C to about 100˚C, more preferably at about reflux temperature, to yield the corresponding compound of formula (Ia).

Alternatively, a suitably substituted compound of formula (X), a known compound or compound prepared by known methods, is reacted with a suitably substituted compound of formula (XI), a known compound or compound prepared by known methods, in the presence of a base such as TEA, DIPEA, pyridine, and the like, in an organic solvent such as DMF, DMSO, and the like, to yield the corresponding compound of formula (I).

Compounds of formula (X) wherein

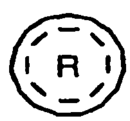

is

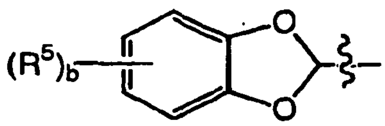

may be prepared according to the process outlined in Scheme 2.

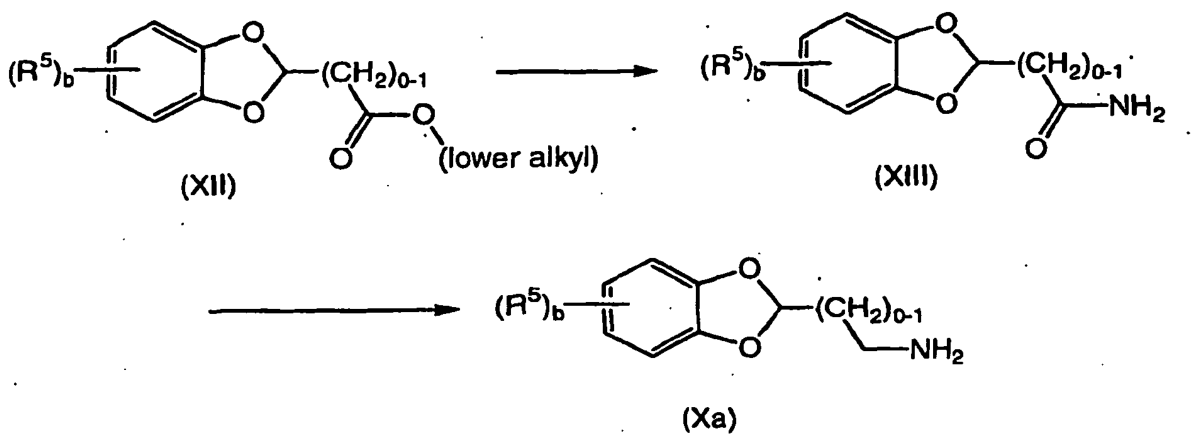

Scheme 2

Accordingly, a suitably substituted compound of formula (XII), a known compound or compound prepared by known method (for example as described in Scheme 3 above) is reacted with $NH_4OH$, a known compound, optionally in an organic solvent such as acetonitrile, and the like, to yield the corresponding compound of formula (XIII).

The compound of formula (XIII) is reacted with a suitably selected reducing agent, such as LAH, and the like, and the like, in an organic solvent such as THF, diethyl ether, and the like, to yield the corresponding compound of formula (Xa).

Compounds of formula (X) wherein

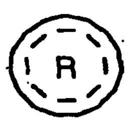

is selected from

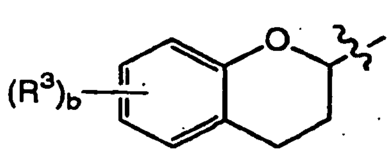

may be prepared according to the process outlined in Scheme 3.

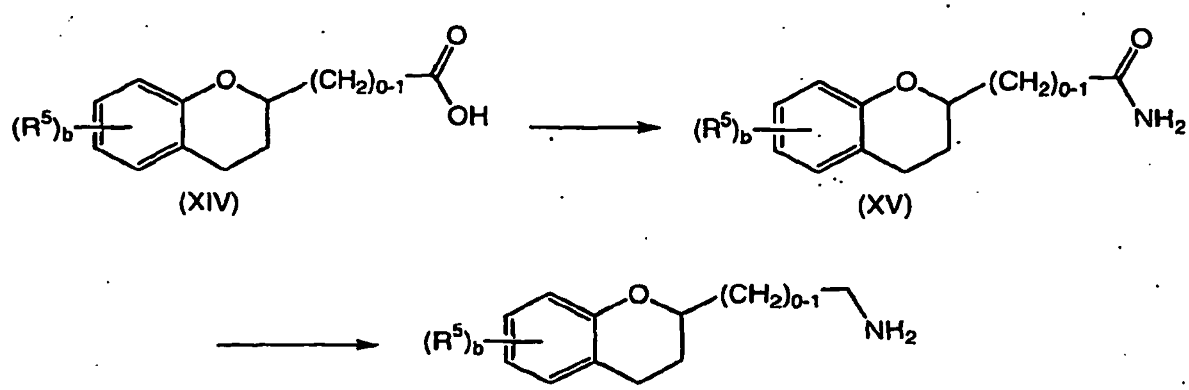

(XIV) (XV)

(Xb)

Scheme 3

Accordingly, a suitably substituted compound of formula (XIV), a known compound or compound prepared by known methods, is reacted with $NH_4OH$, in the presence of a coupling agent such as DCC, and the like, optionally in an organic solvent such as acetonitrile, and the like, to yield the corresponding compound of formula (XV).

The compound of formula (XV) is reacted with a suitably selected reducing agent, such as LAH, and the like, in an organic solvent such as THF, diethyl ether, and the like, to yield the corresponding compound of formula (Xb).

Compounds of formula (X) wherein

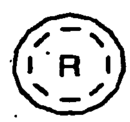

is selected from

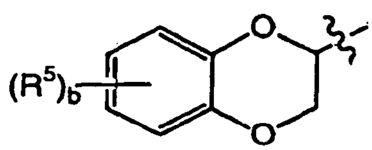

and wherein a is 2, may be prepared according to the process outlined in Scheme 4.

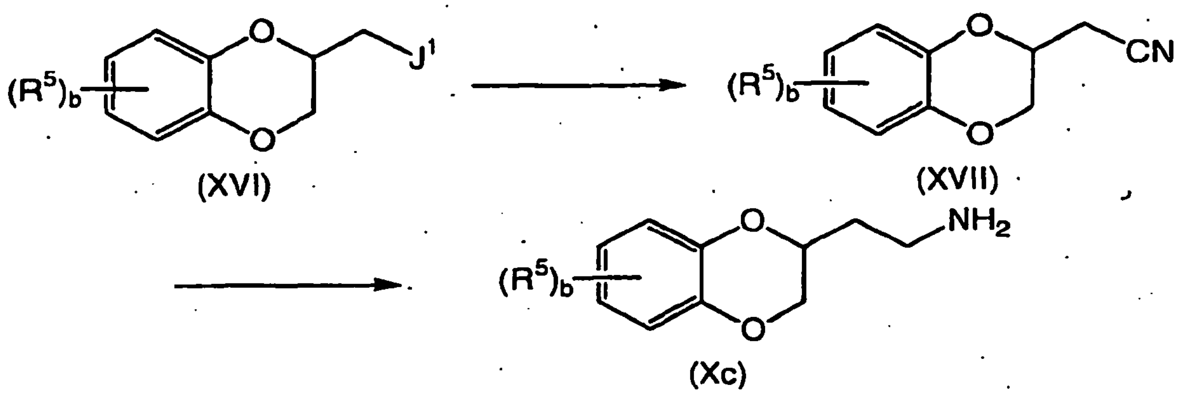

(XVI)

(XVII)

(Xc)

**Scheme 5**

Accordingly, a suitably substituted compound of formula (XVI) wherein $J^1$ is a suitable leaving group such as Br, Cl, I, tosyl, mesyl, triflyl, and the like, a known compound or compound prepared by known methods (for example, by activating the corresponding compound wherein $J^1$ is OH), is reacted with a cyanide such as potassium cyanide, sodium cyanide, and the like, in an organic solvent such as DMSO, DMF, THF, and the like, to yield the corresponding compound of formula (XVII).

The compound of formula (XVII) is reduced according to known methods, for example by reacting with a suitable reducing agent such as LAH, borane, and the like, to yield the corresponding compound of formula (Xc).

Compounds of formula (X) wherein

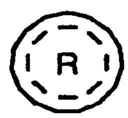

is selected from

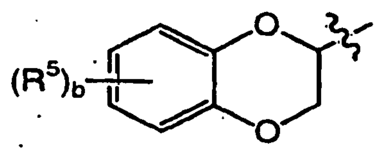

and wherein a is 1, may be prepared according to the process outlined in Scheme 5.

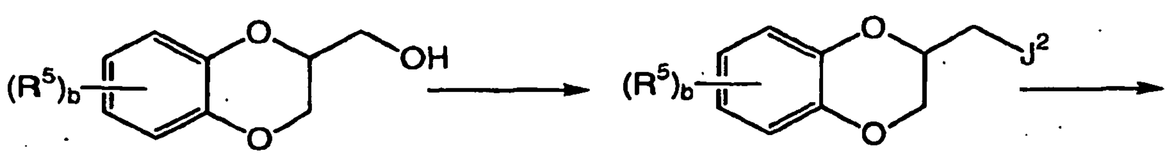

(XVIII)

(XIX)

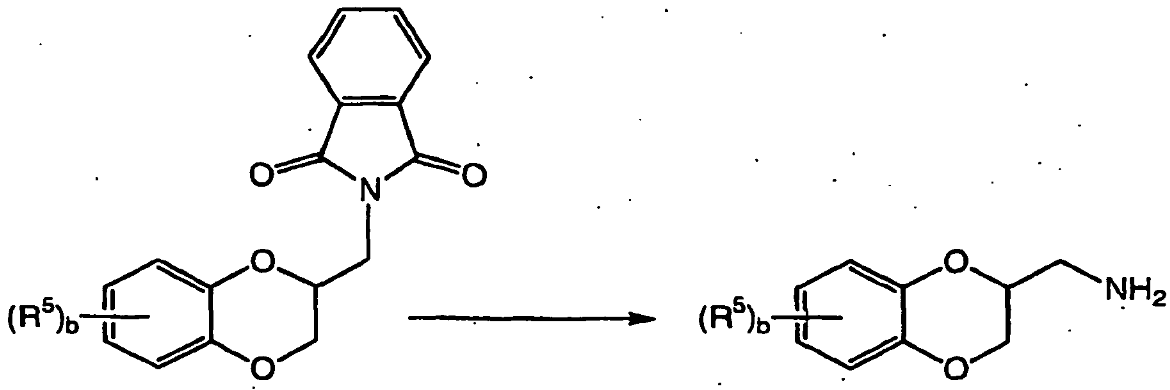

(XX) (Xd)

Scheme 5

Accordingly, a suitably substituted compound of formula (XVIII), a known compound or compound prepared by known methods is activated, according to known method, to yield the corresponding compound of formula (XIX), wherein $J^2$ is a suitable leaving group, such tosylate, Cl, Br, I, mesylate, triflate, and the like.

The compound of formula (XIX) is reacted with a phthalimide salt such as potassium phthlimide, sodium phthalimide, and the like, in an organic solvent such as DMF, DMSO, acetonitrile, and the like, preferably, at an elevated temperature in the range of from 50°C to about 200°C, more preferably, at about reflux temperature, to yield the corresponding compound of formula (XX).

The compound of formula (XX) is reacted with $N_2H_4$, a known compound, in an organic solvent such as ethanol, methanol, and the like, preferably, at an elevated temperature in the range of from about 50°C to about 100°C, more preferably, at about reflux temperature, and the like, to yield the corresponding compound of formula (Xd).

One skilled in the art will recognize that compounds of formula (X) wherein

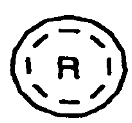

is selected from

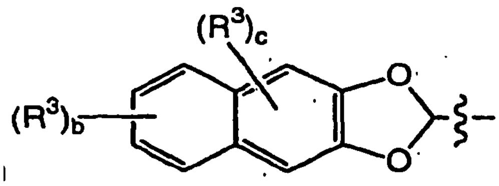

,

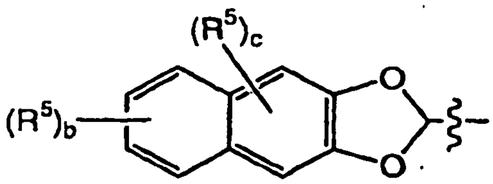

,

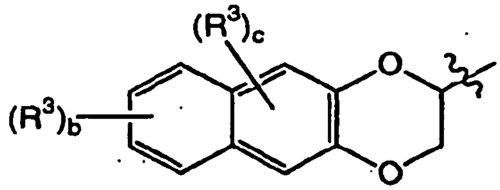

,

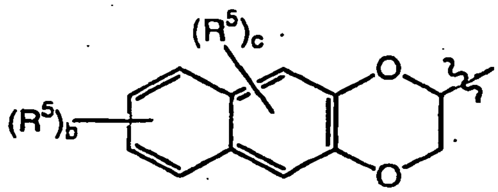,
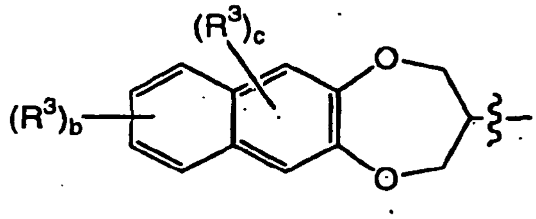

or

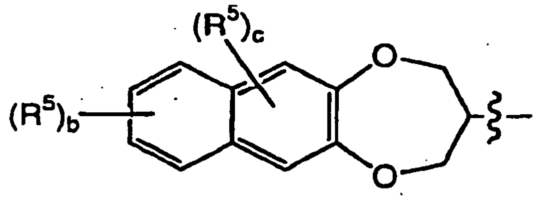

may be similarly prepared according to known methods or for example, according to the processes outlined in Schemes 2 through 5 above, by selecting and substituting the corresponding naphthyl-fused compounds for the benzo-fused starting materials.

One skilled in the art will further recognize that wherein a single enantiomer (or a mixture of enantiomers wherein one enantiomer is enriched) of a compound of formula (X) is desired, the above processes as described in Schemes 1 through 5 may be applied by substituting the corresponding single enantiomer (or mixture of enantiomers wherein one enantiomer is enriched) for the appropriate starting material.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the,compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The present invention further comprises pharmaceutical compositions containing one or more compounds of formula (I) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical

compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents; for solid oral preparations such as, for example, powders, capsules, caplets, gelcaps and tablets, suitable carriers and additives include starches, sugars, diluents, granulating agents, lubricants, binders, disintegrating agents. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful, an amount of the active ingredient necessary to deliver an effective dose as described above. The pharmaceutical compositions herein will contain, per unit dosage unit, e.g., tablet, capsule, powder, injection, suppository, teaspoonful, of from about 0.1-1000 mg and may be given at a dosage of from about 0.01-200.0 mg/kg/day, preferably from about 0.1 to 100 mg/kg/day, more preferably from about 0.5-50 mg/kg/day, more preferably from about 1.0-25.0 mg/kg/day or any range therein. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as com starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

Treating depression as described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery

system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water. Moreover, when desired or necessary, suitable binders; lubricants, disintegrating agents and coloring agents can also be incorporated into the mixture. Suitable binders include, starch, gelatin, natural sugars such as glucose or beta-lactose, com sweeteners, natural and synthetic gums such as acacia, tragacanth or sodium oleate, sodium stearate, magnesium stearate, sodium benzoate, sodium acetate, sodium chloride. Disintegrators include, without limitation, starch, methyl cellulose, agar, bentonite, xanthan gum.

The liquid forms in suitably flavored suspending or dispersing agents such as the synthetic and natural gums, for example, tragacanth, acacia, methyl-cellulose. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment of depression is required.

The daily dosage of the products may be varied over a wide range from 0.01 to 200 mg / kg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 200 mg/kg of body weight per day. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

The following Examples are set forth to aid in the understanding of the invention.

**Example 1**

**((3,4-Dihydro-2H-benzo[b][1,4]dioxepin-3-yl)methyl)sulfamide (Compound #3)**

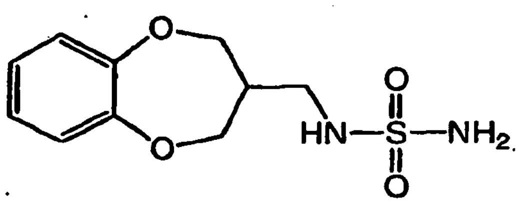

Catechol (5.09 g, 46.2 mmol) and potassium carbonate were combined in acetonitrile and heated to reflux for one hour. 2-Chloromethyl-3-chloro-1-propene (5.78 g, 46.2 mmol) was added and the reaction was continued at reflux for 24 hours. The solution was cooled to room temperature and filtered. The filtrate was evaporated and the residue was diluted with water and extracted with diethyl ether (3 x). The combined organic solution was dried over $MgSO_4$ and concentrated. Chromatography (2% ethyl ether in hexane) yielded 3-methylene-3,4-dihydro-2H-benzo[b][1,4]dioxepine as a colorless oil.

MS (ESI): 163.2 ($M+H^+$)

$^1H$ NMR (300 MHz, $CDCl_3$), δ: 6.94 (m, 4H), 5.07 (s, 2H), 4.76 (s, 4H).

3-Methylene-3,4-dihydro-2H-benzo[b][1,4]dioxepine (5.00 g, 30.8 mmol) was dissolved in dry THF (100 mL). Borane-THF (1.0 M in THF, 10.3 mL) was added at 0°C. The reaction was stirred at RT for 5 hours. Aminosulfonic acid

(6.97 g, 61.6 mmol) was added. The reaction was heated to reflux overnight. The reaction was cooled to room temperature and aqueous sodium hydroxide (3.0 M, 100 mL) was added. The solution was extracted with ethyl acetate (3 x 100 mL). The combined organic solution was dried over $MgSO_4$. The solution was concentrated under vacuum and purified by chromatography (2% to 8% methanol in dichloromethane) to yield ((3,4-dihydro-2H-benzo[b][1,4]dioxepin-3-yl)methyl) amine as a colorless oil.

MS (ESI): 180.1 ($M+H^+$)

$^1H$ NMR (300 MHz, DMSO), δ: 6.92 (m, 4H), 4.21 (m, 2H), 4.07 (m, 2H), 3.33 (broad, 2H), 3.16 (d, *J* = 4 Hz, 1H), 2.72 (d, *J* = 4·Hz, 1H), 2.30 (m, 1H).

((3,4-Dihydro-2H-benzo[b][1,4]dioxepin-3-yl)methyl)amine (2.90, g, 16.2 mmol) and sulfamide (3.11 g, 32.4 mmol) were combined in dry dioxane (60 ml) and heated to reflux overnight. Chloroform was added and the precipitate was removed by filtration. The filtrate was concentrated under vacuum and purified by chromatography (2% to 8% acetone in dichloromethane) to yield the title compound as an off-white solid.

258.8 ($M+H^+$)

$^1H$ NMR (300 MHz, DMSO), δ: 6.92 (m, 4H), 6.71 (broad, 1H), 6.59 (broad, 2H), 4.19 (m, 2H), 4.04 (m, 2H), 3.00 (m, 2H), 2.39 (m, 1H).

**Example 2**

**N-(2.3-Dihydro-benzo[1,4]dioxin-2-ylmethyl)-sulfamide (Compound #1)**

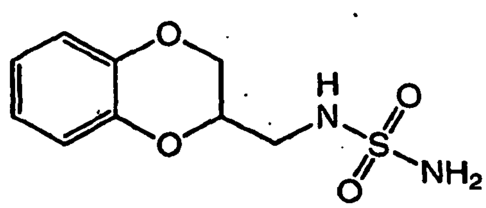

Racemic 2,3-dihydro-1,4-benzdioxin-2-ylmethylamine (4.4 g, 26 mmol) and sulfamide (5.1 g, 53 mmol) were combined in 1,4 dioxane (100 mL) and refluxed for 2 h. The reaction was cooled to room temperature and a small amount of solid was filtered and discarded. The filtrate was evaporated in vacuo and the residue was purified using flash column chromatography (DCM:Methanol - 10:1) to yield a white solid. The solid was recrystallized from DCM to yield the title compound as a white solid.

mp: 97.5 - 98.5˚C

| Elemental Analysis: | | | | |
|---|---|---|---|---|
| Anal Calc: | C, 44.25; | H, 4.95; | N, 11.47; | S, 13.13 |
| Anal Found: | C, 44.28; | H, 4.66; | N, 11.21; | S, 13.15 |

$H^1$ NMR (DMSO d6) δ 6.85 (m, 4H), 6.68 (bd s, 3H, NH), 4.28 (m, 2H), 3.97 (dd, J = 6.9, 11.4 Hz, 1H), 3.20 (m, 1H), 3.10 (m, 1H).

**Example 3**

**(Benzo[1,3]dioxol-2-ylmethyl)sulfamide (Compound #2)**

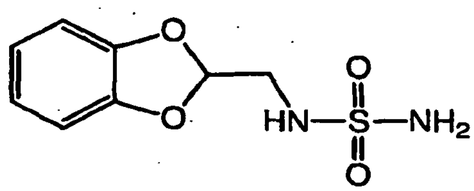

Catechol (10.26 g, 93.2 mmol), sodium methoxide (25% by weight in methanol, 40.3 g, 186 mmol), and methyl dichloroacetate (13.3 g, 93.2 mmol) were combined in dry methanol (100 mL). The solution was heated to reflux overnight. The reaction was cooled to room temperature, acidified by addition of concentrated hydrochloric acid and then reduced in volume under vacuum to about 50 mL. Water was added and the mixture was extracted with diethyl ether (3 x 100 mL). The combined organic solution was dried with $MgSO_4$, concentrated to a brown solid, and chromatographed (2% ethyl acetate in hexane) to yield benzo[1,3]dioxole-2-carboxylic acid methyl ester as a colorless oil.

MS (ESI): 195.10 ($M+H^+$).

$^1H$ NMR (300 MHz, $CDCl_3$), δ: 6.89 (broad, 4H), 6.29 (s, 1H), 4.34 (q, *J* =7 Hz, 2H), 1.33 (t, *J* =7 Hz, 3H).

To benzo[1,3]dioxole-2-carboxylic acid methyl ester (7.21 g, 40.0 mmol) was added ammonium hydroxide (29% in water, 10 mL) and enough acetonitrile to make the mixture homogeneous (~5 mL), The solution was stirred for two hours at room temperature and then distilled water was added. Benzo[1,3]dioxole-2-carboxylic acid amide precipitated as a white solid and was collected by filtration and used without further purification.

MS (ESI): 160.00 ($M+H^+$)

$^1H$ NMR (300 MHz, DMSO), δ: 7.99 (s, broad, 1H), 7.72 (s, broad, 1H), 6.94 (m, 2H) 6.86 (m, 2H), 6.30 (s, 1H).

Benzo[1,3]dioxole-2-carboxylic acid amide (5.44 g, 32.9 mmol) was dissolved in tetrahydrofuran (THF, 100 mL). Lithium aluminum hydride (LAH, 1 M in THF, 39.5 mL, 39.5 mmol) was added slowly to the solution at room temperature. The reaction was stirred at room temperature for 24 hours. Distilled water was added to destroy the excess LAH. Aqueous sodium hydroxide (3.0 M, 100 mL) was added and the solution was extracted with ethyl acetate (3 x 100 mL). The combined organic solution was washed with water and dried over $MgSO_4$. The solvent was evaporated to yield C-benzo[1,3]dioxol-2-yl-methylamine as a colorless oil.

MS (ESI): 152.1 ($M+H^+$)

$^1H$ NMR (300 MHz, $CDCl_3$), δ: 6.87 (m, 4H), 6.09 (t, *J* = 4 Hz, 1H), 3.13 (d, *J* = 4 Hz, 2H)

C-Benzo[1,3]dioxol-2-yl-methylamine (2.94 g, 19.4 mmol) and sulfamide (3.74 g, 38.9 mmol) were combined in dry dioxane (50 mL) and the solution was heated to reflux overnight. The reaction was concentrated and the residue was chromatographed (2% to 10% acetone in dichloromethane) to yield the title compound as a white solid.

MS (ESI): 230.0 ($M+H^+$)

$^1H$ NMR (300 MHz, $CDCl_3$), δ: 6.87 (m, 4H), 6.25 (t, *J* = 4 Hz, 1H), 4.79 (broad, 1H), 4.62 (broad, 1H), 3.64 (d, *J* = 4 Hz, 2H).

## Example 4

### (2S)-(-)-N-(2,3-Dihydro-benzo[1,4]dioxin-2-ylmethyl)-sulfamide (Compound #4)

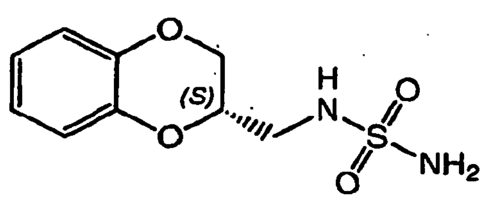

Catechol (13.2 g, 0.12 mol) and potassium carbonate (16.6 g, 0.12 mol) were stirred in DMF (250 mL) and (2R)-glycidyl tosylate (22.8 g, 0.10 mol) was added and the reaction was stirred at 60°C for 24 h. The reaction was cooled to room temperature and diluted with ice water (1 L) and extracted with diethyl ether (4 times). The combined organic solution was washed 3 times with 10% potassium carbonate, once with water, once with brine and evaporated in vacuo to yield a white solid which was purified by flash column chromatography (DCM:Methanol - 50:1) to yield ((2S)-2,3-dihydro-benzo[1,4]dioxin-2-yl)-methanol as a solid.

The solid (13.3 g, 68 mmol) was dissolved in pyridine (85 mL) cooled to 0°C, p-toluenesulfonyl chloride (13.0 g, 68 mmol) was added and the reaction mixture stirred at room temperature for 20h. The reaction was diluted with diethyl ether (1 L) and 1 N HCl (1.2 L). The organic layer was separated and washed 2 times with 1 N HCl (500 mL), 4 times with water (150 mL), once with brine, dried ($MgSO_4$) and evaporated in vacuo to yield a white solid which was purified by flash column chromatography (Hept:EA - 2:1) to yield toluene-4-sulfonic acid (2S)-2,3-dihydro-benzo[1,4] dioxin-2-ylmethyl ester as a white solid.

The white solid was combined with potassium phthalimide (14.4 g, 78 mmol) in DMF (250 mL) and heated to reflux for 1 h, cooled to room temperature and poured into vigorously stirring water (1.5 L) and stirred 30 min. White solid was filtered and the solid was washed several times with water, 2% NaOH, and water again and let air dry to yield

a (2S)-2-(2,3-Dihydro-benzo[1,4]dioxin-2-ylmethyl)-isoindole-1,3-dione as white powdery solid.

The powdery white solid was combined with hydrazine (2.75 g, 86 mmol) in EtOH (225 mL) and heated at reflux for 2 h, cooled to room temperature and 1 N HCl added to pH 1.0 and stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried ($Na_2SO_4$) and evaporated in vacuo to a yield a light yellow oil. The oil was purified by flash column chromatography (DCM:MeOH - 10:1) to yield an oil. A portion of the oil (4.82 g, 29 mmol) in 2-propanol (250 mL) was treated with 1 N HCl (30 mL) and heated on steambath until homogeneous and then let cool to room temperature. After 3 h, the mixture was ice cooled for 2 h. A white flaky solid (the corresponding HCl salt of (2S)-C-(2,3-Dihydro-benzo[1,4]dioxin-2-yl)-methylamine) was filtered off and then recrystallized again from 2-propanol to yield a white solid.

$[\alpha]_D$ = -69.6 (c = 1.06, EtOH)

The white solid was partitioned between DCM and dilute NaOH, and the DCM was dried ($NaSO_4$) and evaporated in vacuo to yield (2S)-C-(2,3-Dihydro-benzo[1,4]dioxin-2-yl)-methylamine as an oil.

$[\alpha]_D$ = -57.8 (c = 1.40, $CHCl_3$)

The oil (2.1 g, 12.7 mmol) and sulfamide (2.44 g, 25.4 mmol) were refluxed in dioxane (75 mL) for 2 h and the crude product was purified by flash column chromatography (DCM:MeOH 10:1) to yield a white solid, which was recrystallized from DCM to yield the title compound as a white crystalline solid.

mp 102-103°C

$[\alpha]_D$ = -45.1 ° (c = 1.05, M);

$^1H$ NMR (DMSOd6) δ 6.86 (m, 4H), 6.81 (bd s, 3H, NH), 4.3 (m, 2H), 3.97 (dd, J = 6.9, 11.4 Hz, 1H), 3.20 (dd, J = 5.5, 13.7 Hz, 1H), 3.10 (dd, J = 6.9, 13.7 Hz, 1H)

| Elemental Analysis: | | | | |
|---|---|---|---|---|
| Anal Calc: | C, 44.25; | H, 4.95; | N, 11.47; | S, 13.13 |
| Anal Found: | C, 44.20; | H, 4.69; | N, 11.40; | S, 13.22. |

**Example 5**

**N-(2,3-Dihydro-benzo[1,4]dioxin-2-ylmethyl)-N',N' dimethylsulfamide (Compound #6)**

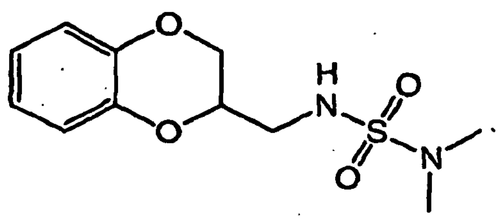

Racemic 2,3-dihydro-1,4-benzdioxin-2-ylmethylamine (8.25 g, 5.0 mmol) and triethylamine (1.52 g, 15 mmol) were combined in DMF (10 mL) and cooled in an ice bath as dimethylsulfamoyl chloride (1.44 g, 10 mmol) was added. The reaction mixture was then stirred for 3 hr with continued cooling. The reaction mixture was partitioned between ethyl acetate and water, and the ethyl acetate solution was washed with brine, dried ($MgSO_4$) and evaporated in vacuo to yield an oil. The oil was purified using flash column chromatography (ethyl acetate:Heptane - 1:1) to yield a white solid, which was recrystallized (ethyl acetate/Hexane) to yield the title compound as a white floccular solid.

mp 76 - 78°C

MS 273 ($MH^+$)

| Elemental Analysis: | | | | |
|---|---|---|---|---|
| Anal Calc: | C, 48.52; | H, 5.92; | N, 10.29; | S, 11.78 |
| Anal Found: | C, 48.63; | H, 5.62; | N, 10.20; | S, 11.90 |

$^1H$ NMR ($CDCl_3$) δ 6.87 (m, 4H), 4.59 (bd m, 1H, NH), 4.35 (m, 1H), 4.27 (dd, J = 2.3, 11.4 Hz, 1H), 4.04 (dd, J = 7.0, 11.4, 1H), 3.36 (m, 2H), 2.82 (s, 6H).

**Example 6**

**N-(2,3-Dihydro-benzo[1,4]dioxin-2-ylmethyl)-N-methylsulfamide (Compound #7)**

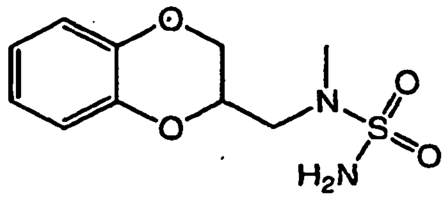

Racemic 2,3-dihydro-1,4-benzdioxin-2-ylmethylamine (825 mg, 5 mmol) was dissolved in ethyl formate (15 mL), refluxed for 30 min and evaporated in vacuo to yield N-(2,3-dihydro-benzo[1,4]dioxin-2-ylmethyl)-formamide as an oil.

The oil in diethyl ether (25 mL) was treated with 1 M LAH in THF (9.0 mL, 9.0 mmol) at 0˚C and stirred for 5 h at room temperature. The reaction was cooled in an ice bath and quenched with water (0.50 mL), followed by 3 N NaOH (0.50 mL) and water (0.50 mL). The mixture was then stirred at room temperature for 1 h. Solid was filtered and the filtrate was evaporated in vacuo to yield a residue which was partitioned between 1 N HCl and diethyl ether. The aqueous phase was basified with 1 N NaOH and extracted witch diethyl ether. The organic phase was dried ($MgSO_4$) and evaporated in vacuo to yield (2,3-dihydro-benzo[1,4]dioxin-2-ylmethyl)-methyl-amine as an oil.

MS 180 ($MH^+$)

$^1H$ NMR ($CDCl_3$) δ 6.85 (m, 4H), 4.30 (m, 2H), 4.02 (dd, J = 7.9, 11.6 Hz, 1H), 2.85 (m, 2H), 2.50 (s, 3H)

The oil (380 mg, 2.1 mmol) and sulfamide (820 mg, 8.5 mmol) were combined in dioxane (15 mL), refluxed for 1.5 h and evaporated in vacuo to yield a crude residue. The residue was purified via column chromatography (ethyl acetate/Heptane 1:1) and the resultant solid was recrystallized from ethyl acetate/Hexane to yield the title compound as a white solid.

mp 97-98˚C

MS 257 ($M^{-1}$)

| Elemental Analysis: | | | | |
|---|---|---|---|---|
| Anal Calc: | C, 46.50; | H, 5.46; | N, 10.85; | S, 12.41 |
| Anal Found: | C, 46.48; | H, 5.65; | N, 10.90; | S, 12.07 |

$^1H$ NMR ($CDCl_3$) δ 6.86 (m, 4H), 4.52 (bs, 2H), 4.46 (m, 1H), 4.29 (dd, J = 2.3, 11.5 Hz, 1H), 4.05 (dd, J = 6.5, 11.5 Hz, 1H), 3.51 (dd, J = 6.7, 14.9 Hz, 1H), 3.40 (dd, J = 5.9, 14.9 Hz, 1H), 2.99 (s, 3H).

**Example 7**

**(2S)-(-)-N-(6-Chloro-2,3-dihydro-benzo[1,4]dioxin-2-ylmethyl)-sulfamide (Compound #8)**

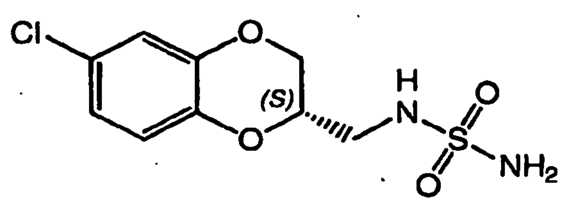

Following the procedure outlined in Example 4 above, 4-chlorocatechol was reacted to yield a mixture of (2S)-C-(7-Chloro-2,3-dihydro-benzo[1,4]dioxin-2-yl)-methylamine and (2S)-C-(6-Chloro-2,3-dihydro-benzo[1,4]dioxin-2-yl)-methylamine (ca. 3:1 ratio of 6-chloro:7-chloro isomers by RP HPLC).

The mixture was dissolved in 2-propanol (100 mL) and 1 N HCl in diethyl ether was added until pH = 1.0 was

attained. The hydrochloride salt that precipitated was filtered (2.65 g) and re-crystallized from methanol/IPA to yield white crystals. The white crystals were partitioned between DCM and dilute NaOH. The DCM was dried and evaporated in vacuo to yield purified (2S)-C-(6-Chloro-2,3-dihydro-benzo[1,4]dioxin-2-yl)-methylamine as an oil.

$[\alpha]_D$ = -67.8 (c =1.51, $CHCl_3$)

The oil (7.75 mmol) and sulfamide (1.50 g, 15.5 mmol) were combined in dioxane (50 mL) and refluxed for 2.0 h, cooled to room temperature and evaporated in vacuo to yield a solid. The product was purified via flash column using DCM/methanol 20:1 to yield the title compound as a white solid

MS 277 ($M^{-1}$)

$[\alpha]_D$ = -59.9° (c = 1.11, M)

$^1$H NMR ($CDCl_3$) δ 6.90 (d, J = 2.2 Hz, 1H), 6.81 (m, 2H), 4.76 (m, 1H), 4.55 (s, 2H), 4.40 (m, 1H), 4.29 (dd, J = 2.4, 11.5 Hz, 1H), 4.05 (dd, J = 7.1, 11.5 Hz, 1H), 3.45 (m, 2H)

| Elemental Analysis: | | | |
|---|---|---|---|
| Anal Calc: | C, 38.78; | H, 3.98; | N, 10.05 |
| Anal Found: | C, 38.80; | H, 3.67; | N, 9.99. |

The filtrates of the crystallized hydrochloride salt of (2S)-C-(6-Chloro-2,3-dihydro-benzo[1,4]dioxin-2-yl)-methylamine prepared above were recovered (ca. 1:1 of 6-chloro:7-chloro isomers) and evaporated in vacuo to yield a solid, which was partitioned between DCM (200 mL) and dilute NaOH (0.5 M, 50 mL). The DCM solution was washed once with brine, dried ($Na_2SO_4$) and evaporated in,vacuo to yield an oil, which was purified via reverse phase HPLC (10 - 50% ACN with 0.16% TFA in water with 0.20% TFA) to yield (2S)-C-(7-Chloro-2,3-dihydro-benzo[1,4]dioxin-2-yl)-methylamine as a residue.

The residue was combined with sulfamide (0.90 g, 9.4 mmol) in dioxane (25 mL) and refluxed for 2.5 h, cooled to room temperature and evaporated in vacuo to yield an oil. The oil was purified by flash column chromatography using DCM/methanol - 10:1 to yield (2S)-(-)-N-(7-Chloro-2,3-dihydro-benzo[1,4]dioxin-2-ylmethyl)-sulfamide as a white solid.

MS 277 ($M^{-1}$)

$^1$H NMR ($CDCl_3/CD_3OD$) δ 6.88 (d, J = 0.7 Hz, 1H), 6.81 (m, 2H), 4.37 (m, 1H), 4.30 (dd, J = 2.3, 11.6 Hz, 1H), 4.04 (dd, J = 7.0, 11.6 Hz, 1H), 3.38 (m, 2H).

**Example 8**

**Chroman-2-ylmethylsulfamide (Compound #10)**

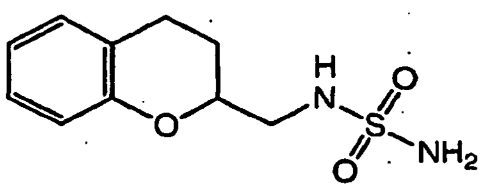

Chroman-2-carboxylic acid (4.5 g, 25 mmol) and HOBT (3.86 g, 25 mmol) were combined in DCM (40 mL) and DMF (10 mL). Dimethylaminopropyl ethylcarbodiimide (EDC, 4.84 g, 25 mmol) was added at room temperature and the reaction mixture was stirred for 30 min. Ammonium hydroxide (2.26 mL, 33.4 mmol) was added and the reaction mixture was stirred for 16h. The reaction mixture was diluted with DCM (50 mL) and water

(50 mL) and the pH of the mixture was adjusted to about pH = 3.0 with 1N HCl. The DCM was separated and the aqueous phase extracted twice with DCM. The combined DCM phase was dried ($Na_2SO_4$) and evaporated in vacuo to yield an oil, which was purified with flash column chromatography (ethyl acetate) to yield an oil.

The oil (5.35 g, 30 mmol) in THF (90 mL) was stirred as 1 M LAH in THF (36 mL, 36 mmol) was added and the reaction mixture was then stirred at room temperature for 20 h. The reaction was quenched with water, stirred for 2 hours, the solution decanted, dried ($Na_2SO_4$) and evaporated in vacuo to yield C-chroman-2-yl-methylamine as an oily amine.

The oily amine (1.63 g, 10 mmol) and sulfamide (1.92 g, 20 mmol) were combined in dioxane (50 mL) and brought to reflux for 2 h. The solution was cooled and evaporated in vacuo to yield an oil, which was purified via column chromatography (DCM:Methanol 10:1) to yield a white solid. The solid was recrystallized from ethyl acetate/hexane to

yield chroman-2-ylmethylsulfamide as a white solid.

mp 100-101˚C

MS 241 ($M^{-1}$)

| | | | | |
|---|---|---|---|---|
| Elemental Analysis: | | | | |
| Anal Calc: | C, 49.57; | H, 5.82; | N, 11.56; | S, 13.23 |
| Anal Found: | C, 49.57; | H, 5.80; | N, 11.75; | S, 13.33. |

**Example 9**

**2-(2,3-Dihydro-benzo[1,4]dioxin-2-yl)-ethylsulfamide (Compound #16)**

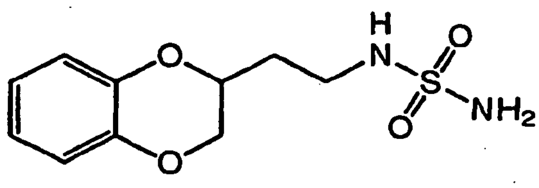

Potassium cyanide (2.05 g, 31.5 mmol) was added to 2-bromomethyl-(2,3 dihydrobenzo[1,4]dioxine) (6.87 g, 30 mmol) in DMSO (90 mL) and stirred at ambient temperature for 20 h. The reaction mixture was then diluted with water (250 mL) and extracted twice with diethyl ether. The diethyl ether was washed with water, then washed twice with brine, dried ($Na_2SO_4$) and evaporated in vacuo to yield 2-cyanomethyl-(2,3 dihydrobenzo[1,4]dioxine) as a white solid.

$^1$H NMR ($CDCl_3$) δ 6.89 (m, 4H), 4.50 (m, 1H), 4.31 (dd, J = 2.3, 11.5 Hz, 1H), 4.08 (dd, J = 6.2, 11.6 Hz, 1H), 2.78 (d, J = 6.1, Hz, 2H)

The 2-cyanomethyl-(2,3 dihydrobenzo[1,4]dioxine) was dissolved in THF (50 mL) and 1 M $BH_3$ in THF (80 mL, 80 mmol) was added and the reaction mixture refluxed for 5 h, then stirred at ambient temperature for 16h. With ice bath cooling, 2N HCl was added until pH = 1.0 was achieved. The reaction mixture was then stirred for 1 h at room temperature and evaporated in vacuo to yield an oil. The oil was partitioned between 3N NaOH and diethyl ether, and the diethyl ether solution was washed with brine, dried ($Na_2SO_4$) and evaporated in vacuo to yield crude 2-(2,3 dihydrobenzo[1,4]dioxin-2-yl)ethylamine.

MS $(M+H)^+$ 180.

The crude 2-(2,3 dihydrobenzo[1,4]dioxin-2-yl)ethylamine in dioxane (100 mL) was combined with sulfamide (3.0 g, 31 mmol) and heated to reflux for 2 h. The solution was cooled and evaporated in vacuo to yield an orange solid, which was purified by column chromatography (DCM:MeOH - 10:1) to yield a white solid. The solid was re-crystallized from DCM to yield the title compound as a solid.

MS (M-1) 257

MP 101 - 103˚C (corr)

$^1$H NMR ($CDCl_3$): δ 6.86 (m, 4H), 4.70 (m, 1H), 4.52 (s, 2H), 4.30 (m, 2H), 3.94 (dd, J = 7.4, 11.3 Hz, 1H), 3.43 (dd, J = 6.4, 12.9 Hz, 2H), 1.94 (dd, J = 6.5, 12.9, 2H).

| | | | | |
|---|---|---|---|---|
| Elemental Analysis: | | | | |
| Measured: | C, 46.48; | H, 5.60; | N, 10.81; | S, 12.41 |
| Calculated: | C, 46.50; | H, 5.46; | N, 10.85; | S, 12.41 |

**Example 10**

**(2S)-(-)-N-(6,7 Dichloro-2,3-dihydro-benzo[1,4]droxin-2-ylmethyl)-sulfamide (Compound #29)**

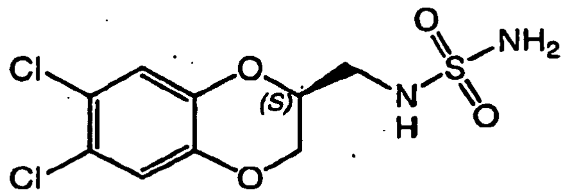

4,5 Dichloroatechol (8.6 g, 48 mmol) and potassium carbonate (6.64 g, 48 mmol) were stirred in DMF (200 mL). (2R)-Glycidyl tosylate (9.12 g, 40 mmol) was added and the reaction mixture was stirred at 60˚C for 24 h. The reaction mixture was cooled to room temperature and then diluted with ice water (600 mL) and extracted with diethyl ether (4 times). The combined organic solution was washed 3 times with 10% potassium carbonate, twice with brine, dried ($MgSO_4$) and evaporated in vacuo to yield a viscous oil of (2S)-2-(6,7-dichloro-2,3-dihydro-benzo[1,4]dioxine) methanol.

The (2S)-2-(6,7 dichloro-2,3-dihydro-benzo[1,4]dioxine) methanol oil (6.4 g, 27 mmol) was dissolved in pyridine (50 mL) cooled to 0˚C. Then, *p*-toluenesulfonyl chloride (5.2 g, 27 mmol) was added and the reaction mixture was stirred at room temperature for 20h. The reaction mixture was diluted with diethyl ether and 1 N HCl (750 mL) and the organic layer was separated and washed 2 times with 1 N HCl (250 mL), once with water (150 mL), twice with brine, dried ($MgSO_4$) and evaporated in vacuo to yield light yellow solid of toluene-4-sulfonic acid (2S)-6,7-dichloro-2,3-dihydro-benzo[1,4]dioxin-2-ylmethyl ester.

$^1$H NMR (CDCl3): δ 7.79 (d, J = 8.3 Hz, 2H), 7.36 (d, J = 8.0 Hz, 2H), 6.94 (s, 1H), 6.83 (s, 1H), 4.37 (m, 1H), 4.2 (m, 3H), 4.03 (dd, J = 6.3, 11.7 Hz, 1H), 2.47 (s, 3H).

Toluene-4-sulfonic acid (2S)-6,7-dichloro-2,3-dihydro-benzo[1,4]dioxin-2-ylmethyl ester (8.0 g, 20.5 mmol) was combined with potassium phthalimide (6.1 g, 33 mmol) in DMF (75 mL) and heated to reflux for 1 h, cooled to room temperature and poured into vigorously stirring water (0.5 L) and then stirred 30 min. White solid was filtered and the solid was washed several times with water, 2% NaOH, and water again and then let air dry to yield (2S)-2-(6,7-dichloro-2,3-dihydro-benzo[1,4]dioxin-2-ylmethyl)-isoindole-1,3-dione (6.0 g, 80%) as a white powdery solid.

The white powdery solid was combined with hydrazine (1.06 g, 33 mmol) in EtOH (80 mL) and heated at reflux for 2 h, then cooled to room temperature. 1 N HCl was added to adjust the reaction mixture's pH to pH 1.0 and the reaction mixture was then stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried ($Na_2SO_4$) and evaporated in vacuo to a yield a viscous oil of (2S)-2-aminomethyl-(6,7-dichloro-2,3-dihydro-benzo[1,4]dioxine).

$^1$H NMR (CDCl3): δ 6.98 (s, 1H), 6.96 (s, 1H), 4.25 (dd, J = 2.0, 11.2 Hz, 1H), 4.15 (m, 1H), 4.0 (m, 1H), 2.97 (d, J = 5.5 Hz, 2H)

A portion of the oil (3.8 g, 16 mmol) and sulfamide (3.1 g, 32.4 mmol) were refluxed in dioxane (100 mL) for 2 h and the crude product was purified by flash column chromatography (DCM:MeOH 20:1) to yield the title compound as a white solid, which was recrystallized from ethyl acetate / hexane to yield the title compound as a white crystalline solid.

MS $[M-H]^-$ 311.0

mp 119-121˚C

$[\alpha]_D$ = -53.4 ˚ (c = 1.17, M)

$^1$H NMR (DMSOd6): δ 7.22 (s, 1H), 7.20 (s, 1H), 6.91 (bd s, 1H), 6.68 (bd s, 2H), 4.35 (m, 2H), 4.05 (dd, J = 6.5, 11.5 Hz, 1H), 3.15 (m, 2H) Elemental Analysis:

| Elemental Analysis: | | | | | |
|---|---|---|---|---|---|
| Measured: | C, 34.52; | H, 3.22; | N, 8.95; | Cl, 22.64; | S, 10.24 |
| Calculated: | C, 34.64; | H, 2.68; | N, 8.87; | Cl, 22.94; | S, 10.35. |

**Example 11**

**(2S)-(-)-N-(7-Amino-2,3-dihydro-benzo[1,4]dioxin-2-ylmethyl)-sulfamide (Compound #36)**

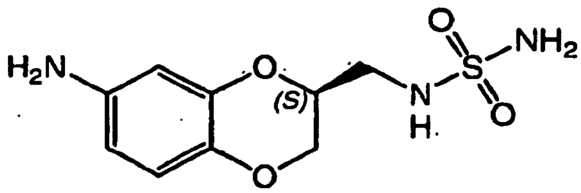

(2S)-(-)-N-(2,3-Dihydro-7-nitro-benzo[1,4]dioxin-2-ylmethyl)-sulfamide (1.2 g, 4.15 mmol), was prepared from 4-nitrocatechol according to the process outlined in Example 4. The (2S)-(-)-N-(2,3-Dihydro-7-nitro-benzo[1,4]dioxin-2-ylmethyl)-sulfamide, was then combined with 10% Pd/C in methanol (120 mL) and shaken under hydrogen atmosphere (39 psi) at room temperature for 3 h. The solids were filtered and washed with 10% M in DCM and the filtrate was evaporated in vacuo to yield crude product. The crude product was dissolved in 0.2 N HCl (25 mL), frozen and lyophilized to yield the title compound as a white flaky solid, as the corresponding hydrochloride salt.

MS $(M+H)^+$ 260

$^1$H NMR (DMSO d6): δ 10.2 (bd s, 3H), 6.86 (m, 1H), 6.85 (s, 1H), 6.74 (dd, J = 2.5, 8.4 Hz, 1H), 4.22 (m, 2H), 3.88 (dd, J = 6.7, 11.4 Hz, 1H), 3.04 (m, 2H)

**Example 12**

**(2S)-(-)-N-(7-Methyl-2,3-dihydro-benzo[1,4]dioxin-2-ylmethyl)-sulfamide (Compound #19)**

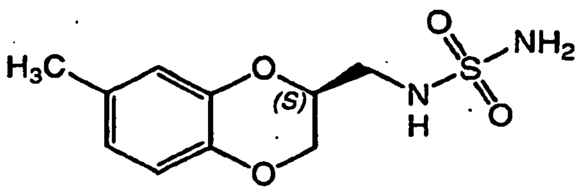

Title compound was prepared according to the procedure described in Example 4 above, starting with 4-methylcatechol, to yield a white solid, which was recrystallized from ethyl acetate/ hexane to yield the title compound as a white solid.

MS $[M-H]^-$ 257

$^1$H NMR (CDCl3): δ 6.76 (m, 1H), 6.66 (m, 2H), 4.80 (m, 1H), 4.57 (bd s, 1H), 4.40 (m, 1H), 4.28 (m, 1H), 4.03 (dd, J = 6.9, 11.4 Hz, 1H), 3.45 (m, 2H), 2.25 (s, 3H).

| Elemental Analysis | | | | |
|---|---|---|---|---|
| Calculated: | C, 46.50; | H, 5.46; | N, 10.85; | S, 12.41 |
| Found: | C, 46.65; | H, 5.60; | N, 10.84; | S, 12.61. |

**Example 13**

**Dominant-Submissive Relations (DSR) in Rat In Vivo Assay**

The DSR assay is divided into two models: Reduction of Dominant Behavior Model (RDBM) of mania and Reduction of Submissive Behavior Model (RSBM) of depression. The RDBM, wherein the dominant animals are treated with test compound, is predictive of the ability of the test compound to treat mania. The RSBM, wherein the submissive animals are treated with test compound, is predictive of the ability of the test compound to treat depression.

Male Sprague Dawley rats (140 to 160g) from Charles River Laboratories Wilmington, MA were used in this assay. Shipments of rats were received at two-week intervals. Each shipment went through five-day quarantine, one-week acclimation period, and one-week selection process, followed by five-weeks of drug or vehicle treatment to those pairs selected.

Rats were housed four per cage. Access to food was restricted to one hour per day after testing on Monday through Thursday. After testing on Friday, rats had free access to food until being fasted again on Sunday. At no time

were the rats deprived of water. The food deprivation periods used had little effect on weight gain as the average weight of rats was about 300g at the end of the study. At the conclusion of experiment rats were sacrificed by decapitation, the trunk blood and brains were collected for in vitro experiments and drug concentration measurements.

The basic testing apparatus consisted of two chambers connected with a tunnel only large enough to allow one rat to pass through at a time. On the floor, at the mid-point of the tunnel was a container of sweetened milk. This basic apparatus was replicated, so that a total of four pairs of rats can be video tracked simultaneously. The camera can distinguish rats marked by different colors. Thus, the rats' heads were colored for the purpose of video tracking, red in one cage and yellow in the other cage. Only one animal at a time can have comfortable access to the feeder, but both animals can drink milk during the five-minute daily session. During the five-minute daily sessions, time spent in the feeder zone by each rat was recorded by the video tracking software and saved into a text file.

The test began with a random assignment of rats into pairs. Each member of a pair was placed in an opposite chamber of the testing apparatus. The time spent in the feeder zone by each animal was recorded. During the first week (five days) of testing the animals habituate to the new environment. Dominance was assigned to the animal with the highest score during the second week of testing if three criteria were achieved. First, there must have been a significant difference (two-tailed t-test, $P<0.05$) between the average daily drinking scores of both animals. Second, the dominant animal score must have been at least 25% greater than the submissive animal's score. Finally, there must have been no "reversals" during the pair selection week where the putative submissive rat out-scored its dominant partner on isolated occasions. Ideally there were minimal reversals during the acclimation week as well. About twenty-five to thirty-three percent of the initial animal pairs achieved these criteria and only these pairs were continued in the study.

Terminal blood samples (0.5-1.0 mL) were collected post experiment into heparinized tubes. Blood samples were centrifuged for cell removal, and 200 μL of plasma supernatant was then transferred to a clean vial, placed on dry ice, and subsequently stored in a -80°C freezer prior to analysis. Two hundred microliters of acetonitrile containing internal standard was added to 100 μl- of plasma or brain tissue to precipitate proteins and/or tissue residues. Samples were centrifuged and supernatant removed for analysis by liquid chromatography-triple quadruple mass spectrometry (LC-MS-MS). Calibration standards were prepared by adding appropriate volumes of stock solution directly into blank plasma or brain tissue homogenates and treated identically to collected samples. Calibration standards were prepared in the range of 0.01 to 10 μM for quantitation. LC-ESI-MS/MS (negative mode) analysis was performed utilizing multiple reaction monitoring (MRM) for detection of characteristic ions for the test compound.

Significant differences between time spent on the feeder by dominant and submissive rats were determined by ANOVA using GraphPad Prism software (GraphPad Software, Inc. San Diego, CA) followed by a two-tailed t-test ($P<0.05$). Comparisons were made between treatment groups using normalized dominance level values in paired animals. The dominance level is a value that measures social relation between paired subjects. Dominance level (DL) = FTD-FTS where FTD is the feeder time of dominant rats and FTS is the feeder time of submissive rats. The normalization was conducted according to the formula:

$$\text{Dominance Level (week n in \%)} = \text{(Dominance Level (week n))} / \text{(Dominance Level (week 2)}$$

The statistical significance of the difference in dominance level between the control group (pairs of rats where both dominant and submissive animals were treated with vehicle) and the treatment group (submissive rats were treated with drug and dominant rats with vehicle) was determined by ANOVA, followed by a t-test. The activity onset time value at 50% of response (AOT-50) and the minimum and maximum response to drug were calculated based on the reduction of the dominance level value using non-linear regression analysis (GraphPad Software, Inc., San Diego, CA). The normalized DL values were used for this calculation, where DL values for treatment weeks were normalized as a percent of the second week (pretreatment) value of that pair according the above formula. In these settings the minimum of the response (DL) determined drug positive activity, corresponding to efficacy, since DL values were always reduced if the response to a drug was positive. In the case of the negative response to a drug (worsening of symptoms) DL values were increased. If the drug did not have such activity the maximum of the response did not exceed 100%. Any maximal DL value significantly higher then control value (about 100%) indicated drug negative activity.

Compound #8 was evaluated in the Rat Reduction of Submissive Behavior Model (RSBM) of depression (Malatynska, E., Rapp, R., Harrawood, D., and Tunnicliff, G., Neuroscience and Biobehavioral Review, 82 (2005) 306-313; Malatynska, E., and Knapp, R.J., Neuroscience and Biobehavioral Review, 29 (2005) 715-737).

More specifically, Compound #8 was administered p.o. (orally) to the submissive rats at 2.5 mg/kg (n=8), 12 mg/kg (n=12), 60 mg/kg (n=12) and 120 mg/kg (n=7), once a day for 5 weeks while the dominant partners were dosed with vehicle (0.5% aqueous methylcellulose). As controls, additional groups of rats were treated i.p. with fluoxetine at 10.0 mg/kg (n=10) and i.p. venlafaxine at 30.0 mg/kg (n=6). All treatments were administered approximately 1 hour prior

to testing. Compound #8 was observed to reduce submissive behavior in a dose-dependent manner.

When submissive animals were treated with Compound #8, the significant difference between dominant and submissive rats was lost after the first week of treatment. This was true for all doses used, indicating that the onset of activity was independent from the dose. By contrast, when submissive animals were treated with fluoxetine the significant difference between dominant and submissive rats was lost after third week of treatment. (This method of data analysis did not take into account the fluctuation of behavior that occurs in the control group.) To compare different drug and dose effects the data were normalized to the initial control week values.

Dominance Level (DL) values in the group of submissive rats treated with 2.5 mg/kg dose of Compound #8 did not significantly differ from control. However, the group treated with Compound #8 at 12.0 mg/kg showed DL values significantly different from vehicle treated controls after the second, fourth and fifth week of treatment. Similarly, the group treated with Compound #8 at 60 mg/kg showed a significant difference in DL values relative to vehicle starting at the first week and continuing through the treatment duration of 5 weeks. At the highest dose, (120 mg/kg) Compound #8 DL values were significantly different from the control group after first week, however, this significance dissipated after the second week of treatment.

Fluoxetine treated animals (10 mg/kg) consistently showed increased submissiveness during first week of treatment. In comparison with fluoxetine treated animals (10 mg/kg), Compound #8 treated groups did not show this effect. At 60.0 mg/kg dose of Compound #8, the difference in DL values with fluoxetine treated group was statistically significant at $p<0.001$ after first week and $p<0.05$ after second week of treatment. There was no significant difference between normalized DL levels of pairs treated with fluoxetine and Compound #8 during subsequent treatment weeks.

To estimate activity onset time, daily average values for feeder time of dominant and submissive animal pairs were plotted and significant differences between these two groups were calculated using the two-tail t-test. The first day of consistent lack of statistical significance occurred after treatment with Compound #8 at 12.0 mg/kg on the $6^{th}$ day and at 60 mg/kg on the $4^{th}$ day. There was no consistent loss of significance between dominant and submissive rats feeder time after treatment with Compound #8 at 2.5 mg/kg and 120.0 mg/kg.

To compare activity onset time between different treatments the activity onset time was estimated from the non-linear regression fit. The non-linear regression model was fit for each drug and dose normalized daily DL values. Activity Onset Time at the 50% effect ($AOT_{50}$) and Emax for Compound #8 at 2.5 mg/kg, 12 mg/kg and 60 mg/kg was 2.1; 5.3 and 1.6 days, respectively and was not significantly different between doses. The maximum of the effect derived from this analysis was 52.4 $\pm$ 32.7% (SEM), 87.9 $\pm$ 42.6% (SEM) and 116.9 $\pm$ 29.5% (SEM) for the 2.5 mg/kg, 12 mg/kg and 60 mg/kg dose respectively and was also not significantly different between these doses.

In summary, the effect of Compound #8 in the RSBM assay was dose dependent, with a calculated $ED_{50}$ of 6.6 $\pm$ 0.8 mg/kg [CI = 3.0 -10.2] and $E_{max}$ of 131.4 $\pm$ 4.7% [CI = 111.3 - 151.5].

In this assay, Compound #8 reduced submissive behavior indicating that the compound is active as an anti-depressant agent.

## Example 14

## Mouse Tail Suspension Assay (Acute)

In the tail suspension test (TST) for evaluating compounds for anti-depressant activity, mice are suspended by their tails to a metal or plastic rod using clip or scotch tape. The test is usually quite short, 5-7 min, and the amount of time the mice spend immobile is recorded either manually or with an automated device. Agents which have antidepressant activity, decrease the duration of immobility of mice in this test.

The basic apparatus for the tail suspension assay consisted of a yellow plastic chamber (91 x 45 x 10 cm) divided for four arenas 25, 20, 20 and 25 cm wide separated by yellow plastic walls 0.75 cm thick. Mice were suspended by their tails using a rubber clip (7 cm long) attached to the plastic rod that was placed on the top of a testing chamber half way through its deep dimension. Each experimental session was videotaped and analyzed for four animals in the real time by computer software ("Depression Scan" Clever Sys Inc.). The computer justification of immobility was calibrated with the use of animals dosed with lorazepam while justification of movement was calibrated with the high dose of desipramine treated animals. Control, vehicle treated animals and animals treated with Compound #8 were analyzed under the calibrated settings. The settings were adjusted separately for dark (CH3/HeJ and C57Bl/6J strains) and white mice (Balb/cJ and A/J strains). A yellow background was used for dark mice and a blue background was used to record movements of white mice.

The ability of a test compound to decrease the duration of immobility, or increase mobility was measured using the TST procedure described above. Acute treatment with clinically effective antidepressants and/or novel compounds that have potential antidepressant properties decrease the duration of immobility and at the same time increases mobility in the TST.

Data were analyzed using GraphPad Prism software (GraphPad Software, Inc. San Diego, CA). For the comparison of the effect of different doses for various drugs on immobility in the TST one-way analyses of variance (ANOVA) were used followed by Dunnett's multiple comparison test. The $ED_{50}$ and $E_{max}$ values were calculated for DMI, VEX, DLX and Compound #8 using non-linear regression analysis with one phase exponential decay equation for curve fitting. The $ED_{50}$ and $E_{max}$ values were compared using two-way ANOVA and Bonferroni post-hoc test.

The dose-response for different antidepressants and Compound #8 in the $CH_3$/HeJ mice was evaluated. Compound #8 was suspended in 0.5% of methylcellulose. Positive controls included duloxetine (DLX), venlafaxine (VLX) and desipramine (DMI) which were dissolved in 0.5% methylcellulose and lorazepam (LOR) which was suspended in 0.5% of methylcellulose in water by sonication. All drugs and vehicles were administered orally (p.o.) by gavage in a volume of 10 mL/kg.

The mice were ordered 5 weeks old and at the beginning of experiment their weight was 20 ± 5g. Animals were housed in groups of four in plastic cages at an ambient temperature of 21 ˚C to 23˚C with an automated 12/12 hours light/dark cycle and access to water and a commercial rodent food *ad libitum.*

This group was divided into eight experiments testing the effects of Compound #8, positive controls (DMI, VLX, DLX) at different doses and negative control (LOR) at 5 mg/kg. Each experiment consisted of seven treatment groups with four animals per group. A total of 28 animals per experiment were used. Every two consecutive experiments (1 & 2, 3 & 4, 5 & 6 and 7 & 8) were exact replicas of each other. This resulted in a total number of eight animals per treatment group at the end of the study. One treatment group of four animals in each experiment was a vehicle treated group. In addition to the vehicle treated group, the effects of DMI at 6 mg/kg, 12 mg/kg, 30 mg/kg, 60 mg/kg and 120 mg/kg and LOR at 5 mg/kg were tested in **experiments 1 and 2.** In **experiments 3 and 4** the effects of Compound #8 at 6 mg/kg, 12 mg/kg, 30 mg/kg, 60 mg/kg, 120 mg/kg and 240 mg/kg were tested. In **experiments 5 and 6** the effects of DLX at 6 mg/kg, 12 mg/kg, 30 mg/kg, 60 mg/kg, 120 mg/kg and LOR at 5 mg/kg were tested. **In experiments 7 and 8** the effects of VLX at 6 mg/kg, 12 mg/kg, 30 mg/kg, 60 mg/kg, 120 mg/kg and LOR at 5 mg/kg were tested. In the course of the study one mouse died due to miss-dosing in the group treated with Compound # 8 12 mg/kg, so this group consisted of seven animals on the end of the study.

Compound #8 and all tested antidepressant drugs decreased immobility time and increased mobility time in CH3/HeJ mice during a 7-min testing session. Compound #8 effects were statistically significant at 12 mg/kg, 60 mg/kg, and 120 mg/kg. Significance was determined in comparison to parallel controls treated with vehicle.

DMI effects were statistically significant at 12, 30, 60 and 120 mg/kg. VLX effects were statistically significant at 6, 12, 30, 60 and 120 mg/kg. DLX effects were statistically significant at 60 and 120 mg/kg.

$ED_{50}$ and $E_{max}$ values were calculated from these results by non-linear regression analysis. $ED_{50}$ and $E_{max}$ values are listed in Table 3 below. $ED_{50}$ values calculated for immobility and mobility were not significantly different across treatments. The $ED_{50}$ value for Compound #8 was significantly lower than the $ED_{50}$ value for DLX but not different than the $ED_{50}$ values for DMI and VLX. The $E_{max}$ values calculated for immobility and mobility were not significantly different for Compound #8 but were significantly different for all tested antidepressant. The $E_{max}$ immobility value for Compound #8 was also significantly lower then than antidepressant drug values.

**Table 3: $ED_{50}$ and $E_{max}$ Values for Different Drugs in the TST**

| Mobility | | | |
|---|---|---|---|
| **Drug** | **$ED_{50}$ mg/kg** | **SEM $ED_{50}$** | **CI** |
| Cmpd #8 | 3.6 | 2.9 | -4.6 - 11.8 |
| DMI | 24.4 | 9.0 | -0.5- 49.3 |
| VLX | 21.1 | 8.4 | -2.1 - 44.4 |
| DLX | 61.5 | 20.9 | 3.6 -119.3 |
| **Drug** | **$E_{max}$ % of C** | **SEM $E_{max}$** | **CI** |
| Cmpd #8 | 95.6 | 13.0 | 59.4 -131.7 |
| DMI | 195.7 | 24.9 | 126.7 - 264.7 |
| VLX | 412.6 | 53.9 | 263.0 - 562.2 |
| DLX | 175.5 | 28.3 | 96.9 - 254.2 |

(continued)

| Immobility | | | |
|---|---|---|---|
| Drug | $ED_{50}$ mg/kg | SEM $ED_{50}$ | CI |
| Cmpd #8 | 5.6 | 3.3 | 1.7 -12.1 |
| DMI | 27.4 | 16.0 | 11.3 -55.0 |
| VLX | 25.0 | 15.4 | 8.8 - 55.1 |
| DLX | 31.4 | 15.7 | 22.4 - 42.6 |
| Drug | $E_{max}$ % of C | SEM $E_{max}$ | CI |
| Cmpd #8 | 22.2 | 6.1 | 2.8 - 41.6 |
| DMI | 38.5 | 6.3 | 18.5 - 58.5 |
| VLX | 63.7 | 11.1 | 28.4 - 98.9 |
| DLX | 80.4 | 6.4 | 59.9 - 100.9 |

In summary, studies described in this example show that Compound #8 has antidepressant-like activity, as measured by the tail suspension test. The $ED_{50}$ for Compound #8 was calculated as 3.6 $\pm$ 2.9 mg/kg and the $E_{max}$ calculated as 22.2 $\pm$ 6.1% under the condition of our study.

## Example 15

## Forced Swim Test (Acute)

The Forced Swim test (FST) is a commonly used procedure to screen compounds for possible antidepressant properties. This test is also known as the behavioral despair test. Rodents placed in familiar tanks filled with water present a wide variety of escape or immobility behaviors. Antidepressant drugs from different classes markedly increase escape behaviors and/or decrease latency or duration of immobility. Since these effects are characteristic of clinically active antidepressants the compounds with unknown clinical activity showing such effects in the FST are interpreted to have potential to treat human mood disorders.

Compound #8 and maprotyline were dissolved in 10% solutol. Venlafaxine and desipramine were dissolved in water. All drugs and their vehicles were administered orally (p.o.) by gavage in a volume of 5 mL/kg.

Male Sprague Dawley rats (140 to 160 g) from Charles River Laboratories Wilmington, MA were used. The animals went through a five-day quarantine period before being subjected to the experimental procedure.

Animals were housed in groups of four in plastic cages at an ambient temperature of 21˚C to 23˚C with an automated 12 hour light/dark cycle and access to water and commercial rodent food *ad libitum.* Animals were not handled more than for the routine bedding change prior to pre-test swim session.

The study was divided into six experiments testing the effects of Compound #8, three positive controls (desipramine, maprotyline, venlafaxine) and a negative control (lorazepam) at different doses. Each experiment consisted of seven treatment groups with n = 4 animals per group. A total of 28 animals per experiment were used. Two consecutive experiments (1 and 2, 3 and 4, and 5 and 6) were exact replicas of each other. This resulted in a total number of eight animals per treatment group at the end of the study. One treatment group of n = 4 animals in each experiment was a vehicle treated group. In addition to the vehicle treated group, the effects of desipramine at 3 mg/kg, 6 mg/kg, 12 mg/kg, 30 mg/kg and 60 mg/kg and lorazepam at 1 mg/kg were tested **in experiments 1 and 2.** In **experiments 3 and 4** the effects of Compound #8 at 3 mg/kg, 6 mg/kg, 12 mg/kg, 30 mg/kg, 60 mg/kg and 120 mg/kg were tested. In experiments 5 and 6 the effects of venlafaxine and maprotyline at 12 mg/kg, 30 mg/kg, and 60 mg/kg were tested. In the course of the study one rat died due to miss-dosing in group treated with desipramine 12 mg/kg, so this group consisted of seven animals on the end of the study.

The basic apparatus consisted of a cylinder (46 cm tall x -20 cm diameter) filled with water to 30 cm deep, at a temperature of 25 $\pm$ 1˚C. The automated version of the FST was used to perform the experiments. Plumbing for automatic filling and emptying water connected the four cylinders. Cylinders were placed in the dividing chambers 25 cm wide to separate animals visually. Each 5-minute experimental session was videotaped and analyzed in real time by computer software (Clever Systems, Inc.) for four animals at a time. The immobility, swimming, climbing and escape times were recorded by the software. The four activities are defined as follow. Immobility: the animal floats motionlessly or makes only those movements necessary to keep its head above water; Climb: the animal vigorously moves vertically

while scratching the wall around the cylinder; Swim: the animal moves horizontally around in the cylinder more than necessary to keep its head above water; and Escape: sum of all vigorous active movements.

The ability of a test compound to decrease the duration or frequency of immobility, or changes in swimming, climbing and escape times, was measured using the FST procedure described above. Clinically effective antidepressants and/or novel compounds that have potential antidepressant properties decrease the duration or frequency of immobility in the FST when administered between the pre-test and the test sessions. The analysis of the results in the studies described was focussed on the immobility time during 5-minute test session.

There were two swim sessions in each experiment. First, a pre-test swim session for 15 minutes was performed. Following 48 hours later was a test session of 5-minute duration. Upon completion of a swim session, each animal was placed under a heat lamp in a cage with soft bedding for approximately 15 minutes to prevent hypothermia.

Animals were pre-treated with a vehicle or test compound after completing the pre-test swim session, then 24 hours later and then shortly prior to the 5-minute test session; i.e., three injections were given to each animal between the two swim sessions that occurred on 3 consecutive days. The time prior to test session was 1 hour for desipramine, maprotyline, venlafaxine, lorazepam or 4 hours for Compound #8; the time of the maximal effect in the maximal electroshock seizures (MES) test.

Data were analyzed using GraphPad Prism software (GraphPad Software, Inc. San Diego, CA). For the comparison of the effect of different doses for various drugs on immobility in the FST one-way ANOVA was used followed by Dunnett's multiple comparison test. The $ED_{50}$ and $E_{max}$ values were calculated for desipramine and Compound #8 using non-linear regression analysis with a one-phase exponential decay equation for curve fitting. The $ED_{50}$ and $E_{max}$ values were statistically compared using two-tait t-test.

All tested antidepressant drugs decreased immobility time during the 5-minute testing session. The desipramine effect was statistically significant at 6 mg/kg, 12 mg/kg, 30 mg/kg, and 60 mg/kg. The calculated $ED_{50}$ for desipramine was 2.0 $\pm$ 0.1 mg/kg (CI = 1.3 - 3.3 mg/kg) and its $E_{max}$ was 50.0 $\pm$ 8.4 seconds (CI = 31.8 - 57.7). The effect of treatment with Compound #8 was statistically significant at 12 mg/kg, 60 mg/kg, and 120 mg/kg compared to vehicle treated controls. The large variability between individual rats rendered the effect of the 30 mg/kg dose of Compound #8 not significantly different from control. For this reason the immobility data for 30 mg/kg dose were not used in the $ED_{50}$ calculation. The $ED_{50}$ calculated for Compound #8 was 5.6 $\pm$ 0.6 mg/kg (CI = 2.2 -15.6 mg/kg) and its $E_{max}$ was 67.0 $\pm$ 11.6 seconds (CI = 30.3 - 103.8). The $ED_{50}$ value for Compound #8 was significantly different from the $ED_{50}$ value for desipramine at $p < 0.001$ (two tail t-test). There was no statistically significant difference between $E_{max}$ values for desipramine and Compound #8. Venlafaxine and maprotyline (postive controls) were tested only at three doses of 12 mg/kg, 30 mg/kg, and 60 mg/kg. The immobility of animals treated at the 30 mg/kg and 60 mg/kg doses were significantly different from vehicle-treated controls for both venlafaxine and maprotyline. However, there were too few data points to calculate an $ED_{50}$ for these two drugs. Lorazepam (negative control) was tested at 1 mg/kg and showed no significant effect on the rats' immobility time during the testing session. The results indicate that Compound #8 has antidepressant-like activity in the FST.

**Table 4. $ED_{50}$ and $E_{max}$ values for Compound #8 and DMI in the FST**

| | $ED_{50}$ mg/kg | SEM $ED_{50}$ | CI $ED_{50}$ | $E_{max}$ % | SEM $E_{max}$ | CI $E_{max}$ |
|---|---|---|---|---|---|---|
| Compound #8 | 5.6 | 0.6 | 2.2 - 15.6 | 57.0 | 11.6 | 30.3 - 103.8 |
| DMI | 2.0 | 0.1 | 1.3-3.3 | 50.0 | 8.4 | 31.8-57.7 |

## Example 16

## Chronic Mild Stress Model (Chronic)

In the chronic mild stress (CMS) model rats subjected to a variety of mild stressors for a prolonged period of time show, among other behavioral, biochemical and physiological impairments, a substantial decrease in their responsiveness to rewarding stimuli. This deficit is usually monitored by a decrease in the consumption of the 1% sucrose solution, but can also be seen in other tests, such as place preference conditioning or intracranial self-stimulation. Since the subsensitivity to reward appears to reflect anhedonia (inability to experience pleasure), which is a core symptom of major depressive disorders, the CMS procedure may serve as a suitable research tool in studies into the mechanisms of antidepressant action.

Male Wistar rats were brought into the laboratory two months before the start of the experiment. Except as described below, the animals were singly housed with food and water freely available, and were maintained on a 12-h light/dark and in a constant temperature (22 $\pm$ 2°C) and humidity (50 $\pm$ 5%) conditions.

The animals were first trained to consume a 1% sucrose solution; training consisted of eight 1 h baseline tests

in which sucrose was presented, in the home cage, following 14h food and water deprivation; the sucrose intake was measured by weighing pre-weighed bottles containing the sucrose solution, at the end of the test. Subsequently, sucrose consumption was monitored, under similar conditions, at weekly intervals throughout the whole experiment.

On the basis of their sucrose intakes in the final baseline test, the animals were divided into two matched groups. One group of animals was subjected to the chronic mild stress procedure for a period of 7 consecutive weeks. Each week of stress regime consisted of: two periods of food or water deprivation, two periods of 45 degree cage tilt, two periods of intermittent illumination (lights on and off every 2h), two periods of soiled cage (250 ml water in sawdust bedding), one period of paired housing, two periods of low intensity stroboscopic illumination (150 flashes/min), and three periods of no stress. All stressors were 10 - 14 h of duration and were applied individually and continuously, day and night. Control animals were housed in separate rooms and had no contact with the stressed animals. They were deprived of food and water for the 14h preceding each sucrose test, but otherwise food and water were freely available in the home cage.

On the basis of their sucrose intakes following initial 2 weeks of stress, both stressed and control animals were each divided further into matched subgroups (n = 8), and for subsequent five weeks they received once daily intraperitoneal administration of vehicle (0.5% methylcellulose, 1 ml/kg), Compound #8 at 12 mg/kg, 30 mg/kg or 60 mg/kg, imipramine at 10 g/kg or venlafaxine at 10 mg/kg as reference treatments: The drugs were administered at approx. 10.00 and the weekly sucrose tests were carried out 24h following the last drug injections. After five weeks all treatments were terminated and 24h later the blood and/or brain samples were collected from all animals and submitted for further biochemical analysis. Stress was continued throughout the entire period of treatment.

Animals were individually removed from their housing rooms to another room for sacrifice. Then they were decapitated in a semi-randomized order. Whole brains were be removed, rapidly frozen in dry ice/n-heptane immediately after sacrifice and stored in plastic vials at -70 ˚C. Trunk blood for plasma was collected into EDTA tubes which contained EDTA (approximately 1.6mg/ml of blood). The EDTA blood was centrifuged directly at 1500 xg for 10 min at 4˚C. The plasma was aspirated and stored in Eppendorf tubes at -70˚C. Additionally, 2 lots of 20ml of plasma from naive animals were prepared for generating a compound standard curve for bioanalysis.

All results obtained in this study were analyzed by multiple analyses of variance with three between-subjects factors (stress/control, drug treatments and successive sucrose tests). The Fisher's LSD test was used for the post-hoc comparisons of means.

Chronic mild stress caused a gradual decrease in the consumption of 1% sucrose solution. In the final baseline test, all animals drank approx. 11 g of sucrose solution. Following the initial two weeks of stress, intakes remained at a similar level in controls but fell to approx. 6 g in stressed animals, resulting in a significant Group effect [$F(1,84) = 87.204$; $p<0.001$]. Such a difference between control and stressed animals treated with vehicle, persisted at similar level for the remainder of the experiment.

As compared to vehicle administration, **Imipramine** was inactive in \ controls [Treatment effect: $F(1,84) = 1.578$; NS] and caused significant Treatment effect: $F(1,84) = 22.651$; $p<0.001$ and Treatment x Weeks interaction: $F(5,84) = 2.717$; $p=0.025$] in stressed animals. Similarly, **Venlafaxine** was inactive in controls [Treatment effect: $F(1,84) = 0.208$; NS] and caused significant Treatment effect: $F(1,84) = 35,724$; $p<0.001$ and Treatment x Weeks interaction: $F(5,84) = 3.219$; $p=0.010$] in stressed animals.

As compared to Week 0 scores, the increases in sucrose intake in stressed animals reached statistical significance after four weeks of treatment with imipramine ($p<0.05$) and venlafaxine ($p<0.01$) and this effect was maintained thereafter. One stressed animal (no 480) did not respond to venlafaxine treatment but it was not excluded from the statistical analysis.

As compared to vehicle administration, Compound #8 did not cause significant Treatment effects in control [$F(3,168) = 1.198$; NS] and in stressed [$F(3,168) = 1.676$; NS] animals, indicating that the compound is inactive in the CMS model of depression.

## Example 17

## Resident / Intruder Assay (also known as Chronic Social Stress Assay)

The behavioral resident / intruder assay is used to screen compounds for anti-depressant-like activity. Compound #8 was tested in this assay, with the assay run according to the procedure as described in Rygula, R., Abumaria, N., Flugge, G., Fuchs, E., Ruther, E., Havemann-Reinecke, U., Behavioral Brain Research, 162 (2005), pp127-134.

Tables 5, 6 and 7 below, list the mean and standard deviation values for measured parameters, for the following compounds, administered p.o. (orally): vehicle, control compounds imipramine at 10 mg/kg and venlafaxine at 10 mg/kg, Compound #8 at 60 mg/kg and Compound #8 at 120 mg/kg.

**Table 5: Resident Intruder Effect on Sucrose Intake**

| | Dose | Sucrose Intake Mean ± SD | | |
|---|---|---|---|---|
| Treatment | mg/kg | Week 3 | Week 4 | Week 5 |
| Vehicle - non-stressed | 0 | 90.2 ± 2.5* | 89.0 ± 4.2* | 91.7 ± 2.1* |
| Vehicle - stressed | 0 | 51.7 ± 26.7 | 69.3 ± 7.5 | 66.3 ± 6.1 |
| Imipramine - non-stressed | 10 | 87.9 ± 2.7 | 89.7 ± 5.3 | 91.0 ± 3.7 |
| Imipramine - stressed | 10 | 70.5 ± 19.5 | 78.3 ± 15.1 | 73.6 ± 19.4 |
| Venlafaxine - non-stressed | 10 | 88.4 ± 9.1 | 91.5 ± 5.6 | 87.9 ± 15.0 |
| Venlafaxine - stressed | 10 | 74.5 ± 16.7 | 76.1 ± 18.2 | 73.3 ± 17.6 |
| Compound #8 - non-stressed | 60 | 90.9 ± 3.5 | 92.3 ± 1.4 | 88.9 ± 7.4 |
| Compound #8 - stressed | 60 ± | 92.1 ± 3.2* | 89.5 ± 6.9* | 92.4 ± 4.5* |
| Compound #8 - non-stressed | 120 | 83.6 ± 22.1 | 91.3 ± 4.6 | 89.3 ± 5.1 |
| Compound #8 - stressed | 120 | 76.1 ± 20.8 | 75.5 ± 20.6 | 87.4 ± 7.9* |
| * statistically significantly different from vehicle stressed animals | | | | |

**Table 6: Resident Intruder Effect on Locomotor Activity**

| Treatment | Dose mg/kg | Locomotor Mean ± SD | Rearing Mean ± SD | Sniffing Up Mean ± SD | Sniffing Dowr Mean ± SD |
|---|---|---|---|---|---|
| Vehicle non-stressed | 0 | week 1 8184.7 ± 1597.2<br>week 5 8102.3 ± 1805.6* | week 1 50.8 ± 10.2*<br>week 5 46.8 ± 7.6* | week 1 47.8 ± 9.6<br>week 5 43 ± 6.6* | week 1 24.5 ± 4.9<br>week 5 29.8 ± 5.3* |
| Vehicle stressed | 0 | week 1 5816.8 ± 1589.6<br>week 5 3920.8 ± 887.3 | week 1 28 ± 11.2<br>week 5 18 ± 9.1 | week 1 27.5 ± 10.8<br>week 5 14.5 ± 6.7 | week 1 15.8 ± 5.7<br>week 5 9 ± 2.6 |
| Imipramine non-stressed | 10 | week 1 8162.8± 929.9<br>week 5 7278 ± 1030 | week 1 51.5 ± 12.6<br>week 5 42.1 ± 2.9 | week 1 48.1 ± 10.3<br>week 5 37.5 ± 2.1 | week 1 25.8 ± 4.9<br>week 5 26.4 ± 5.2 |
| Imipramine stressed | 10 | week 1 6037.8 ± 1382.8<br>week 5 5642.8 ± 998.6 | week 1 30 ± 9.5<br>week 5 27.8 ± 7.4 | week 1 27.5 ± 7.9<br>week 5 24.3 ± 5.1 | week 1 14.4 ± 4.4<br>week 5 17.3 ± 3.4* |
| Venlafaxine non-stressed | 10 | week 1 9094.9 ± 1832.7<br>week 5 8078.6 ± 1665.2 | week 1 48.3 ± 11.2<br>week 5 40.1 ± 8.9 | week 1 43.3 ± 8.9<br>week 5 38.6 ± 7.7 | week 1 27.1 ± 5.2<br>week 5 27 ± 4.7 |
| Venlafaxine stressed | 10 | week 1 6233.6 ± 1087.2<br>week 5 6250.1 ± 789.2* | week 1 30.8 ± 6.6<br>week 5 35.1 ± 7.4 | week 1 28.6 ± 6.5<br>week 5 33.1 ± 7.5* | week 1 14.9 ± 3.5<br>week 5 18.3 ± 3.6* |
| | | week 1 8288.6 ± 2117.5 | week 1 49.6 ± 8.7 | week 1 44.1 ± 9.4 | week 1 20.9 ± 3.3 |

(continued)

| Treatment | Dose mg/kg | Locomotor Mean ± SD | Rearing Mean ± SD | Sniffing Up Mean ± SD | Sniffing Dowr Mean ± SD |
|---|---|---|---|---|---|
| Compound #8 non-stressed | 60 | week 5 7922.9 ± 1476 | week 5 37.4 ± 11.5 | week 5 33.4 ± 11.2 | week 5 23.5 ± 6.4 |
| Compound #8 stressed | 60 | week 1 5858.5 ± 708.2<br>week 5 7080.9 ± 1238.7* | week 1 26.5 ± 6.1<br>week 5 32.8 ± 10.3 | week 1 25 ± 7.3<br>week 5 30.6 ± 11.1 | week 1 13 ± 3.3<br>week 5 24.8 ± 5.7* |
| Compound #8 non-stressed | 120 | week 1 8056.6 ± 867.2<br>week 5 8648.3 ± 1060.9 | week 1 42 ± 10.6<br>week 5 36.5 ± 6.3 | week 1 37.6 ± 8.4<br>week 5 33.5 ± 5.9 | week 1 22.8 ± 3.7<br>week 5 25.5 ± 4.1 |
| Compound #8 stressed | 120 | week 1 6168.3 ± 1132.1<br>week 5 6444 ± 1010.3* | week 1 29.3 ± 7<br>week 5 30.1 ± 6.4 | week 1 27.5 ± 6.1<br>week 5 30.4 ± 7.5 | week 1 13 ± 3.6<br>week 5 22.5 ± 4.1* |
| * statistically significantly different from vehicle stressed animals | | | | | |

**Table 7: Resident Intruder Effect on Forced Swim Test**

| Treatment | Dose mg/kg | Immobility during 5min Week 5 Mean ± SD |
|---|---|---|
| Vehicle - non-stressed | 0 | 165.5 ± 25* |
| Vehicle - stressed | 0 | 227 ± 40.2 |
| Imipramine - non-stressed | 10 | 125.3 ± 50.1 |
| Imipramine - stressed | 10 | 157.5 ± 71.6 |
| Venlafaxine - non-stressed | 10 | 181.8 ± 27.6 |
| Venlafaxine - stressed | 10 | 190.4 ± 36.8 |
| Compound #8 - non-stressed | 60 | 164.3 ± 25.3 |
| Compound #8 - stressed | 60 | 175.1 ± 29.5 |
| Compound #8 - non-stressed | 120 | 136.7 ± 34.9 |
| Compound #8 - stressed | 120 | 128.3 ± 37.4 * |
| * statistically significantly different from vehicle stressed animals | | |

Compound #8 was active in the resident / intruder assay indicating that Compound #8 would be expected to be active as an anti-depressant.

**Example 18**

As a specific embodiment of an oral composition, 100 mg of the Compound #8 prepared as in Example 7 is formulated with sufficient finely divided lactose to provide a total amount of 580 to 590 mg to fill a size O hard gel capsule.

### Claims

**1.** A compound of formula (I)

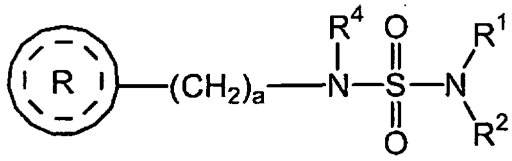

(I)

or a pharmaceutically acceptable salt thereof for use in the treatment of depression, wherein

$R^1$ and $R^2$ are each independently selected from the group consisting of hydrogen and lower alkyl, wherein lower alkyl means a straight or branched carbon chain of 1-4 carbon atoms;

$R^4$ is selected from the group consisting of hydrogen and lower alkyl, wherein lower alkyl means a straight or branched carbon chain of 1-4 carbon atoms;

a is an integer from 1 to 2;

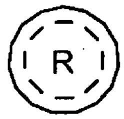

is selected from the group consisting of

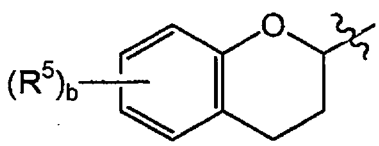

,

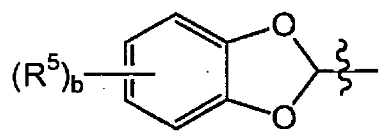

,

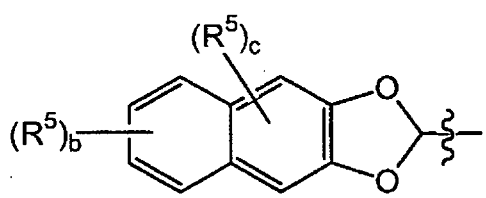

,

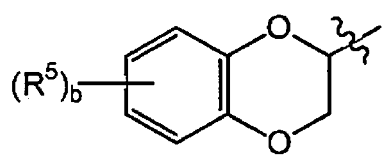

,

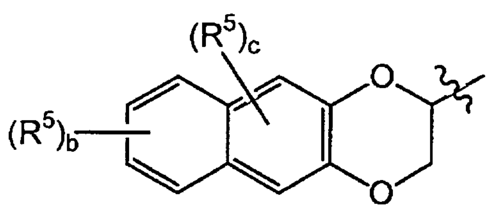

,

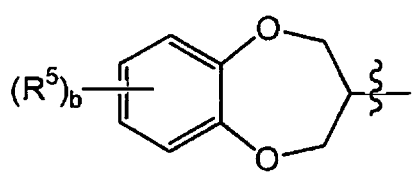

and

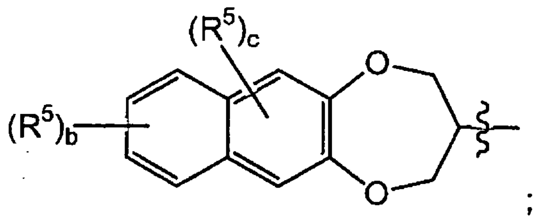

wherein b is an integer from 0 to 4; and wherein c is an integer from 0 to 2;
each $R^5$ is independently selected from the group consisting of halogen, lower alkyl and nitro, wherein lower alkyl means a straight or branched carbon chain of 1-4 carbon atoms;
provided that when

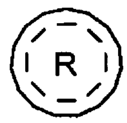

is

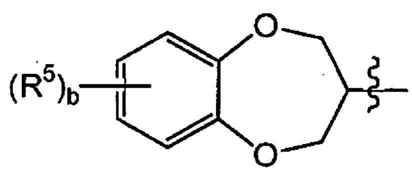

or

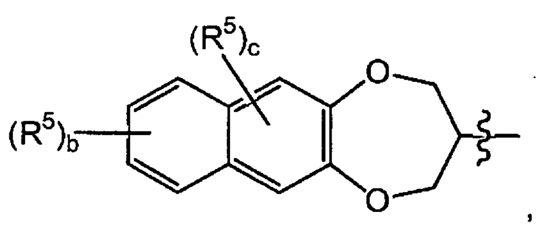

then a is 1.

2. The compound or a pharmaceutically acceptable salt thereof for use as in Claim 1, wherein
$R^1$ and $R^2$ are each independently selected from the group consisting of hydrogen and lower alkyl, wherein lower alkyl means a straight or branched carbon chain of 1-4 carbon atoms;
$R^4$ is selected from the group consisting of hydrogen and lower alkyl, wherein lower alkyl means a straight or branched carbon chain of 1-4 carbon atoms;
a is an integer from 1 to 2;

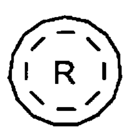

is selected from the group consisting of

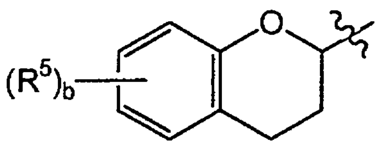 , 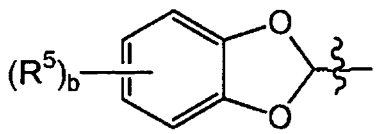 ,

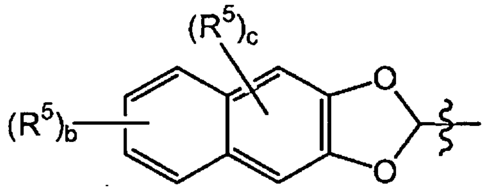 , 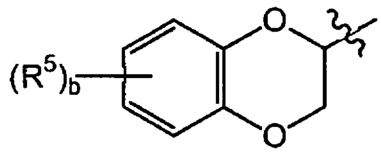 ,

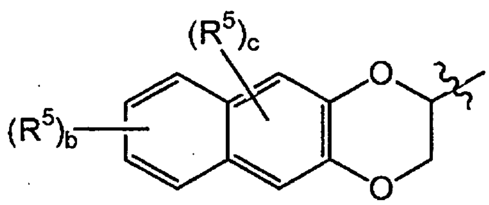 , 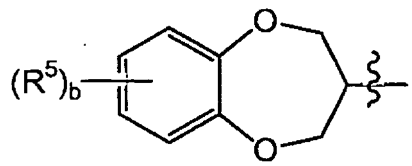

and

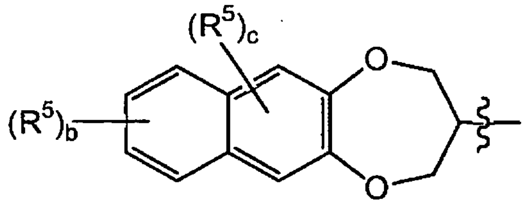 ;

wherein b is an integer from 0 to 2; and wherein c is an integer from 0 to 1;
each $R^5$ is independently selected from the group consisting of halogen, lower alkyl and nitro, wherein lower alkyl means a straight or branched carbon chain of 1-4 carbon atoms;
provided that when

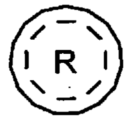

is

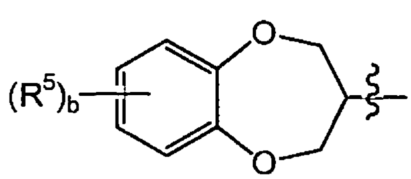

or

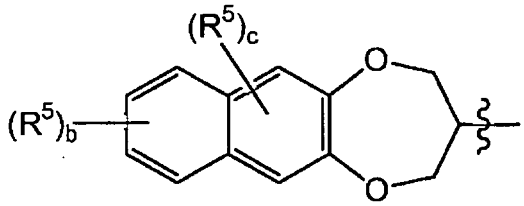

,

then a is 1.

**3.** The compound or a pharmaceutically acceptable salt thereof for use as in Claim 2, wherein
$R^1$ and $R^2$ are each independently selected from the group consisting of hydrogen and lower alkyl, wherein lower alkyl means a straight or branched carbon chain of 1-4 carbon atoms;
$R^4$ is selected from the group consisting of hydrogen and lower alkyl, wherein lower alkyl means a straight or branched carbon chain of 1-4 carbon atoms;
a is an integer from 1 to 2;

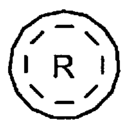

is selected from the group consisting of

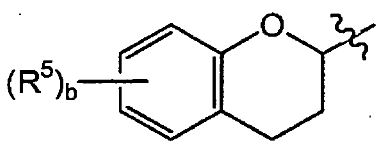

,

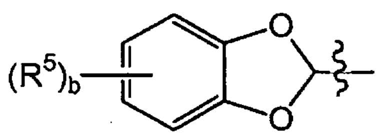

,

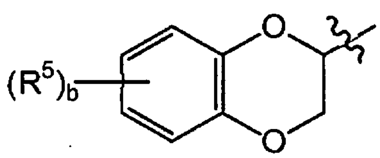

,

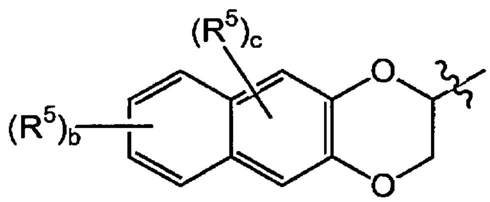

and

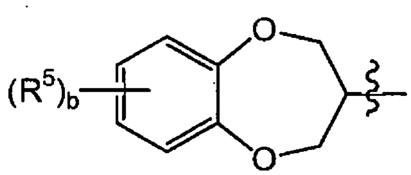

;

wherein b is an integer from 0 to 2; and wherein c is 0;
each $R^5$ is independently selected from the group consisting of halogen, lower alkyl and nitro, wherein lower alkyl means a straight or branched carbon chain of 1-4 carbon atoms;
provided that when

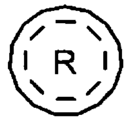

is

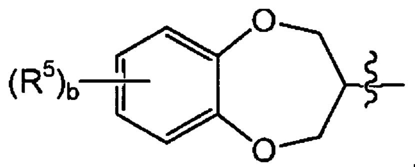

then a is 1.

**4.** The compound or a pharmaceutically acceptable salt thereof for use as in Claim 3, wherein
$R^1$ and $R^2$ are each independently selected from the group consisting of hydrogen and lower alkyl, wherein lower alkyl means a straight or branched carbon chain of 1-4 carbon atoms;
$R^4$ is selected from the group consisting of hydrogen and methyl;
a is an integer from 1 to 2;

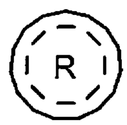

is selected from the group consisting of 2-(2,3-dihydro-benzo[1,4]dioxinyl), 2-(benzo[1,3]dioxolyl), 2-(3,4-dihydro-2H-benzo[1,4]dioxepinyl), 2-(2,3-dihydro-benzo[1,4]dioxinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo [1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl);
provided that when

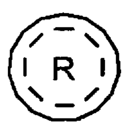

is 2-(3,4-dihydro-2H-benzo[1,4]dioxepinyl), then a is 1.

**5.** The compound or a pharmaceutically acceptable salt thereof for use as in Claim 4, wherein
$R^1$ and $R^2$ are each independently selected from the group consisting of hydrogen and methyl;
$R^4$ is selected from the group consisting of hydrogen and methyl;
a is an integer from 1 to 2;

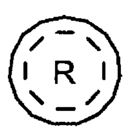

is selected from the group consisting of 2-(benzo[1,3]dioxolyl), 2-(2,3-dihydro-benzo[1,4]dioxinyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl).

**6.** The compound of Claim 1 for use in the treatment of depression, wherein the compound of formula (I) is selected from the group consisting of (2S)-(-)-N-(6-chloro-2,3-dihydro-benzo[1,4]dioxin-2-ylmethyl)-sulfamide; and pharmaceutically acceptable salts thereof.

**7.** A compound selected from the group consisting of (2S)-(-)-N-(6-ch)oro-2,3-dihydro-benzo[1,4]dioxin-2-ylmethyl)-sulfamide; and pharmaceutically acceptable salts thereof for use in the treatment of depression.

**8.** A compound of formula (III)

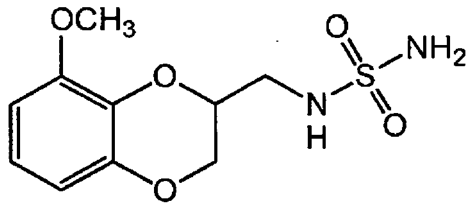

(III)

or a pharmaceutically acceptable salt thereof for use in the treatment of depression.

**9.** The compound or a pharmaceutically acceptable salt thereof of any of Claims 1, 7 and 8 for use in the treatment of depression, wherein the depression is selected from the group consisting of major depressive disorder, unipolar depression, treatment refractory depression, resistant depression, anxious depression and dysthymia.

**10.** The compound or a pharmaceutically acceptable salt thereof of any of Claims 1, 7 and 8 for use in the treatment of depression, wherein the depression is selected from the group consisting of major depressive disorder, unipolar depression, treatment refractory depression, resistant depression and anxious depression.

**11.** The compound or a pharmaceutically acceptable salt thereof of any of Claims 1, 7 and 8 for use in the treatment of depression, wherein the depression is major depressive disorder.

**12.** At least one antidepressant and a compound of claim 1 with formula (I)

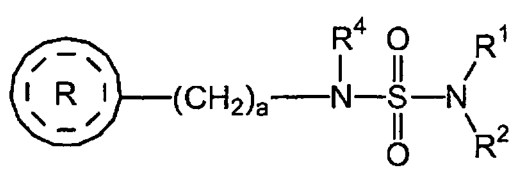

(I)

or a pharmaceutically acceptable salt thereof for use in the treatment of depression.

**13.** At least one antidepressant and a compound of claim 1 with formula (I)

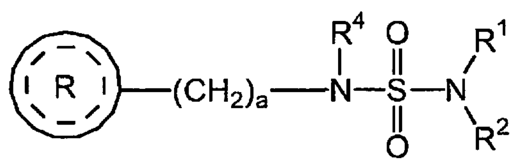

(I)

or a pharmaceutically acceptable salt thereof for use in co-therapy for the treatment of depression.

**14.** At least one antidepressant and a compound of claim 1 with formula (I)

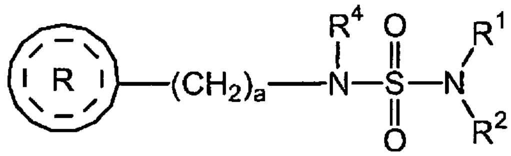
(I)

or a pharmaceutically acceptable salt thereof as a combined preparation for separate, simultaneous or sequential use in the treatment of depression.

**15.** The use of at least one antidepressant in the manufacture of a combined preparation of at least one antidepressant and a compound of claim 1 with formula (I)

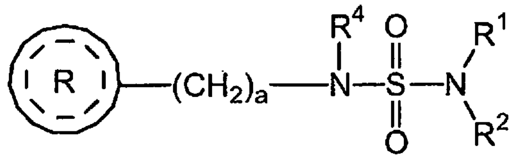
(I)

or a pharmaceutically acceptable salt thereof for simultaneous, separate or sequential use in the treatment of depression.

**16.** The use of a compound of claim 1 with formula (I)

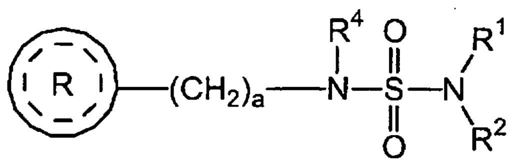
(I)

or a pharmaceutically acceptable salt thereof in the manufacture of a combined preparation of at least one antidepressant and a compound of formula (I) or pharmaceutically acceptable salt thereof for simultaneous, separate or sequential use in the treatment of depression.

**17.** A compound of claim 1 with formula (I)

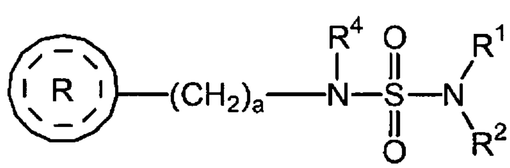
(I)

or a pharmaceutically acceptable salt thereof for use in the treatment of depression, wherein said treatment comprises the simultaneous, separate or sequential administration of at least one antidepressant.

**18.** The compound and at least one antidepressant of any of claims 12-14 and 17 for use in the treatment of depression, or the use of claims 15-16, wherein the compound of claim 1 with formula (I) is selected from the group consisting

of (2S)-(-)-N-(6-chloro-2,3-dihydro-benzo[1,4]dioxin-2-ylmethyl)-sulfamide; and pharmaceutically acceptable salts thereof.

**19.** The compound and at least one antidepressant for use in the treatment of depression as claimed in claim 18, or the use as claimed in claim 18, wherein the compound of claim 1 with formula (I) is (2S)-(-)-N-(6-chloro-2,3-dihydro-benzo[1,4]dioxin-2-ylmethyl)-sulfamide:

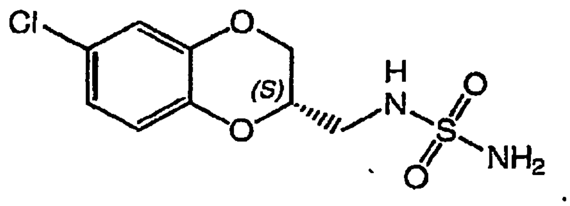

**20.** At least one antidepressant and a compound selected from the group consisting of (2S)-(-)-N-(6-chloro-2,3-dihydro-benzo[1,4]dioxin-2-ylmethyl)-sulfamide; and pharmaceutically acceptable salts thereof for use in the treatment of depression.

**21.** At least one antidepressant and a compound selected from the group consisting of (2S)-(-)-N-(6-chloro-2,3-dihydro-benzo[1,4]dioxin-2-ylmethyl)-sulfamide; and pharmaceutically acceptable salts thereof for use in co-therapy for the treatment of depression.

**22.** At least one antidepressant and a compound selected from the group consisting of (2S)-(-)-N-(6-chloro-2,3-dihydro-benzo[1,4]dioxin-2-ylmethyl)-sulfamide; and pharmaceutically acceptable salts thereof as a combined preparation for separate, simultaneous or sequential use in the treatment of depression.

**23.** The use of at least one antidepressant in the manufacture of a combined preparation of at least one antidepressant and a compound selected from the group consisting of (2S)-(-)-N-(6-chloro-2,3-dihydro-benzo[1,4]dioxin-2-ylmethyl)-sulfamide; and pharmaceutically acceptable salts thereof for simultaneous, separate or sequential use in the treatment of depression.

**24.** The use of a compound selected from the group consisting of (2S)-(-)-N-(6-chloro-2,3-dihydro-benzo[1,4]dioxin-2-ylmethyl)-sulfamide; and pharmaceutically acceptable salts thereof in the manufacture of a combined preparation of at least one antidepressant and a compound selected from the group consisting of (2S)-(-)-N-(6-chloro-2,3-dihydro-benzo[1,4]dioxin-2-ylmethyl)-sulfamide; and pharmaceutically acceptable salts thereof for simultaneous, separate or sequential use in the treatment of depression.

**25.** A compound selected from the group consisting of (2S)-(-)-N-(6-chloro-2,3-dihydro-benzo[1,4]dioxin-2-ylmethyl)-sulfamide; and pharmaceutically acceptable salts thereof for use in the treatment of depression, wherein said treatment comprises the simultaneous, separate or sequential administration of at least one antidepressant.

**26.** The compound and at least one antidepressant of any of claims 20-22 and 25 for use in the treatment of depression, or the use of claims 23-24, wherein the compound is (2S)-(-)-N-(6-chloro-2,3-dihydro-benzo[1,4]dioxin-2-ylmethyl)-sulfamide:

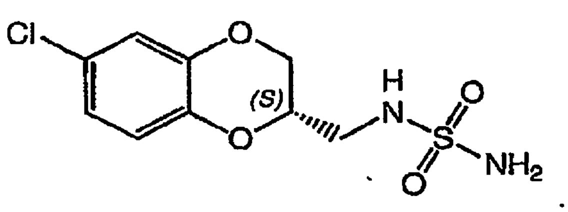

**27.** At least one antidepressant and a compound of formula (III)

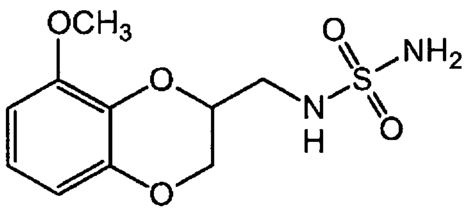

(III)

or a pharmaceutically acceptable salt thereof for use in the treatment of depression.

**28.** At least one antidepressant and a compound of formula (III)

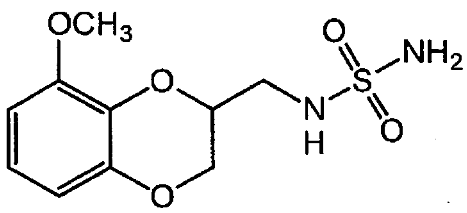

(III)

or a pharmaceutically acceptable salt thereof for use in co-therapy for the treatment of depression.

**29.** At least one antidepressant and a compound of formula (III)

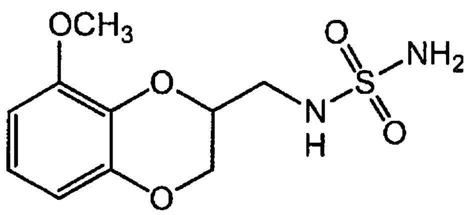

(III)

or a pharmaceutically acceptable salt thereof as a combined preparation for separate, simultaneous or sequential use in the treatment of depression.

**30.** The use of at least one antidepressant in the manufacture of a combined preparation of at least one antidepressant and a compound of formula (III)

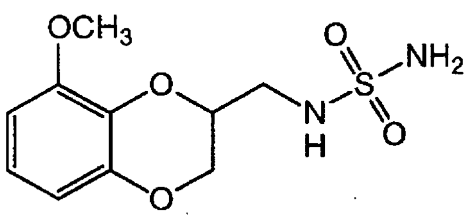

(III)

or a pharmaceutically acceptable salt thereof for simultaneous, separate or sequential use in the treatment of depression.

**31.** The use of a compound of formula (III)

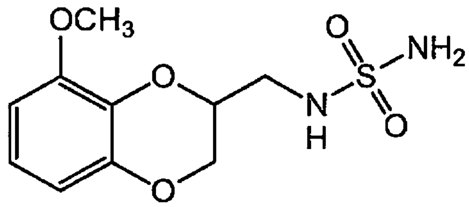

(III)

or a pharmaceutically acceptable salt thereof in the manufacture of a combined preparation of at least one antidepressant and a compound of formula (III) or a pharmaceutically acceptable salt thereof for simultaneous, separate or sequential use in the treatment of depression.

**32.** A compound of formula (III)

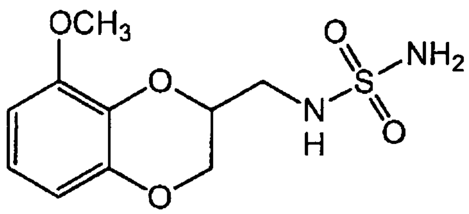

(III)

or a pharmaceutically acceptable salt thereof for use in the treatment of depression, wherein said treatment comprises the simultaneous, separate or sequential administration of at least one antidepressant.

**33.** The compound and at least one antidepressant of any of Claims 12-14, 17, 20-22, 25-29 and 32 for use in the treatment of depression, or the use of any of Claims 15-16, 23-24, 26 and 30-31, wherein the antidepressant is selected from the group consisting of imipramine, amitriptyline, desipramine, nortriptyline, doxepin, protriptyline, trimipramine, maprotiline, amoxapine, trazodone, bupropion, chlomipramine, fluoxetine, duloxetine, escitalopram, citalopram, sertraline, paroxetine, fluvoxamine, nefazadone, venlafaxine, milnacipran, reboxetine, mirtazapine, phenelzine, tranylcypromine, moclobemide, Kava-Kava, St. John's Wart, s-adenosylmethionine, thyrotropin releasing hormone, neurokinin receptor antagonists and triiodothyronine.

**34.** The compound and at least one antidepressant of any of Claims 12-14, 17, 20-22, 25-29 and 32 for use in the treatment of depression, or the use of any of Claims 15-16, 23-24, 26 and 30-31, wherein the antidepressant is selected from the group consisting of mono-amine oxidase inhibitors, tricyclics, serotonin reuptake inhibitors, serotonin noradrenergic reuptake inhibitors; noradrenergic and specific serotonergic agents and atypical antidepressants.

**35.** The compound and at least one antidepressant of any of Claims 12-14, 17, 20-22, 25-29 and 32 for use in the treatment of depression, or the use of any of Claims 15-16, 23-24, 26 and 30-31, wherein the antidepressant is selected from the group consisting of phenelzine, tranylcypromine, moclobemide, imipramine, amitriptyline, desipramine, nortriptyline, doxepin, protriptyline, trimipramine, chlomipramine, amoxapine, fluoxetine, sertraline, paroxetine, citalopram, fluvoxamine, venlafaxine, milnacipran, mirtazapine and bupropion.

**36.** The compound and at least one antidepressant of any of Claims 12-14, 17, 20-22, 25-29 and 32 for use in the treatment of depression, or the use of any of Claims 15-16, 23-24, 26 and 30-31, wherein the antidepressant is selected from the group consisting of neuropeptides, compounds targeting neuropeptide receptors and hormones.

**37.** The compound of any of claims 6 and 7 for use in the treatment of depression, wherein the compound is (2S)-(-)-N-(6-chloro-2,3-dihydro-benzo[1,4]dioxin-2-ylmethyl)-sulfamide:

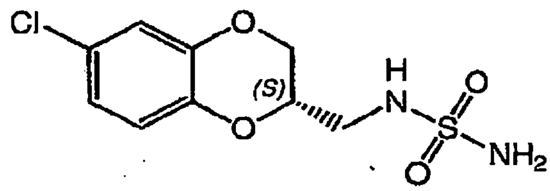

## Patentansprüche

**1.** Eine Verbindung von Formel (I)

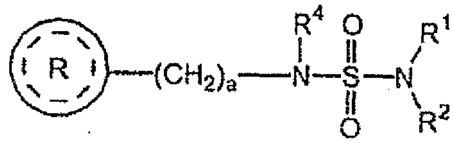

(I)

oder ein pharmazeutisch verträgliches Salz davon zur Verwendung bei der Behandlung von Depression, wobei

$R^1$ und $R^2$ jeweils unabhängig ausgewählt sind aus der Gruppe, bestehend aus Wasserstoff und Niederalkyl, wobei Niederalkyl eine gerade oder verzweigte Kohlenstoffkette von 1-4 Kohlenstoffatomen bedeutet;
$R^4$ ausgewählt ist aus der Gruppe, bestehend aus Wasserstoff und Niederalkyl, wobei Niederalkyl eine gerade oder verzweigte Kohlenstoffkette von 1-4 Kohlenstoffatomen bedeutet;
a eine ganze Zahl von 1 bis 2 ist;

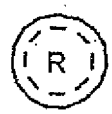

ausgewählt ist aus der Gruppe, bestehend aus

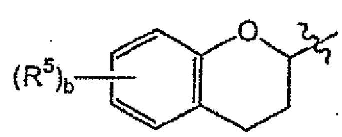

,

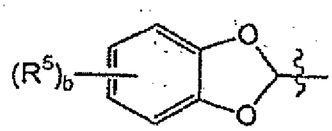

,

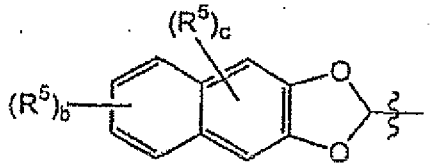

,

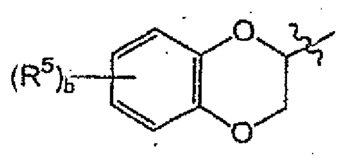

,

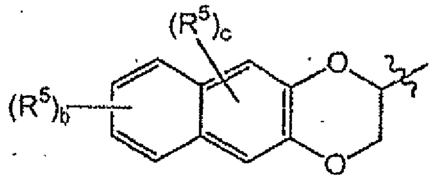

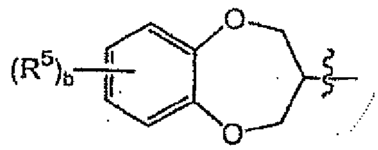

und

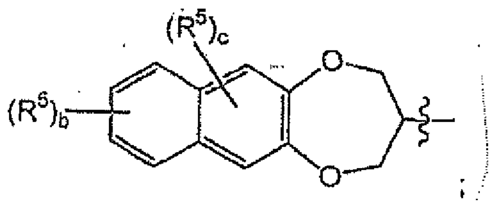

wobei b eine ganze Zahl von 0 bis 4 ist; und wobei c eine ganze Zahl von 0 bis 2 ist;
$R^5$ jeweils unabhängig ausgewählt ist aus der Gruppe, bestehend aus Halogen, Niederalkyl und Nitro, wobei Niederalkyl eine gerade oder verzweigte Kohlenstoffkette von 1-4 Kohlenstoffatomen bedeutet;
vorausgesetzt dass, wenn

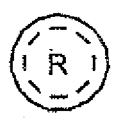

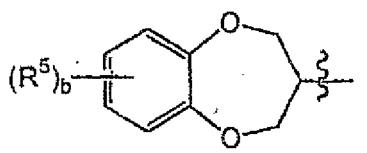

oder

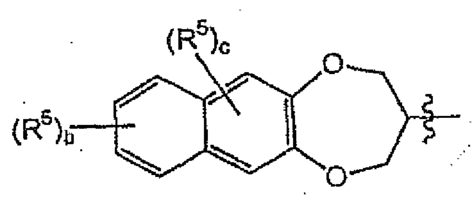

ist, dann a 1 ist.

**2.** Die Verbindung oder ein pharmazeutisch verträgliches Salz davon zur Verwendung wie in Anspruch 1, wobei
$R^1$ und $R^2$ jeweils unabhängig ausgewählt sind aus der Gruppe, bestehend aus Wasserstoff und Niederalkyl, wobei Niederalkyl eine gerade oder verzweigte Kohlenstoffkette von 1-4 Kohlenstoffatomen bedeutet;
$R^4$ ausgewählt ist aus der Gruppe, bestehend aus Wasserstoff und Niederalkyl, wobei Niederalkyl eine gerade oder verzweigte Kohlenstoffkette von 1-4 Kohlenstoffatomen bedeutet;
a eine ganze Zahl von 1 bis 2 ist;

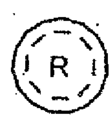

ausgewählt ist aus der Gruppe, bestehend aus

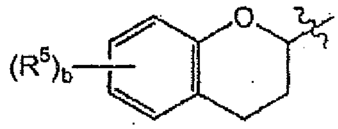
,
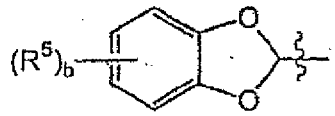
,

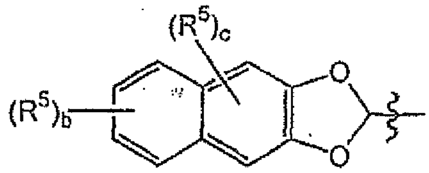
,
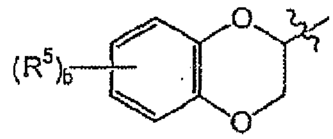
,

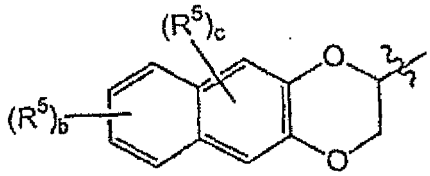
,
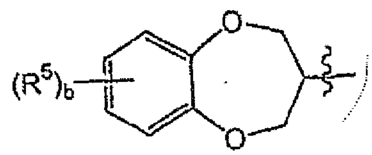

und

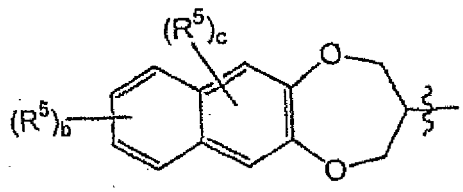
;

wobei b eine ganze Zahl von 0 bis 2 ist; und wobei c eine ganze Zahl von 0 bis 1 ist;
$R^5$ jeweils unabhängig ausgewählt ist aus der Gruppe, bestehend aus Halogen, Niederalkyl und Nitro, wobei Niederalkyl eine gerade oder verzweigte Kohlenstoffkette von 1-4 Kohlenstoffatomen bedeutet;
vorausgesetzt dass, wenn

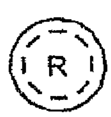
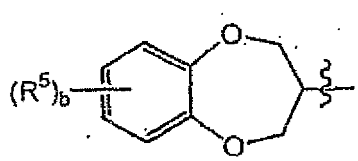

oder

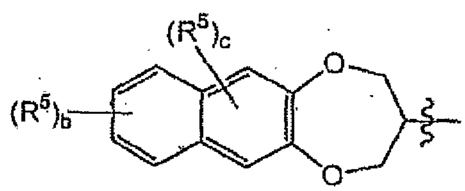

ist, dann a 1 ist.

**3.** Die Verbindung oder ein pharmazeutisch verträgliches Salz davon zur Verwendung wie in Anspruch 2, wobei $R^1$ und $R^2$ jeweils unabhängig ausgewählt sind aus der Gruppe, bestehend aus Wasserstoff und Niederalkyl, wobei Niederalkyl eine gerade oder verzweigte Kohlenstoffkette von 1-4 Kohlenstoffatomen bedeutet;
$R^4$ ausgewählt ist aus der Gruppe, bestehend aus Wasserstoff und Niederalkyl, wobei Niederalkyl eine gerade oder verzweigte Kohlenstoffkette von 1-4 Kohlenstoffatomen bedeutet;
a eine ganze Zahl von 1 bis 2 ist;

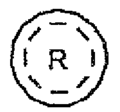

ausgewählt ist aus der Gruppe, bestehend aus

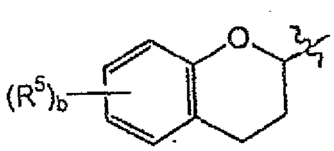,
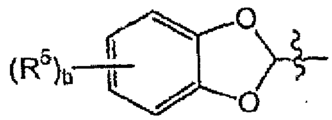,

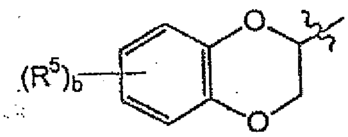,
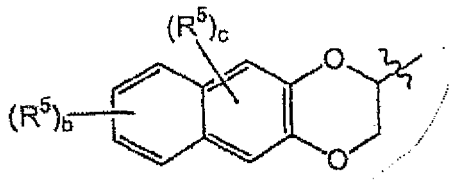

und

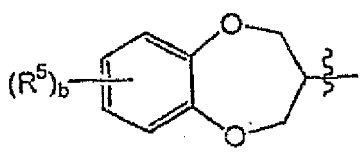;

wobei b eine ganze Zahl von 0 bis 2 ist; und wobei c 0 ist;
$R^5$ jeweils unabhängig ausgewählt ist aus der Gruppe, bestehend aus Halogen, Niederalkyl und Nitro, wobei Niederalkyl eine gerade oder verzweigte Kohlenstoffkette von 1-4 Kohlenstoffatomen bedeutet;
vorausgesetzt dass, wenn

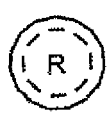
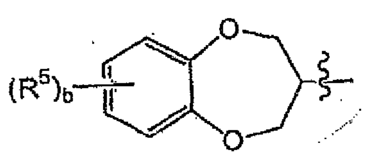

ist, dann a 1 ist.

**4.** Die Verbindung oder ein pharmazeutisch verträgliches Salz davon zur Verwendung wie in Anspruch 3, wobei $R^1$ und $R^2$ jeweils unabhängig ausgewählt sind aus der Gruppe, bestehend aus Wasserstoff und Niederalkyl, wobei Niederalkyl eine gerade oder verzweigte Kohlenstoffkette von 1-4 Kohlenstoffatomen bedeutet;

$R^4$ ausgewählt ist aus der Gruppe, bestehend aus Wasserstoff und Methyl;
a eine ganze Zahl von 1 bis 2 ist;

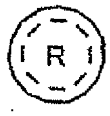

ausgewählt ist aus der Gruppe, bestehend aus 2-(2,3-Dihydrobenzo[1,4]dioxinyl), 2-(Benzo[1,3]dioxolyl), 2-(3,4-Dihydro-2H-benzo[1,4]dioxepinyl), 2-(2,3-Dihydrobenzo[1,4]dioxinyl), 2-(6-Chlor-2,3-dihydrobenzo[1,4]dioxinyl), 2-(6-Fluor-2,3-dihydrobenzo[1,4]dioxinyl), 2-(Chromanyl), 2-(5-Fluor-2,3-dihydrobenzo[1,4]dioxinyl), 2-(7-Chlor-2,3-dihydrobenzo[1,4]dioxinyl), 2-(6-Chlorbenzo[1,3]dioxolyl), 2-(7-Nitro-2,3-dihydrobenzo[1,4]dioxinyl), 2-(7-Methyl-2,3-dihydrobenzo[1,4]dioxinyl), 2-(5-Chlor-2,3-dihydrobenzo[1,4]dioxinyl), 2-(6-Brom-2,3-dihydrobenzo[1,4] dioxinyl), 2-(6,7-Dichlor-2,3-dihydrobenzo[1,4]dioxinyl), 2-(8-Chlor-2,3-dihydrobenzo[1,4]-dioxinyl), 2-(2,3-Dihydronaphtho[2,3-b][1,4]dioxinyl) und 2-(4-Methylbenzo[1,3]-dioxolyl);
vorausgesetzt dass, wenn

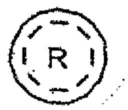

2-(3,4-Dihydro-2H-benzo[1,4]dioxepinyl) ist, dann a 1 ist.

**5.** Die Verbindung oder ein pharmazeutisch verträgliches Salz davon zur Verwendung wie in Anspruch 4, wobei
$R^1$ und $R^2$ jeweils unabhängig ausgewählt sind aus der Gruppe, bestehend aus Wasserstoff und Methyl;
$R^4$ ausgewählt ist aus der Gruppe, bestehend aus Wasserstoff und Methyl;
a eine ganze Zahl von 1 bis 2 ist;

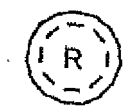

ausgewählt ist aus der Gruppe, bestehend aus 2-(Benzo[1,3]dioxolyl), 2-(2,3-Dihydrobenzo[1,4]dioxinyl), 2-(2,3-Dihydrobenzo[1,4]dioxinyl), 2-(6-Chlor-2,3-dihydrobenzo[1,4]dioxinyl), 2-(7-Chlor-2,3-dihydrobenzo[1,4]dioxinyl), 2-(7-Methyl-2,3-dihydrobenzo[1,4]dioxinyl), 2-(6-Brom-2,3-dihydrobenzo[1,4]-dioxinyl) und 2-(6,7-Dichlor-2,3-dihydrobenzo[1,4]dioxinyl).

**6.** Die Verbindung nach Anspruch 1 zur Verwendung bei der Behandlung von Depression, wobei die Verbindung von Formel (I) ausgewählt ist aus der Gruppe, bestehend aus (25)-(-)-N-(6-Chlor-2,3-dihydrobenzo[1,4]dioxin-2-ylmethyl)-sulfamid; und pharmazeutisch verträglichen Salzen davon.

**7.** Eine Verbindung, ausgewählt aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, zur Verwendung bei der Behandlung von Depression.

**8.** Eine Verbindung nach Anspruch 1 mit Formel (III)

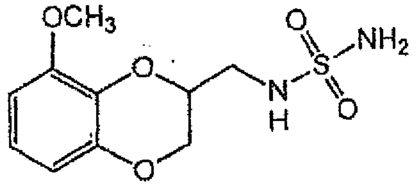

(III)

oder ein pharmazeutisch verträgliches Salz davon zur Verwendung bei der Behandlung von Depression.

**9.** Die Verbindung oder ein pharmazeutisch verträgliches Salz davon nach einem der Ansprüche 1, 7 und 8 zur Verwendung bei der Behandlung von Depression, wobei die Depression ausgewählt ist aus der Gruppe, bestehend aus typischer depressiver Störung, unipolarer Depression, durch Behandlung nicht-beeinflussbarer Depression, resistenter Depression, ängstlicher Depression und Dysthymie.

**10.** Die Verbindung oder ein pharmazeutisch verträgliches Salz davon nach einem der Ansprüche 1, 7 und 8 zur Verwerdung bei der Behandlung von Depression, wobei die Depression ausgewählt ist aus der Gruppe, bestehend aus typischer depressiver Störung, unipolarer Depression, durch Behandlung nicht-beeinflussbarer Depression, resistenter Depression und ängstlicher Depression.

**11.** Die Verbindung oder ein pharmazeutisch verträgliches Salz davon nach einem der Ansprüche 1, 7 und 8 zur Verwendung bei der Behandlung von Depression, wobei die Depression typische depressive Störung ist.

**12.** Wenigstens ein Antidepressivum und eine Verbindung nach Anspruch 1 mit Formel (I)

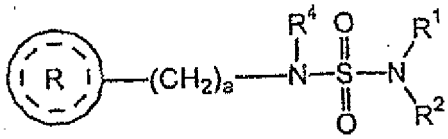

(I)

oder ein pharmazeutisch verträgliches Salz davon zur Verwendung bei der Behandlung von Depression.

**13.** Wenigstens ein Antidepressivum und eine Verbindung nach Anspruch 1 mit Formel (I)

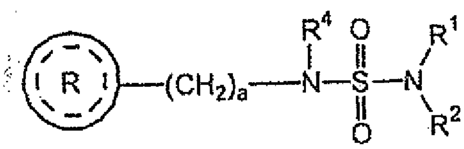

(I)

oder ein pharmazeutisch verträgliches Salz davon zur Verwendung in der Co-Therapie zur Behandlung von Depression.

**14.** Wenigstens ein Antidepressivum und eine Verbindung nach Anspruch 1 mit Formel (I)

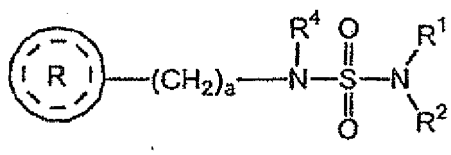

(I)

oder ein pharmazeutisch verträgliches Salz davon als ein Kombinationspräparat zur getrennten, gleichzeitigen oder sequenzielle Verwendung bei der Behandlung von Depression.

**15.** Die Verwendung wenigstens eines Antidepressivums bei der Herstellung eines Kombinationspräparates aus wenigstens einem Antidepressivum und einer Verbindung nach Anspruch 1 mit Formel (I)

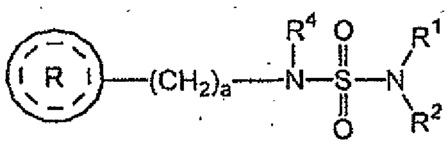

(I)

oder einem pharmazeutisch verträglichen Salz davon für gleichzeitige, getrennte oder sequenzielle Verwendung bei der Behandlung von Depression.

**16.** Die Verwendung einer Verbindung nach Anspruch 1 mit Formel (I)

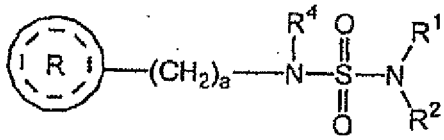

(I)

oder eines pharmazeutisch verträglichen Salzes davon bei der Herstellung eines Kombinationspräparates aus wenigstens einem Antidepressivum und einer Verbindung von Formel (I) oder einem pharmazeutisch verträglichen Salz davon für gleichzeitige, getrennte oder sequenzielle Verwendung bei der Behandlung von Depression.

**17.** Eine Verbindung nach Anspruch 1 mit Formel (I)

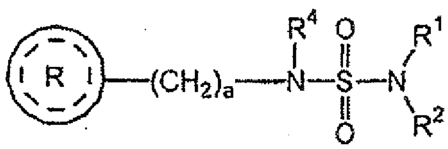

(I)

oder ein pharmazeutisch verträgliches Salz davon zur Verwendung bei der Behandlung von Depression, wobei die Behandlung die gleichzeitige, getrennte oder sequenzielle Verabreichung wenigstens eines Antidepressivums umfasst.

**18.** Die Verbindung und wenigstens ein Antidepressivum nach einem der Ansprüche 12-14 und 17 zur Verwendung bei der Behandlung von Depression oder die Verwendung nach den Ansprüchen 15-16, wobei die Verbindung nach Anspruch 1 mit Formel (I) ausgewählt ist aus der Gruppe, bestehend aus (2S)-(-)-N-(6-Chlor-2,3-dlhydrobenzo[1,4] dioxin-2-ylmethyl)-sulfamid; und pharmazeutisch verträglichen Salzen davon.

**19.** Die Verbindung und wenigstens ein Antidepressivum zur Verwendung bei der Behandlung von Depression nach Anspruch 18 oder die Verwendung nach Anspruch 18, wobei die Verbindung von Anspruch 1 mit Formel (I) (2S)-(-)-N-(6-Chlor-2,3-dihydrobenzo[1,4]dioxin-2-ylmethyl)-sulfamid:

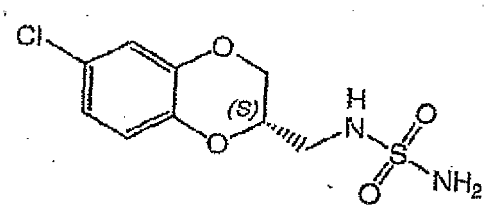

ist.

**20.** Wenigstens ein Antidepressivum und eine Verbindung, die ausgewählt ist aus der Gruppe, bestehend aus (2S)-(-)-N-(6-Chlor-2,3-dihydrobenzo[1,4]-dioxin-2-ylmethyl)-sulfamid; und pharmazeutisch verträglichen Salzen davon, zur Verwendung bei der Behandlung von Depression.

**21.** Wenigstens ein Antidepressivum und eine Verbindung, die ausgewählt ist aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, zur Verwendung in der Co-Therapie zur Behandlung von Depression.

**22.** Wenigstens ein Antidepressivum und eine Verbindung, die ausgewählt ist aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, als ein Kombinationspräparat für getrennte, gleichzeitige oder sequenzielle Verwendung bei der Behandlung von Depression.

**23.** Die Verwendung wenigstens eines Antidepressivums bei der Herstellung eines Kombinationspräparates aus wenigstens einem Antidepressivum und einer Verbindung, die ausgewählt ist aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, für gleichzeitige, getrennte oder sequenzielle Verwendung bei der Behandlung von Depression.

**24.** Die Verwendung einer Verbindung, die ausgewählt ist aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, bei der Herstellung eines Kombinationspräparates aus wenigstens einem Antidepressivum und einer Verbindung, die ausgewählt ist aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, für gleichzeitige, getrennte oder sequenzielle Verwendung bei der Behandlung von Depression.

**25.** Eine Verbindung, die ausgewählt ist aus der Gruppe, bestehend aus (2S)-(-)-N-(6-Chlor-2,3-dihydrobenzo[1,4]dioxin-2-ylmethyl)-sulfamid; und pharmazeutisch verträglichen Salzen davon, zur Verwendung bei der Behandlung von Depression, wobei die Behandlung gleichzeitige, getrennte oder sequenzielle Verabreichung wenigstens eines Antidepressivums umfasst.

**26.** Die Verbindung und wenigstens ein Antidepressivum nach einem der Ansprüche 20-22 und 25 zur Verwendung bei der Behandlung von Depression oder die Verwendung nach den Ansprüchen 23-24, wobei die Verbindung (2S)-(-)-N-(6-Chlor-2,3-dihydrobenzo[1,4]dioxin-2-ylmethyl)-sulfamid:

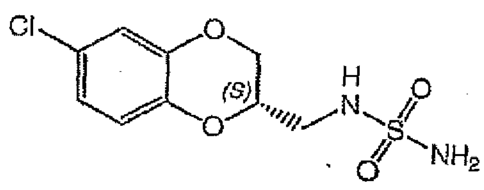

ist.

**27.** Wenigstens ein Antidepressivum und eine Verbindung von Formel (III)

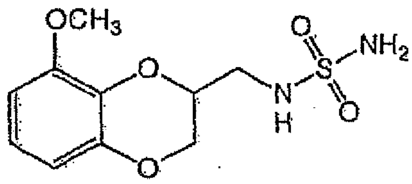

(III)

oder ein pharmazeutisch verträgliches Salz davon zur Verwendung bei der Behandlung von Depression.

**28.** Wenigstens ein Antidepressivum und eine Verbindung von Formel (III)

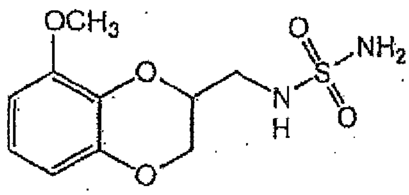

(III)

oder ein pharmazeutisch verträgliches Salz davon zur Verwendung in der Co-Therapie zur Behandlung von Depression.

**29.** Wenigstens ein Antidepressivum und eine Verbindung von Formel (III)

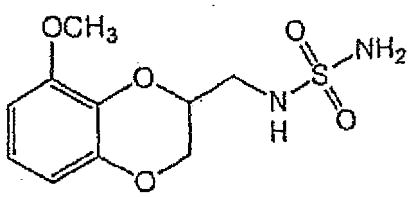

(III)

oder ein pharmazeutisch verträgliches Salz davon als ein Kombinationspräparat für getrennte, gleichzeitige oder sequenzielle Verwendung bei der Behandlung von Depression.

**30.** Die Verwendung wenigstens eines Antidepressivums bei der Herstellung eines Kombinationspräparates aus wenigstens einem Antidepressivum und einer Verbindung von Formel (III)

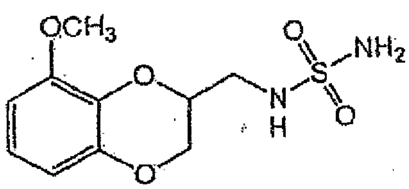

(III)

oder einem pharmazeutisch verträglichen Salz davon, für gleichzeitige, getrennte oder sequenzielle Verwendung bei der Behandlung von Depression.

**31.** Die Verwendung einer Verbindung von Formel (III)

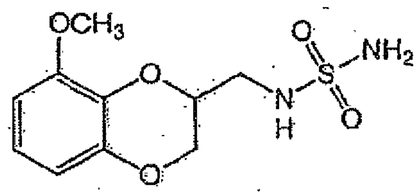

(III)

oder eines pharmazeutisch verträglichen Salzes davon bei der Herstellung eines Kombinationspräparates aus wenigstens einem Antidepressivum und einer Verbindung von Formel (III) oder einem pharmazeutisch verträglichen Salz davon, für gleichzeitige, getrennte oder sequenzielle Verwendung bei der Behandlung von Depression.

**32.** Eine Verbindung von Formel (III)

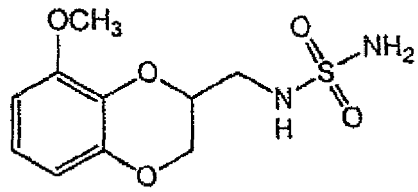

(III)

oder ein pharmazeutisch verträgliches Salz davon zur Verwendung bei der Behandlung von Depression, wobei die Behandlung die gleichzeitige, getrennte oder sequenzielle Verabreichung wenigstens eines Antidepressivums umfasst.

**33.** Die Verbindung und wenigstens ein Antidepressivum zur Verwendung bei der Behandlung von Depression wie in den Ansprüchen 12-14, 17, 20-22, 25-29 und 32 oder die Verwendung nach einem der Ansprüche 15-16, 23-24, 26 und 30-31, wobei das Antidepressivum ausgewählt ist aus der Gruppe, bestehend aus Imipramin, Amitriptylin, Desipramin, Nortriptylin, Doxepin, Protriptylin, Trimipramin, Maprotilin, Amoxapin, Trazodon, Bupropion, Chlomipramin, Fluoxetin, Duloxetin, Escitalopram, Citalopram, Sertralin, Paroxetin, Fluvoxamin, Nefazadon, Venlafaxin, Milnacipran, Reboxetin, Mirtazapin, Phenelzin, Tranylcypromin, Moclobemid, Kava-Kava, Johanniskraut, s-Adenosylmethionin, Thyrotropin-freisetzendem Hormon, Neurokinin-Rezeptor-Antagonisten und Triiodthyronin.

**34.** Die Verbindung und wenigstens ein Antidepressivum zur Verwendung bei der Behandlung von Depression wie in den Ansprüchen 12-14, 17, 20-22, 25-29 und 32 oder die Verwendung nach einem der Ansprüche 15-16, 23-24, 26 und 30-31, wobei das Antidepressivum ausgewählt ist aus der Gruppe, bestehend aus Monoaminoxidase-Inhibitoren, tricyclischen Verbindungen, Serotonin-reuptake-Hemmern, Serotonin-noradrenerg-reuptake-Hemmern, noradrenergen und spezifischen serotonergen Mitteln und atypischen Antidepressiva.

**35.** Die Verbindung und wenigstens ein Antidepressivum zur Verwendung bei der Behandlung von Depression wie in den Ansprüchen 12-14, 17, 20-22, 25-29 und 32 oder die Verwendung nach einem der Ansprüche 15-16, 23-24, 26 und 30-31, wobei das Antidepressivum ausgewählt ist aus der Gruppe, bestehend aus Phenelzin, Tranylcypromin, Moclobemid, Imipramin, Amitriptylin, Desipramin, Nortriptylin, Doxepin, Protriptylin, Trimipramin, Chlomipramin, Amoxapin, Fluoxetin, Sertralin, Paroxetin, Citalopram, Fluvoxamin, Venlafaxin, Milnacipran, Mirtazapin und Bupropion.

**36.** Die Verbindung und wenigstens ein Antidepressivum zur Verwendung bei der Behandlung von Depression wie in den Ansprüchen 12-14, 17, 20-22, 25-29 und 32 oder die Verwendung nach einem der Ansprüche 15-16, 22-24, 26 und 30-31, wobei das Antidepressivum ausgewählt ist aus der Gruppe, bestehend aus Neuropeptiden, Verbindungen, die Neuropeptid-Rezeptoren anzielen, und Hormonen.

**37.** Die Verbindung nach einem der Ansprüche 6 und 7 zur Verwendung bei der Behandlung von Depression, wobei die Verbindung (2S)-(-)-N-(6-Chlor-2,3-dihydrobenzo[1,4]dioxin-2-ylmethyl)-sulfamid:

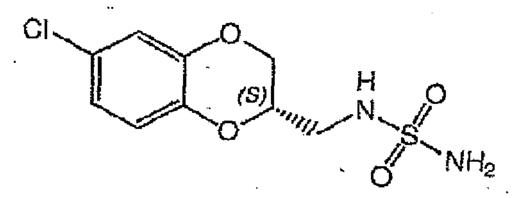

ist.

## Revendications

**1.** Composé de formule (I)

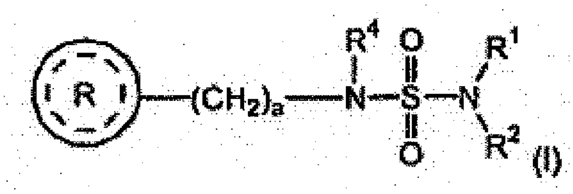

ou sel pharmaceutiquement acceptable de celui-ci à utiliser dans le traitement de la dépression, où

$R^1$ et $R^2$ sont chacun indépendamment choisis dans le groupe consistant en un atome d'hydrogène et un groupe alkyle inférieur, où alkyle inférieur signifie une chaîne carbonée droite ou ramifiée de 1 à 4 atomes de carbone;
$R^4$ est choisi dans le groupe consistant en un atome d'hydrogène et un groupe alkyle inférieur, où alkyle inférieur signifie une chaîne carbonée droite ou ramifiée de 1 à 4 atomes de carbone;
a est un nombre entier de 1 à 2;

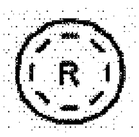

est choisi dans le groupe consistant en

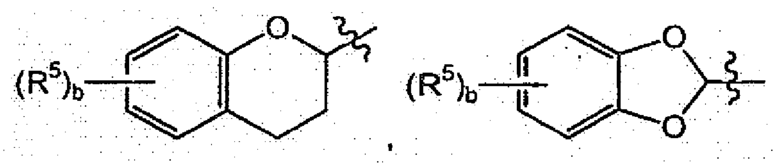

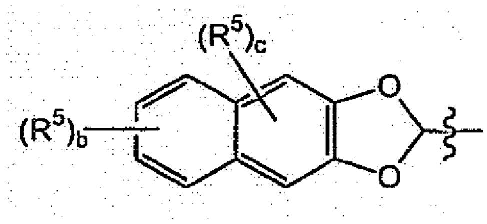

,

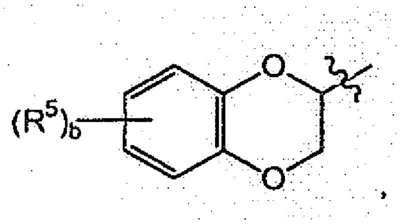

,

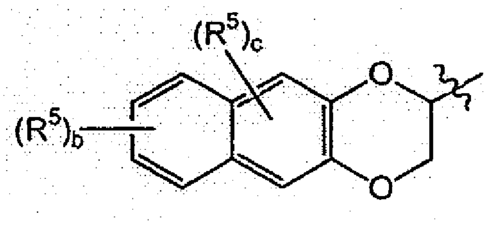

,

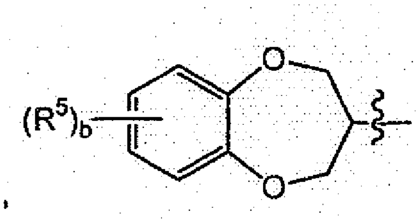

et

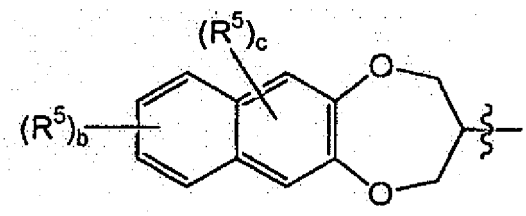

;

où b est un nombre entier de 0 à 4; et où c est un nombre entier de 0 à 2;
chaque $R^5$ est indépendamment choisi dans le groupe consistant en un atome d'halogène, un groupe alkyle inférieur et nitro, où alkyle inférieur signifie une chaîne carbonée de 1 à 4 atomes de carbone;
à condition que, lorsque

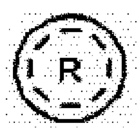

est

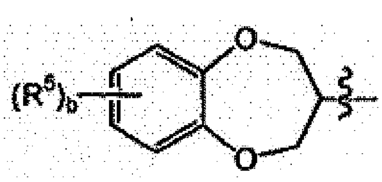

ou

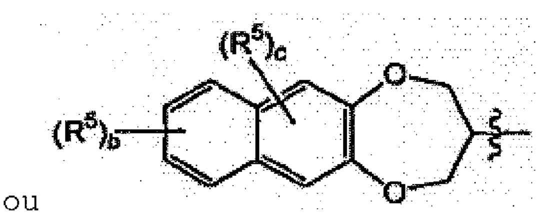

,

a vaille 1.

**2.** Composé ou sel pharmaceutiquement acceptable de celui-ci, à utiliser comme dans la revendication 1, où $R^1$ et $R^2$ sont chacun indépendamment choisis dans le groupe consistant en un atome d'hydrogène et un groupe alkyle inférieur, où alkyle inférieur signifie une chaîne carbonée droite ou ramifiée de 1 à 4 atomes de carbone;
$R^4$ est choisi dans le groupe consistant en un atome d'hydrogène et un groupe alkyle inférieur, où alkyle inférieur signifie une chaîne carbonée droite ou ramifiée de 1 à 4 atomes de carbone;
a est un nombre entier de 1 à 2;

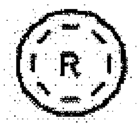

est choisi dans le groupe consistant en

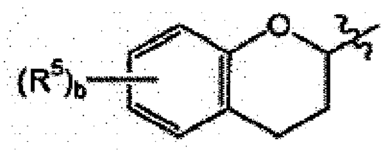 , 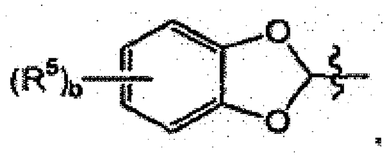 ,

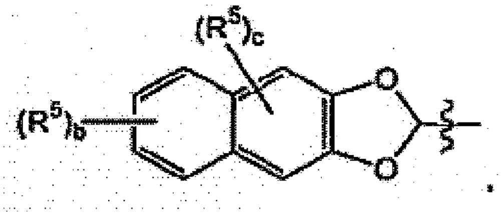 , 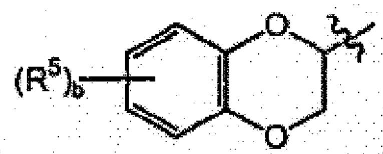 ,

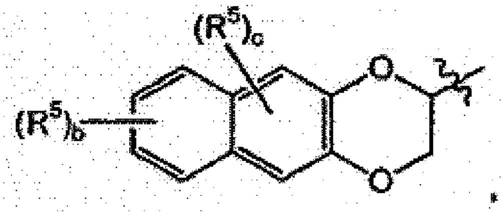 , 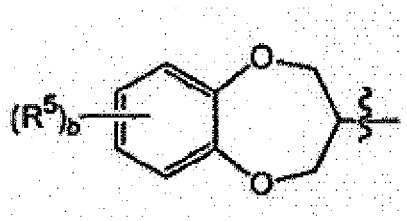

et

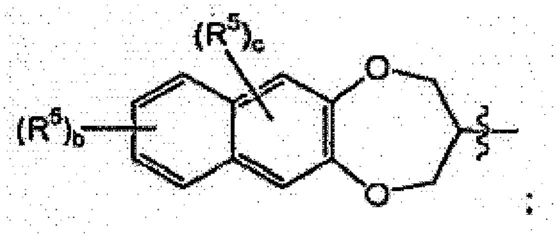 ;

où b est un nombre entier de 0 à 2; et où c est un nombre entier de 0 à 1;
chaque $R^5$ est indépendamment choisi dans le groupe consistant en un atome d'halogène, un groupe alkyle inférieur et nitro, où alkyle inférieur signifie une chaîne carbonée droite ou ramifiée de 1 à 4 atomes de carbone;
à condition que, lorsque

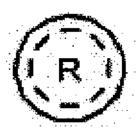

est

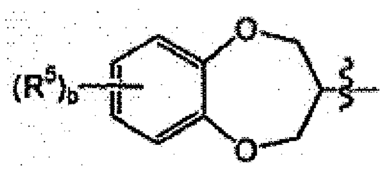

ou

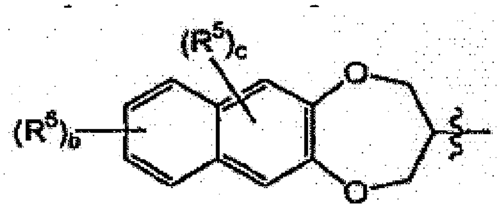

,

a vaille 1.

**3.** Composé ou sel pharmaceutiquement acceptable de celui-ci, à utiliser comme dans la revendication 2, où
$R^1$ et $R^2$ sont chacun indépendamment choisis dans le groupe consistant en un atome d'hydrogène et un groupe alkyle inférieur, où alkyle inférieur signifie une chaîne carbonée droite ou ramifiée de 1 à 4 atomes de carbone;
$R^4$ est choisi dans le groupe consistant en un atome d'hydrogène et un groupe alkyle inférieur, où alkyle inférieur signifie une chaîne carbonée droite ou ramifiée de 1 à 4 atomes de carbone;
a est un nombre entier de 1 à 2;

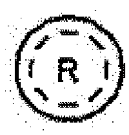

est choisi dans le groupe consistant en

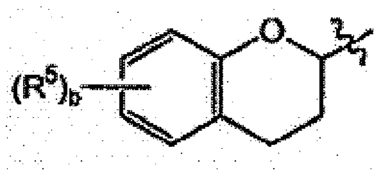

,

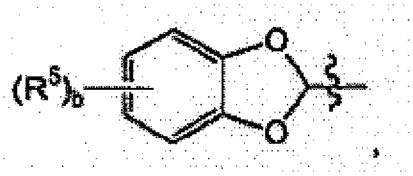

,

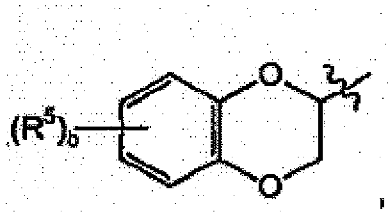

,

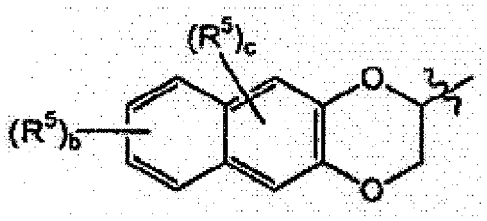

et

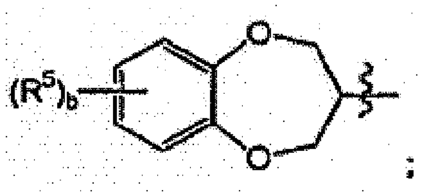

;

où b est un nombre entier de 0 à 2; et où c vaut 0; chaque $R^5$ est indépendamment choisi dans le groupe consistant en un atome d'halogène, un groupe alkyle inférieur et nitro, où alkyle inférieur signifie une chaîne carbonée droite ou ramifiée de 1 à 4 atomes de carbone;
à condition que, lorsque

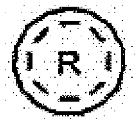

est

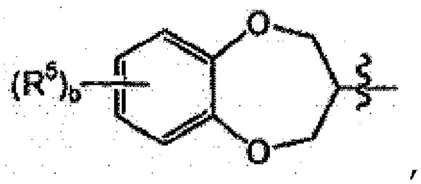

a vaille 1.

**4.** Composé ou sel pharmaceutiquement acceptable de celui-ci, à utiliser comme dans la revendication 3, où
$R^1$ et $P^2$ sont chacun indépendamment choisis dans le groupe consistant en un atome d'hydrogène et un groupe alkyle inférieur, où alkyle inférieur signifie une chaîne carbonée droite ou ramifiée de 1 à 4 atomes de carbone;
$R^4$ est choisi dans le groupe consistant en un atome d'hydrogène et un groupe méthyle;
a est un nombre entier de 1 à 2;

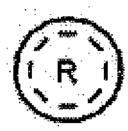

est choisi dans le groupe consistant en 2-(2,3-dihydro-benzo[1,4]dioxinyl), 2-(benzo[1,3]dioxolyl), 2-(3,4-dihydro-2H-benzo[1,4]dioxépinyl), 2-(2,3-dihydro-benzo[1,4]dioxinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-méthyl-2,3-dihydro-benzo[1,4]dioxinyl), 2-(5-chloxa-2,3-dihydro-benzo[1,4]dioxinyl), 2-(6-bromo-2,3-dihydro-benzo [1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydxo-naphto[2,3-b][1,4]dioxinyl) et. 2-(4-méthyl-benzo[1,3]dioxolyl);
à condition que, lorsque

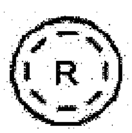

est 2-(3,4-dihydro-2H-benzo[1,4]dioxépinyl), a vaille 1.

**5.** Composé ou sel pharmaceutiquement acceptable de celui-ci, à utiliser comme dans la revendication 4, où
$R^1$ et $R^2$ sont chacun indépendamment choisis dans le groupe consistant en un atome d'hydrogène et un groupe méthyle;
$R^4$ est choisi dans le groupe consistant en un atome d'hydrogène et un groupe méthyle;
a est un nombre entier de 1 à 2;

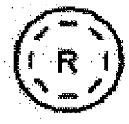

est choisi dans le groupe consistant en 2-(benzo[1,3]dioxolyl), 2-(2,3-dihydro-benzo[1,4]dioxinyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-méthyl-2,3-dihydro-benzo[1,4]dioxinyl), 2-(6-bromo-3,3-dihydro-benzo[1,4]dioxinyl) et 2-(6,7-dichloro-2,3-dihydro-benzo[1,4]dioxinyl).

6. Composé selon la revendication 1, à utiliser dans le traitement de la dépression, où le composé de formule (I) est choisi dans le groupe consistant en le (2S)-(-)-N-(6-chloro-2,3-dihydro-benzo[1,4]dioxin-2-ylméthyl)-sulfamide; et ses sels pharmaceutiquement acceptables.

7. Composé choisi dans le groupe consistant en le (2S)-(-)-N-(6-chloro-2,3-dihydro-benzo[1,4]dioxin-2-ylméthyl)-sulfamide; et ses sels pharmaceutiquement acceptables à utiliser dans le traitement de la dépression.

8. Composé selon la revendication 1, de formule (III)

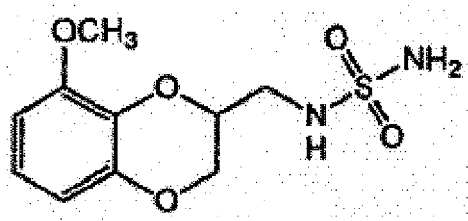

(III).

ou sel pharmaceutiquement acceptable de celui-ci à utiliser dans le traitement de la dépression.

9. Composé ou sel pharmaceutiquement acceptable de celui-ci selon l'une quelconque des revendications 1, 7 et 8, à utiliser dans le traitement de la dépression, où la dépression est choisie dans le groupe consistant en le trouble dépressif majeur, la dépression unipolaire, la dépression réfractaire au traitement, la dépression résistante, la dépression anxieuse et la dysthymie.

10. Composé ou sel pharmaceutiquement acceptable de celui-ci selon l'une quelconque des revendications 1, 7 et 8, à utiliser dans le traitement de la dépression, où la dépression est choisie dans le groupe consistant en le trouble dépressif majeur, la dépression unipolaire, la dépression réfractaire au traitement, la dépression résistante et la dépression anxieuse.

11. Composé ou sel pharmaceutiquement acceptable de celui-ci selon l'une quelconque des revendications 1, 7 et 8, à utiliser dans le traitement de la dépression, où la dépression est un trouble dépressif majeur.

12. Au moins un antidépresseur et un composé de la revendication 1 de formule (I)

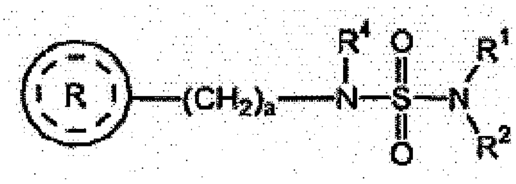

(I).

ou un sel pharmaceutiquement acceptable de celui-ci, à utiliser dans le traitement de la dépression.

**13.** Au moins un antidépresseur et un composé de la revendication 1 de formule (I)

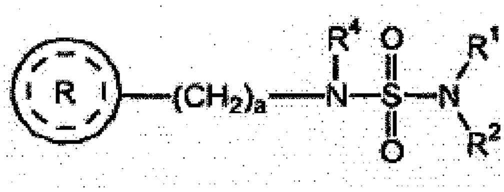

(I)

ou un sel pharmaceutiquement acceptable de celui-ci, à utiliser en co-thérapie pour le traitement de la dépression.

**14.** Au moins un antidépresseur et un composé de la revendication 1 de formule (I)

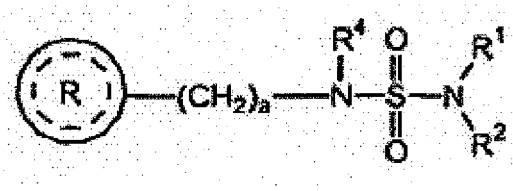

(I)

ou un sel pharmaceutiquement acceptable de celui-ci en tant que préparation combinée pour une utilisation séparée, simultanée ou séquentielle dans le traitement de la dépression.

**15.** Utilisation d'au moins un antidépresseur dans la fabrication d'une préparation combinée d'au moins un antidépresseur et d'un composé de la revendication 1 de formule (I)

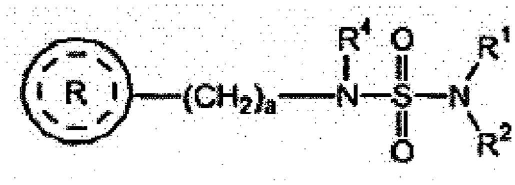

(I)

ou d'un sel pharmaceutiquement acceptable de celui-ci pour une utilisation simultanée, séparée ou séquentielle dans le traitement de la dépression.

**16.** Utilisation d'un composé de la revendication 1 de formule (1)

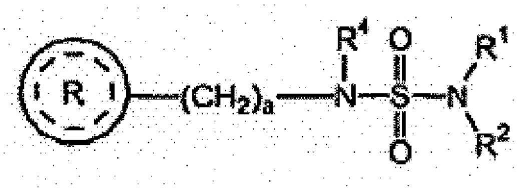

(I)

ou d'un sel pharmaceutiquement acceptable de celui-ci dans la fabrication d'une préparation combinée d'au moins un antidépresseur et d'un composé de formule (I) ou d'un sel pharmaceutiquement acceptable de celui-ci pour une utilisation simultanée, séparée ou séquentielle dans le traitement de la dépression.

**17.** Composé de la revendication 1 de formule (I)

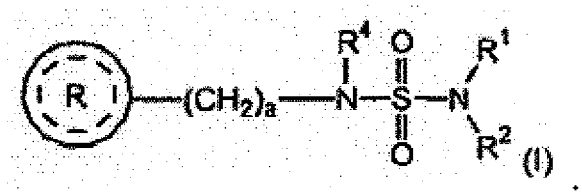

ou sel pharmaceutiquement acceptable de celui-ci à utiliser dans le traitement de la dépression, où ledit traitement comprend l'administration simultanée, séparée ou séquentielle d'au moins un antidépresseur.

**18.** Composé et au moins un antidépresseur selon l'une quelconque des revendications 12 à 14 et 17, à utiliser dans le traitement de la dépression, ou utilisation selon les revendications 15 à 16, où le composé de la revendication 1 de formule (I) est choisi dans le groupe consistant en le (2S)-(-)-N-(6-chloro-2,3-dihydro-benzo[1,4]dioxin-2-ylméthyl)-sulfamide; et ses sels pharmaceutiquement acceptables.

**19.** Composé et au moins un antidépresseur à utiliser dans le traitement de la dépression selon la revendication 18, ou l'utilisation telle que revendiquée dans la revendication 18, où le composé de la revendication 1 ayant la formule (I) est (2S)-(-)-N-(6-chloro-2,5-dihydro-benzo[1,4]dioxin-2-ylméthyl)-sulfamide:

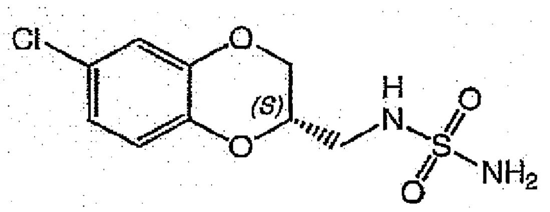

**20.** Au moins un antidépresseur et un composé choisi dans le groupe consistant en le (2S)-(-)-N-(6-chloro-2,3-dihydro-benzo[1,4]dioxin-2-ylméthyl)-sulfamide; et ses sels pharmaceutiquement acceptables à utiliser dans le traitement de la dépression.

**21.** Au moins un antidépresseur et un composé choisi dans le groupe consistant en le (2S)-(-)-N-(6-chloro-2,3-dihydro-benzo[1,4]dioxin-2-ylméthyl)-sulfamide; et ses sels pharmaceutiquement acceptables à utiliser en co-thérapie pour le traitement de la dépression.

**22.** Au moins un antidépresseur et un composé choisi dans le groupe consistant en le (2S)-(-)-N-(6-chloro-2,3-dihydro-benzo[1,4]dioxin-2-ylméthyl)-sulfamide; et ses sels pharmaceutiquement acceptables à utiliser en tant que préparation combinée pour une utilisation séparée, simultanée ou séquentielle dans le traitement de la dépression.

**23.** Utilisation d'au moins un antidépresseur dans la fabrication d'une préparation combinée d'au moins un antidépresseur et d'un composé choisi dans le groupe consistant en le (25)-(-)-N-(6-chloro-2,3-dihydro-benzo[1,4]dioxin-2-ylméthyl)-sulfamide; et ses sels pharmaceutiquement acceptables pour une utilisation simultanée, séparée ou séquentielle dans le traitement de la dépression.

**24.** Utilisation d'un composé choisi dans le groupe consistant en le (2S)-(-)-N-(6-chloro-2,3-dihydro-benzo[1,4]dioxzn-2-ylméthyl)-sulfamide; et ses sels pharmaceutiquement acceptables dans la fabrication d'une préparation combinée d'au moins un antidépresseur et d'un composé choisi dans le groupe consistant en le (2S)-(-)-N-(6-chloro-2,3-dihydro-benzo[1,4]dioxin-2-ylméthyl)-sulfamide; et ses sels pharmaceutiquement acceptables pour une utilisation simultanée, séparée ou séquentielle dans le traitement de la dépression.

**25.** Composé choisi dans le groupe consistant en le (2S)-(-)-N-(6-chloro-2,3-dihydro-benzo[1,4]dioxin-2-ylméthyl)-sulfamide; et ses sels pharmaceutiquement acceptables à utiliser dans le traitement de la dépression, où ledit traitement comprend l'administration simultanée, séparée ou séquentielle d'au moins un antidépresseur.

**26.** Composé et au moins un antidépresseur selon l'une quelconque des revendications 20-22 et 25 pour utilisation dans le traitement de la dépression, ou l'utilisation selon les revendications 23-24, où le composé est (25)-(-)-N-(6-chloro-2,3-dihydro-benzo[1,4]dioxin-2-ylméthyl)-sulfamide:

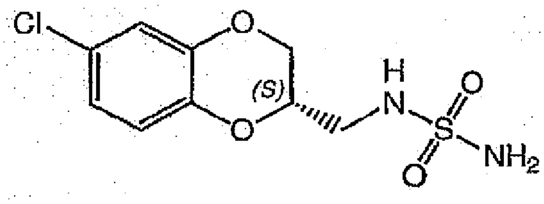

**27.** Au moins un antidépresseur et un composé de formule (III)

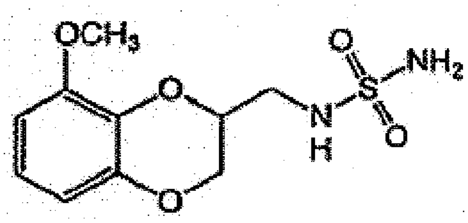

(III)

ou un sel pharmaceutiquement acceptable de celui-ci, à utiliser dans le traitement de la dépression.

**28.** Au moins un antidépresseur et un composé de formule (III)

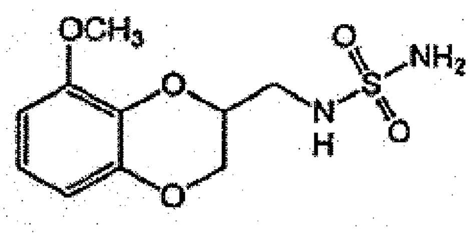

(III)

ou un sel pharmaceutiquement acceptable de celui-ci, à utiliser en co-thérapie pour le traitement de la dépression.

**29.** Au moins un antidépresseur et un composé de formule (III)

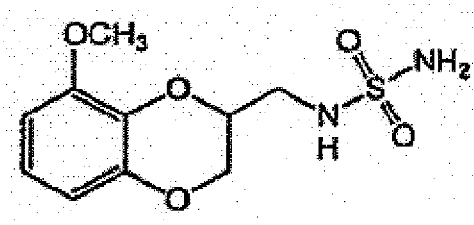

(III)

ou un sel pharmaceutiquement acceptable de celui-ci en tant que préparation combinée pour une utilisation séparée, simultanée ou séquentielle dans le traitement de la dépression.

**30.** Utilisation d'au moins un antidépresseur dans la fabrication d'une préparation combinée d'au moins un antidépresseur et d'un composé de formule (III)

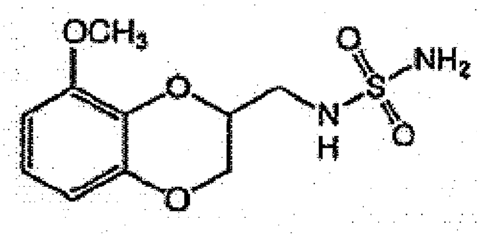

(III)

ou d'un sel pharmaceutiquement acceptable de celui-ci pour une utilisation simultanée, séparée ou séquentielle dans le traitement de la dépression.

**31.** Utilisation d'un composé de formule (III)

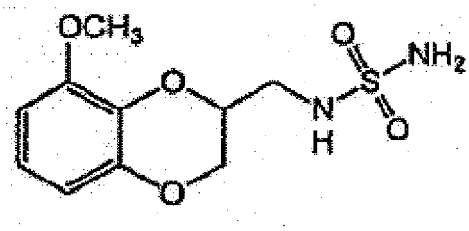

(III)

ou d'un sel pharmaceutiquement acceptable de celui-ci dans la fabrication d'une préparation combinée d'au moins un antidépresseur et d'un composé de formule (III) ou d'un sel pharmaceutiquement acceptable de celui-ci pour une utilisation simultanée, séparée ou séquentielle dans le traitement de la dépression.

**32.** Composé de formule (III)

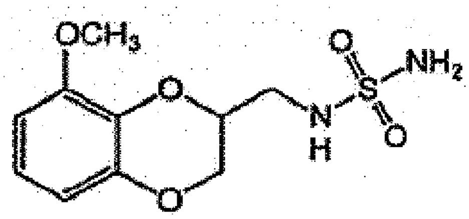

(III)

ou sel pharmaceutiquement acceptable de celui-ci, à utiliser dans le traitement de la dépression, où ledit traitement comprend l'administration simultanée, séparée ou séquentielle d'au moins un antidépresseur.

**33.** Composé et au moins un antidépresseur à utiliser dans le traitement de la dépression tel que dans l'une quelconque des revendications 12 à 14, 17, 20-22, 25 à 29 et 32, ou utilisation selon l'une quelconque des revendications 15 à 16, 23-24, 26 et 30-31, où l'antidépresseur est choisi dans le groupe consistant en l'imipramine, l'amitriptyline, la désipramine, la nortriptyline, la doxépine, la protriptyline, la trimipramine, la maprotiline, l'amoxapine, la trazodone, le bupropion, la chlomipramine, la fluoxétine, la duloxétine, l'escitalopram, le citalopram, la sertraline, la paroxétine, la fluvoxamine, la néfazadone, la venlafaxine, le milnacipran, la reboxétine, la mirtazapine, la phénélzine, la tranylcypromine, le moclobémide, le Kawa, le millepertuis, la s-adénosylméthionine, l'hormone de libération de la thyréostimuline, les antagonistes de récepteur de neurokinine et la triiodothyronine.

**34.** Composé et au moins un antidépresseur à utiliser dans le traitement de la dépression tel que dans l'une quelconque des revendications 12 à 14, 17, 20-22, 25 à 29 et 32, ou utilisation selon l'une quelconque des revendications 15 à 16, 23-24, 26 et 30-31, où l'antidépresseur est choisi dans le groupe consistant en les inhibiteurs de monoamine oxydase, les tricycliques, les inhibiteurs de réabsorption de sérotonine, les inhibiteurs de réabsorption noradrénergique de sérotonine; les agents noradrénergiques et sérotonergiques spécifiques et les antidépresseurs atypiques.

**35.** Composé et au moins un antidépresseur à utiliser dans le traitement de la dépression tel que dans l'une quelconque des revendications 12 à 14, 17, 20-22, 25 à 29 et 32, ou utilisation selon l'une quelconque des revendications 15 à 16, et 23-24, 26 et 30-31, où l'antidépresseur est choisi dans le groupe consistant en la phénélzine, la tranylcypromine, le moclobémide, l'imipramine, l'amitriptyline, la désipramine, la nortriptyline, la doxépine, la protriptyline, la trimipramine, la chlomipramine, l'amoxapine, la fluoxétine, la sertraline, la paroxétine, le citalopram, la fluvoxamine, la venlafaxine, le milnacipran, la mirtazapine et le bupropion.

**36.** Composé et au moins un antidépresseur à utiliser dans le traitement de la dépression tel que dans l'une quelconque des revendications 12 à 14, 17, 20-22, 25 à 29 et 32, ou utilisation selon l'une quelconque des revendications 15 à 16, 23-24, 26 et 30-31, où l'antidépresseur est choisi dans le groupe consistant en les neuropeptides, les composés ciblant les récepteurs de neuropeptide et les hormones.

**37.** Composé selon l'une quelconque des revendications 6 et 7 à utiliser dans le traitement de la dépression, où le composé est (2S)-(-)-N-(6-chloro-2,3-dihydro-benzo[1,4] dioxin-2-ylméthyl)-sulfamide:

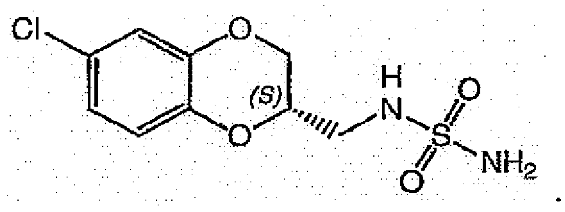

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 75173005 P
- WO 0209694 A


### Non-patent literature cited in the description


- *Harrison's Principles of Internal Medicine,* 2000
- Diagnostic and Statistical Manual of Mental Disorders. American Psychiatric Association, 2000
- **J.M. KENT.** *Lancet,* 2000, vol. 355, 911-918
- **J.W. WILLIAMS JR ; C.D. MULROW ; E. CHIQUETTE ; P.H. NOEL ; C. AGUILAR ; J. CORNELL.** *Ann. Intern. Med.,* 2000, vol. 132, 743-756
- **P.J. AMBROSINI.** *Psychiatr. Serv.,* 2000, vol. 51, 627-633
- **R.B. LYDIARD ; O. BRAWMAN-MINTZER.** *J: Clin. Psychiatry,* 1998, vol. 59 (18), 10-17
- **F. ROUILLON.** *Eur. Neuropsychopharmacol.,* 1999, vol. 9 (3), S87-S92
- **R.M. BERMAN ; M. NARASIMHAN ; D.S. CHARNEY.** *Depress. Anxiety,* 1997, vol. 5, 154-1.64
- **J. ANANTH.** *Psychother. Psychosom.,* 1998, vol. 67, 61-70
- **R.J. CADIEUX.** *Am. Fam. Physician,* 1998, vol. 58, 2059-2062
- **M. HATZINGER ; E. HOLSBOER-TRACHSLER.** *Wien. Med. Wochenschr.,* 1999, vol. 149, 511-514
- **C.B. NEMEROFF.** *Depress: Anxiety,* 1996, vol. 4, 169-181
- **T.A. KETTER ; R.M. POST ; P.I. PAREKH ; K. WORTHINGTON.** *J. Clin. Psychiatry,* 1995, vol. 56, 471-475
- **R.T. JOFFE ; W. SINGER ; A.J. LEVITT ; C. MACDONALD.** *Arch. Gen. Psychiatry,* 1993, vol. 50, 397-393
- Diagnostic and Statistical Manual of Mental Disorders. American Psychiatric Association, 1994
- **H.J. MOLLER ; H. GRUNZE.** *Eur. Arch. Psychiatry Clin. Neurosci.,* 2000, vol. 250, 57-68
- **J.R. CALABRESE ; D.J. RAPPORT ; S.E. KIMMEL ; M.D. SHELTON.** *Eur. Neuropsychopharmacol.,* 1999, vol. 9, S109-S112
- **B.E. Maryanoff ; D.F. McComsey ; M.J. Costanzo ; C. Hochman ; V. Smith-Swintosky ; R.P. Shank.** *J. Med Chem.,* 2004, vol. 48 (6), 1941-1947
- Protective Groups in Organic Chemistry. Plenum Press, 1973
- **T.W. Greene ; P.G.M. Wuts.** Protective Groups in Organic Synthesis. John Wiley & Sons, 1991
- **Malatynska, E. ; Rapp, R. ; Harrawood, D. ; Tunnicliff, G.** *Neuroscience and Biobehavioral Review,* 2005, vol. 82, 306-313
- **Malatynska, E. ; Knapp, R.J.** *Neuroscience and Biobehavioral Review,* 2005, vol. 29, 715-737
- **Rygula, R. ; Abumaria, N. ; Flugge, G. ; Fuchs, E. ; Ruther, E. ; Havemann-Reinecke, U.** *Behavioral Brain Research,* 2005, vol. 162, 127-134